(19) Europäisches Patentamt
European Patent Office
Office européen des brevets

(11) EP 0 421 777 B1

(12) **EUROPEAN PATENT SPECIFICATION**

(45) Date of publication and mention of the grant of the patent:
**28.02.1996 Bulletin 1996/09**

(51) Int. Cl.⁶: **C07H 19/067**, A61K 31/70, C07H 19/073, C07H 19/09

(21) Application number: **90310857.9**

(22) Date of filing: **04.10.1990**

(54) **Further antiviral pyrimidine nucleosides**

Weitere antivirale Pyrimidin-Nukleosiden

Nucléosides pyrimidiniques antiviraux

(84) Designated Contracting States:
**AT BE CH DE DK ES FR GB GR IT LI LU NL SE**

(30) Priority: **04.10.1989 GB 8922322**
**04.10.1989 GB 8922393**
**04.10.1989 GB 8922397**

(43) Date of publication of application:
**10.04.1991 Bulletin 1991/15**

(73) Proprietors:
- **THE UNIVERSITY OF BIRMINGHAM Birmingham B15 2TT (GB)**
- **THE WELLCOME FOUNDATION LIMITED London NW1 2BP (GB)**

(72) Inventors:
- **Walker, Richard, Department of Chemistry Birmingham B15 2TT (GB)**
- **Coe, Paul, Department of Chemistry Birmingham B15 2TT (GB)**
- **Rahim, Saad George, Department of Medicinal Chem. Langley Court, Beckenham, Kent BR6 0TZ (GB)**

(74) Representative: **Cresswell, Thomas Anthony et al London WC1R 5LX (GB)**

(56) References cited:
- **JOURNAL OF ORGANIC CHEMISTRY, vol. 36, no. 1, 15 January 1971; R.L. WHISTLER et al., pp. 108-110**
- **JOURNAL OF MEDICINAL CHEMISTRY, vol. 17, no. 5, May 1974; N. OTOTANI et al., pp. 535-537**
- **JOURNAL OF MEDICINAL CHEMISTRY, vol. 18, no. 8, August 1975; M. BOBEK et al., pp. 784-787**
- **LIEBIGS ANNALEN DER CHEMIE, no. 8, 10 August 1984; G. BENZ et al., pp. 1408-1423**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 421 777 B1

Printed by Rank Xerox (UK) Business Services
2.9.9/3.4

## Description

The present invention relates to pyrimidine nucleosides and their use in medical therapy particularly for the treatment or prophylaxis of virus infections.

Of the DNA viruses, those of the herpes group are the sources of the most common viral illnesses in man. The group includes herpes simplex virus (HSV), varicella zoster virus (VZV), cytomegalovirus (CMV); Epstein-Barr virus (EBV) and human herpes virus 6 (HHV6). HSV 1 and HSV 2 are some of the most common infectious agents of man. Most of these viruses are able to persist in the host's neural cells; once infected, individuals are at risk of recurrent clinical manifestations of infection which can be both physically and psychologically distressing.

HSV infection is often characterised by extensive and debilitating lesions of the skin, mouth and/or genitals. Primary infections may be subclinical although tend to be more severe than infections in individuals previously exposed to the virus. Ocular infection by HSV can lead to keratitis or cataracts thereby endangering the host's sight. Infection in the newborn, in immunocompromised patients or penetration of the infection into the central nervous system can prove fatal.

Transmission of the virus is by direct physical contact between a host and a recipient; the spread of HSV infection is therefore considered a very significant social problem, particularly as no effective vaccine is yet available.

Varicella zoster (VZV) is a herpes virus which causes chickenpox and shingles. Chickenpox is the primary disease produced in a host without immunity and in young children is usually a mild illness characterised by a vesicular rash and fever. Shingles or zoster is the recurrent form of the disease which occurs in adults who were previously infected with varicella-zoster virus. The clinical manifestions of shingles are characterised by neuralgia and a vesicular skin rash that is unilateral and dermatomal in distribution. Spread of inflammation may lead to paralysis or convulsions. Coma can occur if the meninges become affected. In immunodeficient patients VZV may disseminate causing serious or even fatal illness. VZV is of serious concern in patients receiving immunosuppressive drugs for transplant purposes or for treatment of malignant neoplasia and is a serious complication of AIDS patients due to their impaired immune system.

In common with other herpes viruses, infection with CMV leads to a lifelong association of virus and host and, following a primary infection, virus may be shed for a number of years. Congenital infection following infection of the mother during pregnancy may give rise to clinical effects such as death or gross disease (microcephaly, hepatosplenomegaly, jaundice, mental retardation), retinitis leading to blindness or, in less severe forms, failure to thrive, and susceptibility to chest and ear infections. CMV infection in patients who are immunocomprimised for example as a result of malignancy, treatment with immunosuppressive drugs following transplantation or infection with Human Immunodeficiency virus may give rise to retinitis, pneumoitis, gastrointestinal disorders and neurological diseases. CMV infection in AIDS patients is a predominant cause of morbidity as, in 50-80% of the adult population, it is present in a latent form and can be re-activated in immuno-compromised patients.

Epstein-Barr virus (EBV) causes infectious mononucleosis, and is also suggested as the causative agent of nasopharyngeal cancer, immunoblastic lymphoma, Burkitt's lymphoma and hairy leukoplakia.

HBV is a viral pathogen of world-wide major importance. The virus is aetiologically associated with primary hepatocellular carcinoma and is thought to cause 80% of the world's liver cancer. In the United States more than ten thousand people are hospitalised for HBV illness each year, and average of 250 die with fulminant disease. The United States currently contains an estimated pool of 500,000-1-million infectious carriers. Chronic active hepatitis generally develops in over 25% of carriers, and often progresses to cirrhosis. Clinical effects of infection with HBV range from headache, fever, malaise, nausea, vomiting, anorexia and abdominal pains. Replication of the virus is usually controlled by the immune response, with a course of recovery lasting weeks or months in humans, but infection may be more severe leading to persistent chronic liver disease outlined above.

We have now found that certain pyrimidine 4′-thionucleosides have potent activity against herpes viruses. The present invention therefore relates to pyrimidine 4′-thionucleosides of the formula (I)

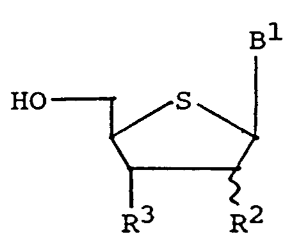

(I)

where $B^1$ is a pyrimidine base;

and either (a) $R^2$ is hydrogen and $R^3$ is hydrogen, hydroxy or fluoro,

or (b) $R^2$ is hydroxy and $R^3$ is hydrogen, hydroxy or fluoro,

or (c) $R^2$ is fluoro and $R^3$ is hydrogen or hydroxy,

or (d) $R^2$ and $R^3$ together form a carbon-carbon bond; and physiologically functional derivatives thereof, with the proviso that when $R^2$ is hydrogen and $R^3$ is hydroxy, $B^1$ is not a pyrimidine base of formula (X)

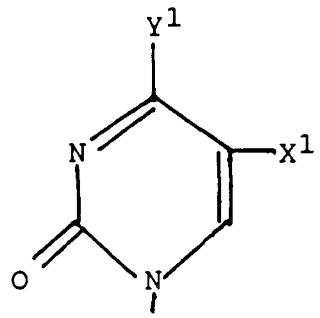

(X)

where $Y^1$ is hydroxy or amino and $X^1$ is chloro, bromo, iodo, triflouromethyl, $C_{2-6}$ alkyl, $C_{2-6}$ alkenyl, $C_{2-6}$ haloalkenyl or $C_{2-6}$ alkynyl.

The compounds excluded by the above proviso are those described and claimed by us in our co-pending application, EP-A-90307820.2, filed 17 July 1990.

In formula (I) the bond to $R^2$ is shown as "⋦" to indicate that $R^2$, when it is hydroxy or fluoro, may be in either the α- or β- conformation.

As used herein the term "pyrimidine base" refers to any nitrogenous base comprising the pyrimidine nucleus and having substituents at any one or more of the 2-, 3-, 4-,5-and/or 6- positions. Substituents at the 2-position include a sulphur atom in place of the oxo group. Particular pyrimidine bases include bases of formula (II)

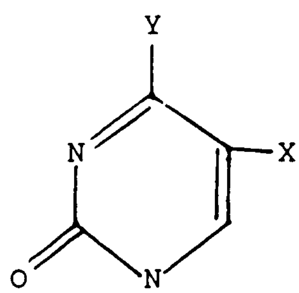

(II)

wherein Y is hydroxy or amino, monoalkylamino or dialkylamino; and X is halogen, alkoxy, alkyl, halo, alkenyl, haloalkenyl, alkynyl, amino, monoalkylamino, dialkylamino, cyano or nitro.

It will be appreciated that, by virtue of the definition of the group Y, the bases of formula (II) are derivatives either of uracil or of cytosine.

In the definition of formula (II), references to alkyl groups include groups which, when they contain at least three carbon atoms may be branched or cyclic but which are preferably straight (particular alkyl groups include ethyl); references to alkenyl groups include $C_{2-6}$ alkenyl groups; the alkenyl groups may be in the E- or Z-form or a mixture thereof, and, when they contain at least three carbon atoms, may be branched but are preferably straight; and references to alkynyl groups include $C_{2-6}$ alkynyl groups; alkynyl groups which contain at least four carbon atoms may be branched but are preferably straight; particular alkenyl groups include vinyl and E-(1-propenyl) and particular alkynyl groups include ethynyl and prop-1-ynyl. References to alkoxy include $C_{1-6}$ alkoxy groups; alkoxy groups which contain at least three carbon atoms may be branched but are preferably straight. References to halogens include fluorine, chlorine, bromine and iodine. References to halo-substituted groups include chloro, bromo, iodo and fluoro substituted groups and groups substituted with two or more halogens which may be the same or different, for example perhalo substituted groups (particular haloalkyl groups include trifluoromethyl and particular haloalkenyl groups include E-(2-bromovinyl)). References to alkylamino and dialkylamino groups include $C_{1-6}$alkylamino and di($C_{1-6}$)alkylamino groups; the alkyl groups may be branched but are preferably straight. References to dialkylamino groups include amino groups where the alkyl substituents are different.

Preferred compounds of the formula II include those in which the group X is $C_{2-4}$ alkyl or haloalkenyl, or $C_{3-4}$ alkenyl or alkynyl. Preferred haloalkenyl groups are straight chain haloalkenyl groups having a single halogen group on the terminal carbon. Also preferred are haloalkenyl groups having a double bond in the 1-position. Of such compounds, those having a 2-halovinyl group which is in the E configuration are preferred.

Particular compounds of formula (I) are those wherein (a) $R^2$ is hydrogen and $R^3$ is hydroxy or fluoro or (b) $R^2$ and $R^3$ are both hydrogen or both hydroxy, or (c) $R^2$ is $\alpha$-fluoro or $\beta$-fluoro and $R^3$ is hydrogen or $\alpha$-hydroxy or (d) $R^2$ and $R^3$ together form a carbon-carbon bond (giving a 4-hydroxymethyl-1,4-dihydropthiophenyl moiety).

Particular compounds of formula (I) are those wherein $B^1$ is a pyrimidine base of formula (II) selected from:

1. 5-Iodouracil
2. 5-Iodocytosine
3. 5-Ethynyluracil
4. 5-Prop-l-ynyluracil
5. 5-Vinyluracil
6. E-5-(2-Bromovinyl)uracil
7. 5-(2-Chloroethyl)uracil
8. E-5-(1-Propenyl)uracil
9. 5-Ethyluracil
10. 5-Trifluoromethyluracil
11. 5-Nitrouracil
12. 5-Aminouracil
13. 5-Methoxyuracil
14. 5-Cyanouracil

and the 4-thiopentofuranoside is selected from the residues of:

1. Thio-D-ribofuranose
2. 4-Thio-D-arabinofuranose
3. 2-Deoxy-4-thio-D-ribofuranose
4. 2,3-Dideoxy-4-thio-D-pentenofuranose
5. 2,3-Dideoxy-4-thio-D-ribofuranose
6. 2-Fluoro-4-thio-D-arabinofuranose
7. 2-Fluoro-4-thio-D-ribofuranose
8. 2,3-Dideoxy-3-fluoro-4-thio-D-ribofuranose

Derivatives of the compounds of the formula I include the corresponding sulphones and sulphoxides.

Preferred compounds of formula (I) are:
5-(2-chloroethyl)-2'-deoxy-4'-thiouridine;
5-nitro-2'-deoxy-4'-thiouridine;
5-amino-2'-deoxy-4'-thiouridine;
5-methylamino-2'-deoxy-4'-thiouridine;
5-ethynyl-4'-thiourdine;
E-5-(2-bromovinyl)-4'-thiouridine;
5-ethynyl-1-(4-thio-$\beta$-D-arabinofuranosyl)uracil;
5-ethyl-1-(4-thio-$\beta$-D-arabinofuranosyl)uracil;
5-(prop-1-ynyl)-1-(4-thio-$\beta$-D-arabinofuranosyl)uracil;
E-5-(2-bromovinyl)-1-(4-thio-$\beta$-D-arabinofuranosyl)uracil;
1-(2-fluoro-4-thio-$\beta$-D-arabinofuranosyl)-5-methyl uracil;
1-(2-fluoro-4-thio-$\beta$-D-arabinofuranosyl)-5-iodocytosine;
1-(2,3-didehydro-2,3-dideoxy-4-thio-D-ribofuranosyl)-5-methyluracil; and
2'-deoxy-2'-fluoro-4'-thiocytidine.

The following compounds are known (Bobek et al; J. Medicinal Chem. (1975), Vol 18, No. 8, 784-787):

1. 1-(2-deoxy-4-thio-$\beta$-D-erythro-pentofuranosyl)-5-fluorouracil;
2. 4'-thiocytidine;
3. 2'-deoxy-5-fluoro-4'-thiouridine;
4. 5-chloro-4'-thiouridine 2',3'-diacetate;
5. 5-fluoro-4'-thiouridine 2',3'-diacetate;
6. 5-chloro-4'-thiouridine;
7. 5-fluoro-4'-thiouridine;
8. 5-iodo-4'-thiouridine;

9. 5-bromo-4'-thiouridine;
10. 1-(4-thio-β-D-arabinofuranosyl)cytosine;
11. 1-(4-thio-β-D-arabinofuranosyl)thymine;
12. 1-(4-thio-β-D-arabinofuranosyl)uracil;
13. 4'-thiocytidine hydrochloride;
14. 4'-thiouridine;
15. 5-methyl-4'-thiouridine;

no claim is made to any of these compounds per se.

It will be appreciated that the compounds of formula (I) may exist in various tautomeric forms.

The above-mentioned derivatives also include the pharmaceutically acceptable salts; esters and salts of esters, or any other compound which, upon administration to a human subject, is capable of providing (directly or indirectly) the antivirally active metabolite or residue thereof.

Preferred mono- and di-esters according to the invention include carboxylic acid esters in which the non-carbonyl moiety of the ester grouping is selected from straight or branched chain alkyl, (e.g. tertiarybutyl); cyclic alkyl (e.g. cyclohexyl); alkoxyalkyl (e.g. methoxymethyl), carboxyalkyl (e.g. carboxyethyl), aralkyl (e.g. benzyl), aryloxyalkyl (e.g. phenoxymethyl), aryl (e.g. phenyl optionally substituted by halogen, $C_{1-4}$ alkyl or $C_{1-4}$ alkoxy); sulphonate esters such as alkyl- or aralkyl- sulphonyl (e.g. methanesulphonyl); mono-, di- or tri-phosphate esters which may or may not be blocked, amino acids esters and nitrate esters. With regard to the above-described esters, unless otherwise specified, any alkyl moieties present in such esters advantageously contain 1 to 18 carbon atoms, particularly 1 to 4 carbon atoms, in the case of straight chain alkyl groups, or 3 to 7 carbon atoms in the case of branched or cyclic alkyl groups. Any aryl moiety present in such esters advantageously comprises a phenyl group. Any reference to any of the above compounds also includes a reference to a physiologically acceptable salt thereof.

Salts according to the invention which may be conveniently used in therapy include physiologically acceptable base salts, eg derived from an appropriate base, such as alkali metal (e.g. sodium), alkaline earth metal (e.g. magnesium) salts, ammonium and $NR_4$ (wherein R is $C_{1-4}$ alkyl) salts. When Y represents an amino group, salts include physiologically acceptable acid addition salts, including the hydrochloride and acetate salts.

Such nucleosides and their derivatives will be hereinafter referred to as the compounds according to the invention. The term "active ingredient" as used hereafter, unless the context requires otherwise, refers to a compound according to the invention.

The present invention further includes:

a) compounds according to the invention for use in the treatment or prophylaxis of viral infections particularly herpes virus infections such as those mentioned above and more particularly HSV, VZV or CMV infections.
b) a method for the treatment or prophylaxis of a herpes virus infection such as those mentioned above in a mammal including man, particularly HSV, VZV or CMV infection which comprises treating a subject with an effective non-toxic amount of a compound according to the invention.
c) use of a compound according to the invention in the manufacture of a medicament for use in the treatment or prophylaxis of a herpes virus infection, such as those mentioned above, particularly HSV, VZV or CMV infections.

Examples of the clinical conditions which may be treated in accordance with the invention include those infections caused HSV 1 & 2, VZV, CMV or HBV described above.

We have found that compounds according to the invention have high oral bioavailability and low toxicity. This provides compounds with a favourable therapeutic index.

The compounds according to the invention may be administered to mammals including humans by any route appropriate to the condition to be treated, suitable routes including oral, rectal, nasal, topical (including buccal and sublingual), vaginal and parenteral (including subcutaneous, intramuscular, intravenous, intradermal, intrathecal and epidural). It will be appreciated that the preferred route may vary with, for example, the condition of the recipient.

For each of the above-indicated utilities and indications the amount required of the individual active ingredients will depend upon a number of factors including the severity of the condition to be treated and the identity of the recipient and will ultimately be at the discretion of the attendant physician. In general, however, for each of these utilities and indications, a suitable, effective dose will be in the range 0.1 to 250 mg per kilogram body weight of recipient per day, preferably in the range 1 to 100 mg per kilogram body weight per day and most preferably in the range 5 to 30 mg per kilogram body weight per day; an optimum dose is about 15 mg per kilogram body weight per day (unless otherwise indicated all weights of active ingredient are calculated as the parent compound; for salts and esters thereof the figures would be increased proportionately.) The desired dose may if desired be presented as two, three, four or more sub-doses administered at appropriate intervals throughout the day. These sub-doses may be administered in unit dosage forms, for example, containing 10 to 1000 mg, preferably 20 to 500 mg and most preferably 100 to 400 mg of active ingredient per unit dosage form.

While it is possible for the compounds to be administered alone it is preferable to present them as pharmaceutical formulations. The formulations of the present invention comprise at least one active ingredient, as above defined, together with one or more acceptable carriers thereof and optionally other therapeutic ingredients. The carrier(s) must be "acceptable" in the sense of being compatible with the other ingredients of the formulation and not deleterious to the recipients thereof.

The formulations include those suitable for oral, rectal, nasal, topical (including buccal and sublingual), vaginal or parenteral (including subcutaneous, intramuscular, intravenous, intradermal, intrathecal and epidural) administration. The formulations may conveniently be presented in unit dosage form and may be prepared by any of the methods well known in the art of pharmacy. Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder (e.g. povidone, gelatin, hydroxypropylmethyl cellulose), lubricant, inert diluent, preservative, disintergrant (e.g. sodium starch glycolate, cross-linked povidone, cross-linked sodium carboxymethyl cellulose), surface-active or dispersing agent. Moulded tablets may be made by moulding in a suitable machine a mixture of the powdered compound moistened with an inert liquid diluent. The tablets may optionally be coated or scored an may be formulated so as to provide slow or controlled release of the active ingredient therein using, for example, hydroxpropylmethylcellulose in varying proportions to provide desired release profile.

For infections of the eye or other external tissues, e.g., mouth and skin, the formulations are preferably applied as a topical ointment or cream containing the active ingredient in an amount of, for example, 0.075 to 20% w/w, preferably 0.2 to 15% w/w and most preferably 0.5 to 10% w/w. When formulated in an ointment, the active ingredients may be employed with either a paraffinic or a water-miscible ointment base. Alternatively, the active ingredients may be formulated in a cream with an oil-in-water cream base.

If desired, the aqueous phase of the cream base may include, for example, at least 30% w/w of a polyhydric alcohol, i.e. an alcohol having two or more hydroxyl groups such as propylene glycol, butane-1,3-diol, mannitol, sorbitol, glycerol and polyethylene glycol and mixtures thereof. The topical formulations may desirably include a compound which enhances absorption or penetration of the active ingredient through the skin or other affected areas. Examples of such dermal penetration enhancers include dimethylsulphoxide and related analogues.

The oily phase of the emulsions of this invention may be constituted from known ingredients in a known manner. While this phase may comprise merely an emulsifier (otherwise known as an emulgent), it desirably comprises a mixture of at least one emulsifier with a fat or an oil or with both a fat and an oil. Preferably, a hydrophilic emulsifier is included together with a lipophilic emulsifier which acts as a stabilizer. It is also preferred to include both an oil and a fat. Together, the emulsifier(s) with or without stabilizer(s) make up the so-called emulsifying wax, and the wax together with the oil and/or fat make up the so-called emulsifying ointment base which forms the oily dispersed phase of the cream formulations.

Emulgents and emulsion stabilizers suitable for use in the formulation of the present invention include Tween 60, Span 80, cetostearyl alcohol, myristyl alcohol, glyceryl mono-stearate and sodium lauryl sulphate.

The choice of suitable oils or fats for the formulation is based on achieving the desired cosmetic properties, since the solubility of the active compound in most oils likely to be used in pharmaceutical emulsion formulations is very low. Thus the cream should preferably be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitats or a blend of branched chain esters known as Crodamol CAP may be used, the last three being preferred esters. These may be used alone or in combination depending on the properties required. Alternatively, high melting point lipids such as white soft paraffin and/or liquid paraffin or other mineral oil can be used.

Formulations suitable for topical administration to the eye also include eye drops wherein the active ingredient is dissolved or suspended in a suitable carrier, especially an aqueous solvent for the active ingredient. The active ingredient is preferably present in such formulations in a concentration of 0.5 to 20%, advantageously 0.5 to 10% particularly about 1.5% w/w.

Formulations suitable for topical administration in the mouth include lozenges comprising the active ingredient in a flavoured basis, usually sucrose and acacia or tragacanth; pastilles comprising the active ingredient in an inert basis such as gelatin and glycerin, or sucrose and acacia; and mouth-washes comprising the active ingredient in a suitable liquid carrier.

Formulations for rectal administration may be presented as a suppository with a suitable base comprising for example cocoa butter or a salicylate.

Formulations suitable for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose. Suitable formulations wherein the carrier is a liquid, for administration as for example a nasal spray or as nasal drops, include aqueous or oily solutions of the active ingredient.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents, and liposomes or other microparticulate systems which are designed to target the compound to blood components or one or more organs. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Injection solutions and suspensions may be prepared extemporaneously from sterile powders, granules and tablets of the kind previously described.

Preferred unit dosage formulations are those containing a daily dose or unit, daily sub-dose, as herein above recited, or an appropriate fraction thereof, of an active ingredient.

It should be understood that in addition to the ingredients particularly mentioned above the formulations of this invention may include other agents conventional in the art having regard to the type of formulation in question, for example those suitable for oral administration may include flavoring agents.

The compounds of formula (I) may be produced by various methods known in the art of organic chemistry in general and nucleoside synthesis in particular. Starting materials are either known and readily available from commercial sources or may themselves be produced by known and conventional techniques.

The present invention further provides a process for producing a compound of formula (I) as hereinbefore defined which process comprises:

A) reacting a compound of formula (III-A)

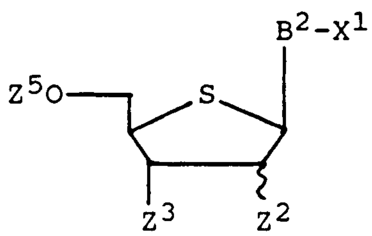

(III-A)

wherein $X^1$ is a precursor for a substituent of a pyrimidine base as defined in relation to $B^1$ in formula (I);

$B^2$ is a pyrimidine substituted by the group $X^1$;

$Z^2$ and $Z^3$ are the same or different and are groups $R^2$ and $R^3$ as defined in relation to formula (I) or protected hydroxyls; and

$Z^5$ is hydrogen or a hydroxyl protecting group with a reagent or reagents serving to convert the group $X^1$ to the desired group X as defined in relation to formula (II);

B) reacting a compound of formula (IV-A)

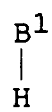

(IV-A)

or a protected form thereof with a 4-thio sugar derivative serving to introduce the 4-thio sugar moiety, or a protected form thereof, at the 1-position of the base $B^1$;

C) reacting a compound of formula (V-A)

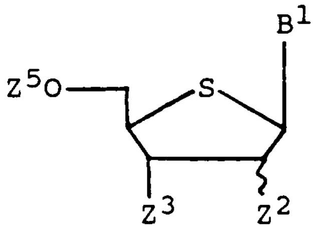

(V-A)

wherein
$B^1$ is a pyrimidine base as hereinbefore defined;
$Z^5$ is hydrogen or a hydroxy protecting group, and $Z^2$ and $Z^3$ are as defined above wherein at least one of $Z^2$ and $Z^3$ represents a precursor group for the group(s) $R^2$ and/or $R^3$ in formula (I)
under conditions or with a reagent serving to convert the groups $Z^2$ and/or $Z^3$ into the respective groups $R^2$ and/or $R^3$.
When $B^1$ is a compound of the formula (II) the particular processes which may be used include
D) reacting a compound of formula (III)

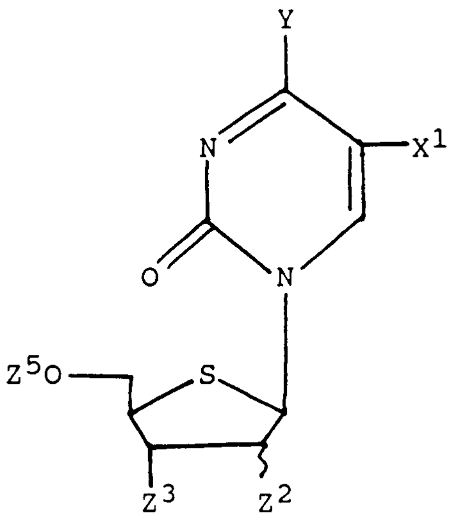

(III)

wherein $X^1$ is a precursor for the group X as defined in relation to formula (I);
Y is as defined in relation to formula (II);
$Z^2$ and $Z^3$ are the same or different and are groups $R^2$ and $R^3$ or protected hydroxyl groups and $Z^5$ is hydrogen or a hydroxyl protecting group with a reagent or reagents serving to convert the group $X^1$ to the desired group X;
E) reacting a compound of formula (IV)

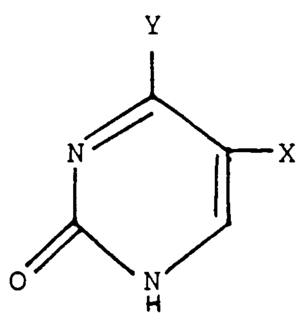

(IV)

wherein X and Y are as defined in relation to formula (I) or a protected form thereof with a 4-thio sugar derivative serving to introduce the 4-thio sugar moiety of formula (I), or a protected form thereof, at the 1-position of the compound of formula (IV);

F) reacting a compound of formula (V)

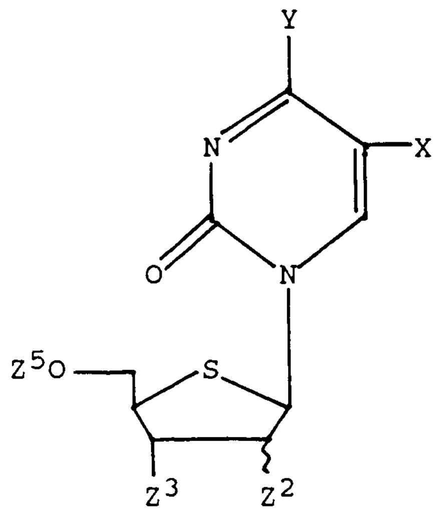

(V)

wherein X and Y are as defined in relation to formula (I), $Z^5$ is a hydroxy protecting group or hydrogen; $Z^2$ and $Z^3$ are as defined above wherein at least one of $Z^2$ and $Z^3$ represents a precursor group for the group(s) $R^2$ and/or $R^3$ in formula (I) under conditions or with a reagent serving to convert the groups $Z^2$ and/or $Z^3$ into the respective groups $R^2$ and/or $R^3$.

Where necessary or desired, following one or more of processes A to F above, any one or more of the following further steps in any desired or necessary order may be performed:

a) removing each of the protecting groups,
b) converting a compound of formula (I) or a protected form thereof into a further compound of formula (I) or a protected form thereof,
c) converting the compound of formula (I) or a protected form thereof into a physiologically acceptable derivative of the compound of formula (I) or a protected form thereof,
d) converting a physiologically acceptable derivative of the compound of formula (I) or a protected form thereof into the compound of formula (I) or a protected form, thereof,
e) converting a physiologically acceptable derivative of the compound of formula (I) or a protected form thereof into another physiologically acceptable derivative of the compound of formula (I) or a protected form thereof,
f) where necessary, separating the $\alpha$ and $\beta$ anomers of the compound of formula I or a protected derivative thereof or of a physiologically acceptable derivative of a compound of formula (I) or a derivative thereof, and
g) when 4'-sulphone or sulphoxide sugar moiety derivatives are required, partially or completely oxidising the sulphur of the 4-thio sugar moiety of the corresponding compound of formula (I) or a protected form thereof or a compound of formula (II) or a protected form thereof, with either a limited amount of a peracid such as m-chloroperbenzoic acid or with an excess of such a peracid to form a sulphoxide or sulphone derivative of the compound of formula (I).

The term "4-thio sugar" or "4-thio sugar compound" is used herein to denote a compound containing an optionally substituted 4-thiopentofuranoside wherein the 5-hydroxyl group is optionally protected, the optional substituents at the 2- and 3- positions include fluorine and hydrogen, and wherein the 1-position is optionally substituted by a leaving group.

Processes B and E may be effected, for example, by any one of the following:

a) Reacting the compound of formula (IV) or (IV-A), or a protected form thereof, with a 4-thio sugar compound of formula (VI)

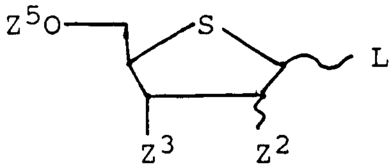

(VI)

wherein $Z^2$, $Z^3$ and $Z^5$ are as defined above and L is a leaving group, for example halogen, e.g. chloro, acyloxy (e.g. $C_{1-6}$ alkanoyloxy such as acetoxy), or S-benzyl. The group $Z^5$, for instance in formula (VI), is preferably a hydroxy protecting group, particularly a benzyl or toluoyl group. When $Z^2$ and $Z^3$ are protected hydroxyl groups, the hydroxy protecting groups may be those defined in relation to $Z^5$.

The reaction may be performed using standard methods including the use of a Lewis Acid catalyst such as mercuric chloride or bromide or stannic chloride or trimethylsilyltrifluoromethane-sulphonate in solvents such as acetonitrile, 1-2 dichloroethane, dichloromethane, chloroform or toluene at reduced, ambient or elevated temperature such as from -78°C to reflux.

b) Reacting of the compound of formula (IV-A) or (IV), or a protected form thereof, with a compound of formula (VII)

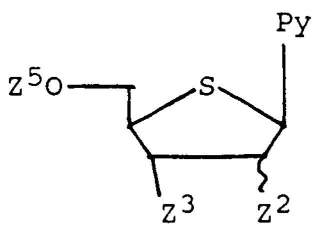

(VII)

wherein $Z^2$, $Z^3$ and $Z^5$ are as defined above and Py represents a pyrimidine base in the presence of a silylating agent such as N,O-bis-(trimethylsilyl)acetamide and in the presence of a Lewis Acid catalyst such as trimethylsilyltrifluoromethane sulphonate in a solvent such as acetonitrile. In the compound of formula (VII), Py is preferably the uracil or thymine base.

The 4-thio-sugar compound may be produced by conventional methods prior to coupling with the base or derived by modification of another sugar moiety which is already part of a nucleoside. Particular methods are as described in the Examples.

Particular methods for producing the compounds of formula (I) in accordance with the above processes will be described below and these may be combined in order to produce further compounds within formula (I).

Reference may be made to the following texts:

Synthetic Procedures in Nucleic Acid Chemistry, Eds. W.W. Zorbach R.S. Tipson, Vol. 1, Interscience, 1973;

Nucleic Acid Chemistry - Improved and New Synthetic Procedures, Methods and Techniques, Eds. L.B. Townsend and R.S.Tipson, Parts 1 and 2, Wiley-Interscience, 1978 and Part 3, Wiley-Interscience, 1986;

Nucleoside Analogues-Chemistry, Biology and Medical Applications Eds R.T Walker, E. De Clercq & F. Eckstein, NATO Advanced Study Institutes Series, Plenum Press, 1979;

Basic Principles in Nucleic Acid Chemistry, Eds. P.O.P Ts'O, Academic Press, 1974.

With regard to the use of protecting groups as referred to above, it will be appreciated that the particular nature of such groups will be dependent on the identity and nature of the particular group(s) to be protected and will therefore be selected in accordance with conventional techniques. Examples of protecting groups that may be generally used include acyl groups for example $C_{1-6}$ alkanoyl (e.g. acetyl) or aroyl (e.g. benzoyl or toluoyl), ether groups such as tri-$C_{1-6}$alkylsilyl (e.g. trimethylsilyl) or tert-butyl diphenylsilyl; or arylmethyl groups such as benzyl or triphenylmethyl groups.

The above groups may be removed in conventional manner, for example the acyl groups being removed advantageously under basic conditions (e.g. using sodium methoxide), the silyl ether groups being removed advantageously under aqueous or acidic conditions (e.g. using aqueous methanol to remove trimethylsilyl groups) and the arylmethyl groups being removed advantageously under reducing conditions.

Protection of hydroxy groups with trialkylsilyl, eg. trimethylsilyl, groups on the pyrimidine ring is conveniently achieved by reaction with (a) chlorotrimethylsilane together with triethylamine or with (b) hexamethyldisilazane, optionally together with chlorotrimethylsilane and/or ammonium sulphate.

The following techniques are particularly convenient:

X is halogen

5-Halopyrimidines are commercially available and may be coupled to the 4-thiosugar compound by conventional techniques, for instance by reacting a protected 5-halopyrimidine with a protected 4-thio sugar compound having a leaving group in the 1-position. The leaving group on the 4-thio sugar compound may be a halogen, benzylthio or preferably acetate group.

Reaction of the protected 4-thio sugar compound with the protected 5-halopyrimidine is conducted under conventional conditions using Lewis Acid catalysis such as by treatment with mercuric chloride or mercuric dibromide with cadmium carbonate or with stannic chloride, or preferably trimethylsilyltrifluoromethane sulphonate in toluene, acetonitrile, dichloromethane or 1,2-dichloroethane, as solvent followed by treatment as necessary with aqueous methanol (which also serves to remove the protecting groups from any hydroxyls on the pyrimidine ring).

Protecting groups may be removed by conventional techniques, for instance trimethylsilyl groups may be removed from hydroxyl groups on the pyrimidine ring by treatment with aqueous methanol, benzyl groups are removed from the hydroxyl groups on the 4-thio sugar compound by treatment with boron trichloride in dichloromethane at -78°C, and p-toluoyl groups are removed from the hydroxyl groups on the sugar by treatment with sodium methoxide in methanol at room temperature.

Alternatively the 5-halo substituent may be introduced into the pre-formed 5-unsubstituted 4′-thiopyrimidine nucleosides having protected or unprotected hydroxyl groups.

When the hydroxyl groups on the 4-thio sugar compound are protected (for instance with ethers such as silyl ethers or esters such as acetate, benzoate or p-toluate esters), reaction with N-chlorosuccinimide in glacial acetic acid or with chlorine and iodobenzene and glacial acetic acid will introduce a 5-chloro substituent and reaction with iodine monochloride in dichloromethane will introduce a 5-iodo substituent, while reacting the unprotected 4′-thio sugar pyrimidine nucleoside with chlorine in carbon tetrachloride and acetic acid also introduces the 5-chloro substituent. Reaction with iodine and nitric acid also introduces the 5-iodo substituent. Reaction with bromine and acetic acid introduces a 5-bromo substituent to the unprotected nucleoside. Deprotection where necessary is by conventional techniques and is performed as the final step.

The 5-unsubstituted 4′-thionucleoside starting material of formula (II) may be produced as described above by coupling a 5-unsubstituted pyrimidine to a 4-thio sugar compound. Protection of the hydroxy groups of the 4-thio sugar moiety may be effected at any convenient stage. may be effected at any convenient stage.

X is alkynyl

5-Alkynyl nucleosides may be produced by reacting a 5-iodo nucleoside wherein the hydroxyl groups of the 4-thio sugar are protected (for instance by reaction of the unprotected nucleoside with p-toluoylchloride in pyridine to introduce p-toluoyl ester groups on the hydroxyl groups of the 4-thio sugar) with trimethylsilyl acetylene or a terminal alkyne in the presence of bis(triphenylphosphine) palladium dichloride, cuprous iodide and triethylamine and, where necessary, removal of the protecting groups using sodium methoxide in methanol [c.f. M.J. Robin et al; Can. J. Chem., 60:554 (1982)].

Alternatively the 5-alkynyl group may be introduced by reacting a 5-iodo pyrimidine with trimethylsilylacetylene or a terminal alkyne in the presence of bis(triphenylphosphine)palladium dichloride, cuprous iodide, triethylamine and dimethylformamide followed, where necessary, by removal of the protecting groups and reacting the 5-alkynyl pyrimidine in suitably protected form (for instance the trimethylsilyl-protected form) with a protected 4-thio sugar compound as previously described followed by deprotection of the pyrimidine and sugar moieties as required.

X is alkenyl

5-Alkenyl compounds may be produced by partial hydrogenation on the corresponding 5-alkynyl substituted base or on the nucleoside for instance using Lindlar catalyst poisoned with quinoline, and subsequently, in the case of the base, coupling with a 4-thio sugar compound as described above.

Alternatively the 5-iodo nucleoside may be reacted with an appropriate 2-alkenoic acid ester (for instance the methyl ester) in the presence of palladium (II) acetate and triphenylphosphine to form the 5-(2-methoxycarbonyl alkenyl) derivative. The ester group is then removed by hydrolysis using sodium hydroxide forming the 2-carboxy alkenyl compound which itself is subjected to treatment with triethylamine in dimethylformamide at 100°C [c.f. S.G. Rahim et al., Nucleic Acids Research, 10(17):528(1982)].

Yet another method for producing the 5-alkenyl derivatives involves coupling the terminal alkene with a 5-iodo or 5-chloromercuri nucleoside (formed by reaction of the 5-unsubstituted nucleoside with mercury (II) acetate and sodium chloride), in the presence of a palladium salt such as palladium (II) acetate and a copper salt such as copper (I) chloride or preferably a palladium catalyst such as dilithium palladium tetrachloride. Reaction of a 5-iodo or 5-chloromercuri-nucleoside with allyl halides such as chloride or bromide in the presence of dilithium palladium tetrachloride leads to the formation of the corresponding 5-(alk-2-enyl) derivative which can be rearranged to form the 5-(alk-1-enyl) derivatives by treatment with tris(triphenylphosphine)rhodium chloride. The above processes are exemplified by J.L. Ruth & D.E. Bergstrom, J. Org. Chem, 43 (14): 2870 (1978), J. Goodchild et al., J. Med. Chem, 26: (1983), D.E. Bergstrom & J.L. Ruth, J. Am. Chem. Soc., 98: 1587 (1976) and D.E. Bergstrom & M.K. Ogawa, J. Am. Chem. Soc., 100: 8106 (1978).

X is 5-haloalkenyl

5-(Haloalkenyl) substituents may be introduced by conventional methods. For example, in order to prepare 5-(2-halovinyl) compounds the corresponding 5-(2-carboxyvinyl) nucleoside is treated with N-halosuccinimide in aqueous potassium acetate, or with potassium carbonate in dimethylformamide when the halogen is bromo or iodo. A 5-(2-chlorovinyl) nucleoside may aslo be made from the corresponding 5-(2-carboxyvinyl)nucleoside using chlorine gas in, for example, dimethylformamide.

Alternatively the 5-haloalkenyl group may be introduced into the free base which is subsequently coupled with a 4-thio compound as described above; this may be achieved by treating a 2,4-dimethoxy-protected 5-iodo-pyrimidine with an 2-alkenoic acid ester in the presence of palladium (II) acetate, triphenylphosphine and dioxane followed by removal of the methoxy protecting groups, hydrolysis of the ester with sodium hydroxide and reaction of the resulting 5-(2-carboxyvinyl) dervative with N-halosuccinimide (where halo is bromo or iodo) or chlorine gas (where halo is chloro) in the presence of a base such as sodium hydrogen carbonate in dimethylformamide. The 5-(2-carboxy vinyl) compound may also be produced by treating an unprotected 5-(hydroxymethyl)pyrimidine with an oxidising agent such as persulphate or manganese dioxide to form the corresponding aldehyde and followed by treatment of the aldehyde with malonic acid. The above processes are exemplifed by A.S. Jones et al, Tetrahedron Letts, 45; 4415 (1979) and P.J. Barr et al, J. Chem. Soc. Perkin Trans 1, 1981, 1665.

The 5-(2-haloalkenyl) base may alternatively be made by a novel route starting with a 2,4-dimethoxy protected 5-bromopyrimidine. This may be converted to the corresponding 5-lithium derivative by treatment with an organolithium reagent, preferably n-butyllithium at reduced temperature such as -70°C in an ethereal solvent such as diethylether. Reaction of the lithio derivative in situ with an appropriate ester of formic acid, such as ethyl formate at reduced temperature such as -70°C gives rise to the corresponding 5-formyl compound. Treatment of the formyl compound with malonic acid as described above give rise to the 5-(2-carboxyvinyl) derivative. Similar halogenation to the process described above gives rise to the required 5-(2-haloalkenyl) compound which is in the 2,4-dimethoxy protected from. Deprotection can then be carried out by conventional techniques.

5-Halovinyl compounds having more than one halogen substituent may be produced from a 5-unsubstituted 2,4-dimethoxy protected pyrimidine by reaction with a strong base such as butyl lithium and the resulting lithio derivative treated with the appropriate haloalkene followed by removal of the protecting groups and coupling to the 4-thio sugar compound as described above [c.f. P.L. Coe et al., J. Med. Chem. 25:1329 (1982)].

Alternatively, the halogen atoms may be introduced sequentially into the 5-substituent of the pyrimidine base. Thus for example treatment of 5-acetyl uracil with chlorinating agent such as phosphorus oxychloride provides the 5-(1-chlorovinyl) group with simultaneous chlorination of the pyrimidine hydroxyl groups. Treatment with potassium ethoxide then hydrogen chloride and finally bromine leads to bromination of the 5-unsaturated side chain of the pyrimidine base with simultaneous conversion of the 2,4-dichloro groups on the pyrimidine ring to form the corresponding uracil derivative. The resulting pyrimidine base can then be coupled to the 4-thio sugar compounds as described above [c.f. P.J. Barr et al. Nucleic Acids Res. 3: 2845 (1976) and P.J. Barr et al., J. Chem. Soc. Perkin Trans 1, 1981: 1665].

X is 5-haloalkyl

5-Fluoroalkyl substituents may be generated from the corresponding 5-hydroxyalkyl substituents, preferably starting from nucleosides having protected sugar hydroxyl groups on the 4-thio sugar moiety. Suitable protecting groups include tert-butyl diphenylsilyloxy groups which may be introduced using tert-butyldiphenylsilylchloride. The protected 5-hydroxyalkyl nucleoside is treated with a fluorinating agent such as diethylaminosulphurtrifluoride followed by deprotection of the hydroxyl groups using tetra-n-butylammonium fluoride to give the monofluoroalkyl derivative. Alternatively treatment of the 4′-thio sugar-protected 5-hydroxyalkyl nucleoside with manganese dioxide or pyridinium dichromate produces the corresponding aldehyde which may be treated with diethylamino- sulphur trifluoride. Treatment with tetra-n-butylammonium fluoride removes the protecting groups and liberates the 5-difluoroalkyl derivative.

Other 5-haloalkyl, for example haloethyl, substituents may also be generated using the corresponding 5-hydroxyalkyl substituents. The 5-hydroxyalkyl compound, in the form of either a base or a nucleoside is reacted with carbon tetrachloride and triphenylphosphate to introduce a chloro substituent, or with N-bromosuccinimide and triphenylphosphate to introduce a bromo substituent, or with N-bromosuccinimide, triphenylphosphine and tetrabutylammonium iodide to introduce an iodo substituent [c.f. J.D. Fissekis & F. Sweet, J. Org. Chem. 1973, 38, 264, and WO84/00759].

The above 5-hydroxyalkyl nucleoside starting materials where the alkyl group is a methylene are obtained from the corresponding 5-methyl-nucleosides by protection (for instance using tert- butyldiphenylsilylchloride) of the hydroxyl groups of the 4-thio sugar moiety, photolytic bromination (for instance, using bromine, N-bromosuccinimide in carbon tetrachloride) and hydrolysis of the bromoalkyl side chain using sodium bicarbonate.

X is alkyl

5-Alkyl eg. 5-ethyl substituted nucleosides are produced by hydrogenation of the corresponding 5-alkynyl or 5-alkenyl pyrimidine base followed by coupling to the 4-thio sugar compound. Conventional hydrogenation conditions, such as hydrogen over palladium/charcoal catalysts, may be adopted.

5-Trihaloalkyl substituents may be formed by displacement of a leaving group, for example iodo, in the 5-position of the suitably protected nucleoside by reaction with an appropriate trihaloalkylating agent In the case of the trifluoromethyl group, this may be introduced by reaction of the 5-iodo nucleoside with tritylchloride in pyridine to protect the hydroxyl groups of the sugar moiety followed by reaction with trifluoromethyl iodide in the presence of copper and deprotection using acetic acid [c.f. D. Bärwolf & D. Murawski, DDR Patent WP 113,361, 1973, J. Matulic-Adamic et al, J.Med.Chem., 1988, 31: 1642 (1988) & Y.Kobayashi et al, J.Chem. Soc., Perkin Trans 1, 1980: 2755]. 5-Trifluoromethyl uracil is commercially available and may be condensed with a 4-thio sugar compound in accordance with process B or E described above.

X is nitro or optionally substituted amino

Nitro-substituents are introduced at the 5-position of the 5-unsubstituted 4′thio-nucleosides by reaction with a nitrating agent for example nitronium tetrafluoroborate ($NO_2BF_4$), and these may be reduced using hydrogen over palladium/charcoal or tin (II) chloride to provide the corresponding amino substituent. [c.f. G-F. Huang and P.F. Torrence, J. Org. Chem., 42: 3821 (1977)]. 5-Nitro-substituted pyrimidines are readily available and may be coupled with 4-thio sugar compounds as described above. 5-Alkylamino and 5-dialkylamino substituents may be introduced by reacting a suitably protected 5-iodo-nucleoside with a corresponding alkylamine or dialkylamine. Protection is preferably by acylation for example by acetylation using acetic anhydride in pyridine.

X is alkoxy

Alkoxy substituents are introduced at the 5-position by reaction of the corresponding 5-hydroxy -4′-thio-nucleoside with a base, for example sodium hydroxide followed by alkylation with an appropriate alkyl halide in a suitable solvent such as methanol. The starting 5-hydroxy 4′-thio-nucleoside may be obtained from the 5-unsubstituted 4′-thio-nucleoside by treatment with bromine in an aqueous solvent such as aqueous tetrahydrofuran followed by treatment with base such as trimethylamine.

Alternatively, alkoxy substituents may be introduced by treatment of the corresponding 5-iodo-4′-thoinucleoside with an alkoxylating agent such as sodium alkoxide in an appropriate solvent such as methanol or dimethylformamide or the corresponding alkanol.

X is cyano

Cyano substituents are introduced at the 5-position by reaction of the corresponding 5-iodo 4′-thio-nucleoside with potassium cyanide in the presence of potassium acetate in a suitable solvent such as dimethylformamide, preferably at elevated temperature, for example 80°C-120°C, preferably 100°C [c.f. P.F. Torrence & B. Bhoosham J. Med. Chem., 20, 974 (1977)].

Other Process C) may be carried out using the following procedures to prepare compounds of formula (I) in which $R^2$ and $R^3$ have the following meanings include:-

a) $R^2$ and $R^3$ together form a carbon-carbon bond

Such compounds may be prepared from a corresponding 3′5′,-anhydro compound for example by treatment with a strong base eg. potassium tert-butoxide. Such 3′,5′-anhydro compounds may be prepared by treating the corresponding 3′,5′-methanesulphonate diester with a base.

For the preparation of compounds of formula (I) in which $R^2$ and $R^3$ are each hydroxy groups, in either the ribo or arabino configuration, the process of condensing a 4-thio-sugar with a base, as described above may be employed.

The 4-thio-sugar compound may be produced by conventional methods prior to coupling with the base or derived by modification of another sugar moiety which is already part of a nucleoside.

4-Thio-sugars

The 4′-thio-D-ribonucleosides may be obtained from the corresponding protected and C-1 activated 4-thioribofuranose compounds by conventional condensation procedures as described above. The starting 4-thioribofuranose sugar is obtained according to the procedure of Whistler et al., J. Org Chem., 29: 3723 (1964) or Reist et al., J. Amer. Chem. Soc., 86:5658 (1964), starting from α-L-lyxose.

The 4′-thio-β-D-arabinonucleosides may be obtained from the corresponding 4′-thioribonucleoside according to the procedure of Otatoni and Whistler, J. Med. Chem., 17, 535 (1974) via the $O^2$,2′-anhydride using conventional techniques. Unsaturated sugars (where $R^2$ and $R^3$ form a carbon-carbon bond) are produced by analogous methods to those described by J.P. Horowitz et al., in "Synthetic Procedures in Nucleic Acid Chemistry", Eds Zorbach and Tipson, Interscience, 1973 and P. Herdewijn et al., J. Med. Chem., 30:1270 (1987) starting from the 2′-deoxy-4′-thionucleosides.

The starting 4-thio-2-fluoro-β-D-arabinose may be obtained from the corresponding fully protected 4-thioribose sugar by partial deprotection at C2 followed by activation of the resulting free hydroxyl as imidazole sulphonate and fluorination with an appropriate fluorinating agent, for example potassium hydrogen fluoride and hydrogen fluoride in a suitable solvent such as 2,3-butane diol.

2′-Fluoro-4′-thio-β-D-arabinonucleosides may be obtained by condensation of the protected and C-1 activated 4-thio-2-fluoro-β-D-arabinose by conventional condensation procedures as described above [c.f. P.F. Bradfuehrer et al., J. Org. Chem., 50: 2597 (1985); C. H. Tann et al., J. Org. Chem., 50: 3644 (1985))].

The 2′-deoxy-3′-fluoro-4′-thioribonucleosides may be obtained by inversion of the 3′-hydroxyl group of the corresponding 2′-deoxy-4′-thionucleoside using an analogous procedure to that described by J.J. Fox and N.C. Miller (J. Org. Chem., 28, 936 (1963)) followed by fluorination using a suitable fluorinating agent such as diethylaminosulphurtrifluoride in a suitable solvent mixture such as dichloromethane/tetrahydrofuran (9:1) in an analogous manner to that described in J. Med. Chem., 32: 1743-1749 (1989).

1-(2-deoxy-2-fluoro-β-D-4-thioribofuranosyl)pyrimidines, for example 1-(2-deoxy-2-fluoro-β-D-ribofuranosyl)uracil may be produced in a manner analogous to that described by J.F. Coddington et al, J. Org. Chem. 29, 558 (1964).

The above reactions are all suitable for producing uracil nucleosides; most may also be used to form cytosine nucleosides. When this is not convenient or possible, cytosine analogues can be prepared most conveniently from the uracil compounds using an analogous procedure to that described by W.L. Sung, J. Chem. Soc. Chem. Commum., 1981, 1089]: for example the acetylated uracil nucleoside (produced for instance by reactions as described above and acetylated using acetic anhydride in pyridine) is treated with p-chlorophenylphosphorodichloridate, 1,2,4-triazole and pyridine to produce the 4-(1,2,4-triazol-1-yl) derivative which is then treated with ammonia in dioxane (which also removes the 4-thio sugar protecting group(s)) to form the corresponding unprotected cytosine 4′-thionucleoside.

The use of alkylamines instead of ammonia in the last step can produce the appropriate 4-N-alkylcytosine nucleosides.

The derivatives of the compounds of formula (I) may be prepared in conventional manner. For example, esters may be prepared by treating a compound of formula (I) with an appropriate esterifying agent, for example, an acyl halide or anhydride. Salts may be prepared by treating a compound of formula (I) with an appropriate base, for example an alkali metal, alkaline earth metal or ammonium hydroxide, or where necessary, an appropriate acid, such as hydrochloric acid or an acetate, eg. sodium acetate.

The anomers of compounds of the formula I may be separated by conventional means, for example by chromatography or fractional crystallisation.

Compounds of the formula (V) wherein $Z^2$ is hydrogen and $Z^3$ is a protected hydroxy group may be made by reference to our co-pending application, EP-A-90307820.2, the contents of which are incorporated herein by reference.

The invention is illustrated by the following non-limiting Examples. Examples A to E illustrate the production of intermediate compounds useful for the production of compounds according to the invention.

Example A

Preparation of Methyl 3,5-di-0-benzyl-2-deoxy-D-erythropentoside

To a solution of 2-deoxy-D-ribose (50g, 373 mmol) in dry methanol (900ml) was added a 1% solution of dry hydrogen chloride in methanol (100ml). The mixture was kept in a stoppered flask for 30 minutes after which the reaction was stopped by adding, with vigorous stirring, silver carbonate (10g). The mixture was filtered by gravity and the colourless filtrate evaporated to a syrup using a dry rotary evaporator. Residual methanol was then removed by repeated evaporation with dry THF. The syrup was then dissolved in dry THF (470ml). Under an atmosphere of dry nitrogen, at 0°C, with stirring sodium hydride in a 50% oil-dispersion (39.4g, 821 mmol) was slowly added to the THF mixture. Next, dry tetrabutylammonium iodide (30.3g, 82.1 mmol) was added followed by benzyl bromide (140g, 821 mmol), which was added over 1 hour. After stirring for 60 hours at room temperature, with exclusion of moisture, TLC (hexane-ethyl acetate [4:1]) showed almost complete conversion to two faster moving components ($R_f$ 0.47 and 0.36). The THF was removed

in vacuo, the residue dissolved in dichloromethane and then poured into ice/water. The dichloromethane solution was extracted from this mixture and then dried over magnesium sulphate. The dichloromethane was evaporated under reduced pressure and the resulting residue applied to a silica gel column eluted with hexane-ethyl acetate (4:1). Combination of the appropriate fractions gave the α (Rf0.36) and β ($R_f$0.47) isomers of the title product as a clear, colourless syrup

NMR SPECTRA

α - isomer

($^1$H) δ ($d_6$DMSO):7.56 - 7.17 (10H,d,aromatic), 5.12-5.00 (1H,q;H-1), 4.60-4.45 (4H,m, Ph$\underline{CH_2}$0), 4.40-3.86 (2H,m,H-3, H-4), 3.58-3.42 (2H,d,H-5), 3.40 (3H,s,$CH_3$),2.40-1.80 (2H,m,H-2).

($^{13}$C) δ ($CDCl_3$): 128.3-127.6 (aromatic), 105.2 (C-1), 82.1 (C-3 or C-4), 78.6 (C-3 or C-4), 73.4 (Ph$\underline{CH_2}$0), 71.5 (Ph$\underline{CH_2}$0), 70.2 (C-5), 55.1 (0Me), 38.9 (C-2).

β - isomer

($^1$H) δ ($d_6$DMSO):7.50 - 7.20 (10H,d,aromatic), 5.18-5.02 (1H,q,H-1), 4.65-4.43, (4H,d, Ph$\underline{CH_2}$0), 4.43-4.00 (2H,m,H-3,H-4), 3.60-3.42 (2H,m,H-5), 3.30 (3H,s,$CH_3$), 2.45-2.05 (2H,m,H-2).

($^{13}$C) δ ($CDCl_3$):128.3-127.6 (aromatic), 105.4 (C-1), 82.8 (C-3 or C-4), 80.0 (C-3 or C-4), 73.3 (Ph$\underline{CH_2}$0), 72.0 (Ph$\underline{CH_2}$0), 70.2 (C-5), 54.9 (OMe), 39.3 (C-2).

Preparation of 3,5-di-0-benzyl -2-deoxy-D-erythro-pentose dibenzyl dithioacetal

Concentrated hydrochloric acid (150ml) was added dropwise to a stirred mixture of methyl 3,5-di-0-benzyl-2-deoxy D-erythro-pentoside (77.5g, 236 mmol) and benzyl thiol (147g, 1.19 mol) at room temperature. The temperature was then raised to 40°C and the mixture stirred for 18 hours. At the end of this time TLC (hexane-ethyl acetate [4:1]) showed two faster moving minor components ($R_f$ 0.58 and 0.53), a major component ($R_f$0.29) and a slower moving minor component ($R_f$0.22). The mixture was dissolved in chloroform, poured into ice/water, neutralised with sodium hydrogen carbonate and extracted with chloroform. The chloroform extracts were dried over magnesium sulphate and the chloroform was evaporated under reduced pressure. The residue was applied to a silica gel column which was eluted with hexane-ethyl acetate (4:1). The first component to be eluted from the column, as a clear colourless syrup was the α, β anomers of benzyl 3,5-di-0-benzyl-2-deoxy-1-thio-D-erythro-pentofuranoside.

NMR SPECTRUM

($^1$H) δ ($d_6$DMSO):7.43-7.15 (15H,m,aromatic), 5.12-4.94 (1H,m,H-1), 4.59-4.36 (4H,m,Ph$\underline{CH_2}$0), 4.12-3.30 (6H,m,H-3,H-4,H-5,Ph$\underline{CH_2}$S), 2.35-1.35 (2H,m,H-2).

ELEMENTAL ANALYSIS

Found: C,74.4; H,6.5. $C_{26}H_{28}0_3S$ requires C,74.3; H,6.7%

MASS SPECTRUM

m/z 420 $M^+$, 297 $[M-SBn]^+$, 3-noba matrix.

The title product was the second component to be eluted from the column as a clear syrup (109g,85%).

NMR SPECTRUM

($^1$H) δ ($d_6$DMSO):7.35-7.05 (20H,m,aromatic), 4.97-4.95 (1H,d,0H-4), 4.47-3.95 (4H,m,Ph$\underline{CH_2}$0),3.81-3.66 (7H,m,H-1,H-3,H-4, Ph$\underline{CH_2}$S), 3.44-3.32 (2H,d,H-5), 2.10-1.83 (2H,m,H-2).

ELEMENTAL ANALYSIS

Found: C,73.0; H,6.5. $C_{33}H_{36}O_3S_2$ requires C,72.8; H,6.7%.

MASS SPECTRUM

m/z 298 $[M-2.SBn]^+$, 3-noba matrix.

SPECIFIC ROTATION

$[\alpha]_D^{25}$ = -101.8° (c1.2 in EtOH)

Preparation of 4-0-benzoyl-3,5-di-O-benzyl-2-deoxy-L-threo-pentose dibenzyl dithioacetal

To a solution of 3,5-di-O-benzyl-2-deoxy-D-erythro-pentose dibenzyl dithioacetal (54.1g,99.3 mmol),triphenylphosphine (39.1g, 149 mmol) and benzoic acid (18.2g, 149 mmol) in dry THF (800ml) was added a solution of DEAD (26.0g, 149 mmol) in dry THF (200ml) dropwise, with stirring, at room temperature.

After stirring at room temperature for 18 hours, TLC (hexane-ethyl acetate [4:1]) revealed a faster moving component (Rf 0.56) and slower moving starting material (Rf 0.36). The THF was removed in vacuo and the residue applied to a silica gel column eluted with hexane-ethyl acetate (85:15). Combination of the appropriate fractions gave the title product as a white solid. Starting material could also be recovered.

NMR SPECTRUM

($^1H$) δ ($d_6$DMSO):7.99-6.98 (25H,m,aromatic), 5.39-5.22 (1H,m,H-4), 4.54-4.04 (4H,m,Ph$CH_2$0), 4.01-3.62 (8H,m,H-1,H-3,H-5,Ph$CH_2$5), 2.17-1.84 (2H,m,H-2).

ELEMENTAL ANALYSIS

Found: C,74.3; H,6.4. $C_{40}H_{40}O_4S_2$ requires C,74.0; H,6.2%

MASS SPECTRUM

m/z 525 $[M-SBn]^+$, 435 $[M-2.SBn]^+$, glycerol matrix.

SPECIFIC ROTATION

$[\alpha]_D^{25}$ = 51.6° (c0.7 in $CH_2Cl_2$).

Preparation of 3,5-di-0-benzyl-2-deoxy - L - threo-pentose dibenzyl dithioacetal

To a solution of 4-0-benzoyl-3,5-di-0-benzyl-2-deoxy-L-threo-pentose dibenzyl dithioacetal (88.8g, 137 mmol) in dichloromethane (500ml) was added a solution of sodium methoxide (11.1g, 206 mmol) in methanol (205ml) dropwise, with stirring, at 0°C. The reaction mixture was then allowed to warm to room temperature over a period of 3 hours. At the end of this time TLC (hexane-ethyl acetate [4:1]) revealed complete conversion to a slower moving component (Rf 0.31). The mixture was then poured into a 5% solution of $NaH_2PO_4$ and extracted with dichloromethane. The dichloromethane extracts were then washed with a 5% solution of sodium hydrogen carbonate and water, dried (magnesium sulphate) and evaporated. The crude title product was applied to a silica gel column eluted with hexane-ethyl acetate (4:1). Combination of the appropriate fractions gave the title product as a clear colourless syrup.

NMR SPECTRUM

($^1H$) δ ($d_6$DMSO):7.34-7.06 (20H,m,aromatic), 4.88-4.86 (1H,d,0H-4), 4.55-4.00 (4H,m,Ph$CH_2$0), 4.83-3.32 (9H,m, H-1,H-3,H-4,H-5, Ph$CH_2$5), 2.08-1.84 (2H,m,H-2).

ELEMENTAL ANALYSIS

Found: C,72.6; H,6.9. $C_{33}H_{36}O_3S_2$ requires C,72.8; H,6.7%

MASS SPECTRUM

m/z 297 $[M-2.SBn + H]^+$, glycerol matrix.

SPECIFIC ROTATION

$[\alpha]_D^{25}$ = -75.6° (c1.9 in EtOH).

Preparation of 3,5-di-0-benzyl-2-deoxy-4-0-methanesulphonyl-L-threo-pentose dibenzyl dithioacetal

To a solution of 3,5-di-0-benzyl-2-deoxy - L - threo-pentose dibenzyl dithioacetal (61.4g, 113 mmol) in dry pyridine (700ml) was added methanesulphonyl chloride (19.4g, 169 mmol) in dry pyridine (200ml) dropwise, with stirring, at 0°C. The temperature of the mixture was raised to room temperature and stirring continued for 18 hours. The pyridine was then removed in vacuo and the residue dissolved in dichloromethane. The dichloromethane extracts were then successively washed with 2M hydrochloric acid, 1M sodium carbonate and water, dried (magnesium sulphate) and evaporated to give the title product as a thick viscous syrup. A sample of this was crystallised from hexane-ethyl acetate to give the title product as white crystals, m.p 82-83°C.

NMR SPECTRUM

($^1$H) δ ($d_6$DMSO): 7.64-6.89 (20H,m,aromatic), 4.88-4.64 (1H,m,H-4),4.60-4.09 (4H,m,Ph$CH_2$0), 4.05-3.43 (8H,m,H-1,H-3,H-5,Ph$CH_2$S), 3.11 (3H,s,$CH_3$), 2.12-1.80 (2H,m,H-2)

ELEMENTAL ANALYSIS

Found: C,65.5; H,6.1 $C_{34}H_{38}O_5S_3$ requires C,65.6; H,6.2%

MASS SPECTRUM

m/z 499 $[M-SBn]^+$, 393 $[M-SBn-0Bn + H]^+$, glycerol matrix.

SPECIFIC ROTATION

$[\alpha]_D^{25}$ = -58.4° (c2.4 in $CH_2Cl_2$).zz

Preparation of benzyl 3,5-di-0-benzyl-2-deoxy-1,4-dithio-D-erythro-pentofuranoside

A suspension of 3,5-di-0-benzyl-2-deoxy-4-0-methanesulphonyl L-threo-pentose dibenzyl dithioacetal (29.4g, 47.4 mmol), sodium iodide (74.0g, 494 mmol), barium carbonate (148g, 750 mmol) and dry acetone (1L) was boiled under reflux for 42 hours. At the end of this time the suspension was filtered and the solids were washed with chloroform. The filtrate was sequentially washed with water, sodium thiosulphate solution (5%) and water, dried (magnesium sulphate) and evaporated. The resultant residue was applied to a silica gel column, eluted with hexaneethyl acetate (9:1). Combination of the appropriate fractions gave the title product as a clear, slightly yellow, syrup, and recovered starting material.

NMR SPECTRA

($^1$H) δ ($d_6$DMSO):7.50-7.12 (1SH,m,aromatic), 4.66-4.13 (6H,m,H-1,H-4,Ph$CH_2$0), 4.09-3.35 (SH,m,H-3,H-5,Ph$CH_2$S), 2.44-1.94 (2H,m,H-2).

Major anomer

($^{13}$C) δ ($CDCl_3$):129.0-127.1 (aromatic), 83.04 (C-1), 73.1 (Ph$CH_2$0), 73.1 (Ph$CH_2$0), 71.0 (C-3), 53.2 (Ph$CH_2$S), 49.9 (C-4), 41.3 (C-5), 37.0 (C-2).

Minor anomer

($^{13}C$) δ ($CDCl_3$):129.0-127.1 (aromatic), 82.7 (C-1), 72.9 (Ph$\underline{CH_2}$0), 72.9 (Ph$\underline{CH_2}$0), 71.6 (C-3), 53.0 (Ph$\underline{CH_2}$S), 49.0 (C-4), 41.0 (C-5), 37.0 (C-2).

ELEMENTAL ANALYSIS

Found: C,71.8;H,6.7;S,14.4. $C_{26}H_{28}0_2S_2$ requires C,71.5;H,6.5;S,14.7%

MASS SPECTRUM

m/z 437 $[M+H]^+$, 345 $[M\text{-}Bn]^+$, 329 $[M\text{-}0Bn]^+$, 313 $[M\text{-}SBn]^+$, 223 $[M\text{-}SBn\text{-}Bn+H]^+$, glycerol matrix.

Preparation of 3′,5′-di-0-benzyl-4′-thio-thymidine and its α-anomer

A suspension of benzyl 3,5-di-0-benzyl-2-deoxy-1,4-dithio-D-erythro-pentofuranoside (22.5g, 51.6 mmol), bis TMS-thymine (46g, 170 mmol), mercuric bromide (20.5g, 56.7 mmol), cadmium carbonate (29.3g, 170 mmol) and dry toluene (1L) was boiled under reflux, with stiring, for 24 hours. The hot mixture was then filtered and the solids were washed with toluene. The filtrate was successively washed with potassium iodide solution (30%) and water and then evaporated. The residue was taken up in 4:1 methanol-water, stirred for 30 minutes, the suspension filtered and the filtrate evaporated. The residue was applied to a silica gel column (hexane-ethyl acetate (1:1)) and combination of the appropriate fractions gave the title product as a clear colourless syrup. $^1H$ NMR indicated the ratio of α- to β- anomers to be 2.8:1. Further column chromatography gave more of the separated anomers. The first compound to be eluted from the column was 3′,5′-di-O-benzyl-4′-thio-thymidine as a colourless syrup. This could be crystallised from methanol to give colourless crystals m.p. 140-142°C.

NMR SPECTRUM

($^1H$) δ ($d_6$DMSO):11.36 (1H,s,NH), 7.69 (1H,s,H-6),7.48-7.22 (10H,m,aromatic), 6.33-6.27 (1H,t,H-1′) 4.61-4.51 (4H,m,Ph$\underline{CH_2}$0), 4.30 (1H,s,H-3′), 3.76-3.66 (3H,m,H-4′,H-5′)2.42-2.32 (2H,m,H-2),1.66 (3H,s,$CH_3$).

UV SPECTRUM

max 269.1nm (ε,14,300).

ELEMENTAL ANALYSIS

Found: C,66.0;H,6.0;N,6.3. $C_{24}H_{26}N_20_4S$ requires C,65.7;H,6.0;N,6.4%

MASS SPECTRUM

m/z 439 $[M+H]^+$, 347 $[M\text{-}Bn]^+$, 331 $[M\text{-}0Bn]^+$, 3-noba matrix.
The next component to be eluted from the column was the α-anomer, as a colourless syrup.

NMR SPECTRUM

($^1H$) δ (d6DMSO): 11.28 (1H,s,NH), 7.95 (1H,s,H-6), 7.43-7.20 (10H,m, aromatic), 6.25-6.21 (1H,d,H-1′), 4.66-4.47 (7H,m, Ph C$\underline{H}_2$O), 4.25 (1H,s,H-3′), 4.10-4.06 (1H,m,H-4′), 3.57-3.42 (2H,m,H-5′), 2.68-2.26 (2H,m,H-2′), 1.55 (3H,s, $CH_3$).

UV SPECTRUM

Max 268.1 nm (ε, 10,900).

ELEMENTAL ANALYSIS

Found: C,65.4; H,6.1;N,6.7;S,7.4. $C_{24}H_{26}N_2O_4S$ requires C,65.7; H, 6.0; N 6.4; S,7.3%.

MASS SPECTRUM

m/z 439 $[M+H]^+$, 461 $[M+Na]^+$, 3-noba matrix.

Preparation of β -4′-thio-thymidine

To a 2M boron trichloride solution in dry dichloromethane (55ml) cooled to -78°C, was added a solution of β-3′,5′-di-O-benzyl-4′-thio-thymidine (1.6g, 3.7mmol) in dry dichloromethane (30ml). Stirring was continued for 5 hours at -78°C. This was then followed by the dropwise addition of a 1:1 methanol-dichloromethane solution (200ml) over 40 minutes. The reaction mixture was allowed to warm to room temperature over 1 hour and the solvent removed in vacuo and coevaporated with dry methanol (3 x 30 ml). The residue was applied to a silica gel column eluted with chloroform-methanol (85:15) to give the title title product. This could be crystallised from methanol to give colourless crystals m.p. 208-209°C.

NMR SPECTRUM

($^1H$) δ ($d_6DMSO$) 11.34 (1H,s,NH), 7.81 (1H,s,H-6), 6.32-6.26 (1H,t,H-1′), 5.26-5.25 (1H,d,OH-3′), 5.20-5.16 (1H,t, OH-S′), 4.40-4.35 (1H,m,H-3′) 3.18-3.16 (3H,m,H-4′, H-5 2.25-2.13 (2H,m,H-2′), 1.80 (3H,s,$CH_3$).

UV SPECTRUM

max 270.5nm (ε,10,300).

ELEMENTAL ANALYSIS

Found: C,46.2; H,5.3;N,10.6 $C_{10}H_{14}N_2O_4S$ requires C,46.5; H,5.5; N,10.9%.

MASS SPECTRUM

m/z 259 $[M+H]^+$, 3-noba matrix.

Preparation of benzyl 2-deoxy-1,4-dithio-3,5-di-O-p-toluoyl-D-erythro-pentofuranoside

To a 2M boron trichloride solution in dry dichloromethane (150ml) cooled to -78°C, was added a solution of benzyl 3,5-di-O-benzyl-2-deoxy-1,4-dithio-D-erythro -pentofuranoside (4.2g, 10mmol) in dry dichlormethane (100ml), dropwise, over 30 minutes. Stirring was continued for 5 hours at -78°C. This was then followed by the dropwise addition of a 1:1 methanol-dichloromethane solution (200ml) over 40 minutes. The reaction mixture was allowed to warm to room temperature over 1 hour and the solvent removed in vacuo and coevaporated with dry methanol (3x30ml). The crude residue was dissolved in dry pyridine (25ml), cooled to 0°C, and a solution of p-toluoyl chloride (4.6g, 30 mmol) in dry pyridine (25ml) added, dropwise, with stirring. The pyridine was removed in vacuo, the residue extracted with chloroform, and the extract successively washed with 2M hydrochloric acid, 1M sodium carbonate and water, dried (magnesium sulphate) and evaporated. The residue was applied to a silica gel column eluted with hexane-ethyl acetate (9:1) to give the title product as a clear, slightly yellow, syrup (2.5g, 53%).

NMR SPECTRUM

($_1H$) δ (d6DMSO): 7.94-7.25 (13H,m,aromatic), 5.68-5.62 (1H,m,H-1′) 4.74-4.66 (1H,m,H-3′), 4.39-3.83 (6H,m,H-3′,H-4′,H-5′, Ph$CH_2$S), 2.51-2.25(2H,m,H-2′), 2.39 (6H,s,$CH_3$).

ELEMENTAL ANALYSIS

Found: C,67.2;H,5.7. $C_{28}H_{28}O_4S_2$.1/2$H_2O$ requires C,67.0;H,5.8%.

MASS SPECTRUM

m/z 515 $[M+Na]^+$,401 $[M-Bn]^+$,369$[M-SBn]^+$,357 $[M-O_pTol]^+$, 3-noba matrix.

Preparation of E-5(2-bromovinyl-2′-deoxy-4′-thio-3′,5′di-O-p-toluoyl-uridine and its α -anomer

To a solution of benzyl 2-deoxy-1,4-dithio-3,5-di-O-p-toluoyl-D-erythro-pentofuranoside (1.4g,2.8 mmol) in carbon tetrachloride (15ml) was added a solution of bromine (0.49g, 3.1mmol) in carbon tetrachloride (15ml) with stirring at room temperature. After 5 minutes the mixture was concentrated under diminished pressure and then carbon tetrachloride (5ml) was added and the mixture was evaporated to remove the excess bromine. The evaporation procedure was repeated four times. The resulting syrupy bromide was unstable and was used directly in the next step.

To a solution of the bromide in carbon tetrachloride (10ml) was added the bis TMS-derivative of E-5-(2-bromovinyl)uracil (1.7g, 4.7mmol) in carbon tetrachloride (10ml). The mixture was stirred until homogenous, evaporated and the residue heated for 1 hour at 90-100°C. The cooled, dark residue was dissolved in 4:1 methanol-water (30ml), the solution boiled for 15 minutes under reflux and then evaporated. The residue was triturated with chloroform (40ml) and the solid 5-(2-bromovinyl) uracil that separated filtered off. The filtrate was successively washed with aqueous sodium hydrogen carbonate and water, dried (sodium sulphate) and evaporated. The residue was applied to a silica gel column eluted with hexane-ethyl acetate (3:2). Combination of the appropriate fractions gave the title product as a white solid. 1H NMR indicated the ratio of α - to β -anomers to be 1.8:1. Further column chromatography (chloroformpropan-2-ol (98:1)) gave more separated anomers. The first compound to be eluted from the column was E-5-(2-bromovinyl)-2′-deoxy-4′-thio-3′5′di-O-p-toluoyl-uridine which could be crystallised from methanol to give colourless crystals m.p. 182-184°C.

NMR SPECTRUM

$(^1H)\delta(d_6DMSO)$: 11.73 (1H,s,NH),8.10 (1H,s,H-6), 7.94-7.86 (4H,m,aromatic), 7.39-7.19(SH,m, aromatic and vinylic H), 6.89 (1H,d,vinylic H,J=14Hz), 6.45-6.40 (1H,t,H-1′), 5.85-5.80 (1H,m,H-3′), 4.71-4.53 (2H,m, H-5′), 4.00-3.92 (1H,m, H-4′), 2.83-2.50 (2H,m, H-2′), 2.39 (6H,s, $CH_3$).

UV SPECTRUM

max 241.6nm (ε,34,960), 296.9nm (E,10,100
min 271.4nm (ε,7,700).

MASS SPECTRUM

m/z 586 $[M+H]^+$, thioglycerol matrix.
The next component to be eluted from the column was the α - anomer which could be crystallised from methanol to give colourless crystals m.p.176-172°C.

NMR SPECTRUM

$(^1H)\delta(d_6DMSO)$ 11.64 (1H,s,NH),8.36 (1H,s,H-6), 7.91-7.77 (4H,m,aromatic), 7.36-7.22 (5H,m,aromatic, vinylic H) 6.80 (1H,d,vinylic H, J=5Hz), 6.29-6.27 (1H,d,H1′) 5.74-5.62 (1H,m,H-3′)4.48-4.39 (3H,m,H-4′,H-5′), 2.94-2.85 (2H,m,H-2′), 2.37 (6H,s,$CH_3$).

UV SPECTRUM

max 241.6nm (ε,47,000) 296.1nm (ε,12,500).
min 273.1nm (ε,10,000)

ELEMENTAL ANALYSIS

Found C,54.4; H,4.4;N,4.5. $C_{27}H_{25}BrN_2O_6S$1/2$H_2O$ requires C,54.6;H,4.2;N,4.7%.

MASS SPECTRUM

m/z 586 $[M+H]^+$, thioglycerol matrix.

EXAMPLE B

3′,5′-Di-O-benzyl-2′-deoxy-5-iodo-β-4′-thiouridine

Mercuric bromide (370 mg; 1.03 mmol) and cadmium carbonate (480 mg; 2.8 mmol) were added to a stirred solution, protected from moisture, of benzyl 3,5-di-O-benzyl-2-deoxy-1,4-dithio-D-erythro-pentofuranoside (436 mg; 1.0 mmol) in dry MeCN (3 ml). A solution of 5-iodo-bis-O-trimethylsilyluracil (3 mmol) in MeCN (12 ml) was added via syringe. The progress of the reaction was monitored by analytical HPLC while the mixture was heated under reflux for 1 h. When cooled to ambient temperature, water (200 μl) was added and after stirring for 30 min. the suspension was filtered. The filtrate was evaporated and redissolved in dry MeCN, the precipitated 5-iodouracil was removed by filtration. The filtrate was purified by preparative HPLC on a 2.5 cm (1 in.) Zorbax C8 reverse phase column eluted at 20 ml $min^{-1}$ with a gradient [0 - 95% MeCN-water containing a constant 0.2% trifluoroacetic acid] over 20 min.; half-minute fractions were collected. Fractions containing pure product were pooled and evaporated to yield 330 mg of product as a gum. The anomer ratio was 2.8: 1,α:β as determined by 200 MHz $^1H$-NMR. [$CDCl_3$ δ :8.72 (s, 0.26H, β-6-H); 8.55 (s, 0.74H, α-6-H); 6.35 (t, 0.26H, β-1′-H); 6.24 (dd , 0.74H, α-1′-H)]

EXAMPLE C

3′5′-Di-O-benzyl-5-bromo-2′deoxy′-β-4′-thiouridine.

This compound was prepared by a method similar to the iodo compound above with the following modifications:

1. The total solvent (MeCN) volume for the reaction was 3 ml.
2. The $CdCO_3$ was omitted.
3. The excess of 5-bromo-bis-O-trimethylsilyuracil was reduced to 1.5 mole equivalents.

The yield of HPLC-purified compound was 193 mg; mass spectrum gave m/z 502 expected for $C_{23}H_{23}BrN_2O_4S$.

EXAMPLE D

1-Acetoxy-3,5-di-p-toluoyl-2-deoxy-4-thio-D-erythro-pentofuranoside

A solution of benzyl 3,5-di-O-benzyl-2-deoxy-1-4-dithio-D-erythro-pentofuranoside (3.68 g; 8.76 mmol) in dry $CH_2CH_2$ (20ml) was added dropwise to a stirred 1M solution of $BCl_3$ in $CH_2Cl_2$ (125 ml; 0.125 ml; 0.125 mol) at 78°C under $N_2$. The mixture was stirred at -78°C for 4.5h, then a mixture of MeOH-$CH_2Cl_2$(1:1,v/v) was added slowly. After warming to room temperature the solvents were evaporated to give the crude O-debenzylated thiosugar. The gum was dissolved in dry pyridine at 0°C under $N_2$ and a solution of p-toluoyl chloride (3.47 ml; 26.3 mmol) was added slowly. The mixture was stirred at O°C for 3 hours then the solvents were evaporated. The residue was dissolved in $CH_2Cl_2$, washed with 2M HCl, 1M $Na_2CO_3$ and water, dried over $MgSO_4$ and evaporated. The residue was purified by flash chromatography on $SiO_2$ eluted with EtOAc-hexane (1:9, v/v) to give the bis-toluoylthiosugar derivative (2.18 g;ca.50%): mass spectrum m/z 492. This product was dissolved in acetic anhydride (16 ml) and stirred at O°C. Conc. $H_2SO_4$ (8 μl) was added followed after 10 min. by a second aliquot (8 μl); the reaction was monitored by TLC. After a further 55 min. stirring $NaHCO_3$ (100 mg.) was added and after 20 min. the mixture was cautiously poured into ice-water containing $NaHCO_3$. The product was extracted into $CH_2Cl_2$, dried and evaporated. The residue was purified by flash chromatography on $SiO_2$ eluted with 20-25% EtOAc-hexane. Yield 0.97g:- 200 MHz $^1H$ NMR DMSO-d6, δ:7.7-8.1(m,4H,ArH); 7.1-7.4(m, 4H + solvent, ArH); 6.35 (dd,0.55H, 1-H);6.27(q,0.45H, 1-H);5.7-5.9 (m,1H, 3-H); 3.7-4.7(m,3H,5-$H_2$+4-H);2.2-2.7(m+2.2-2.7(m+2xs, 8H, 2x$ArCH_3$ + 2-$H_2$); 2.0-2.1 (2xs, 3H, $CH_3CO$-α & β). The anomer ratio was approximately 1.1/1; the material was sufficiently pure for use.

EXAMPLE E

Preparation of E-5-(2-bromovinyl)Uracil

5-Bromo-2,4-dimethoxypyrimidine

A solution of 5-bromo-2,4-dichloropyrimidine (16 g: 70.2 mmol) [D.M. Mulvey et al. J. Het. Chem., 1973,p79] in dry MeOH (55 ml) was added slowly to a stirred solution of sodium (3.23 g: 140.4 mmol) in MeOH (55 ml) at O°C over 30 min. The ice-bath was removed and the reaction mixture stirred at ambient temperature for 18 h. The precipitated salt was removed by filtration and the filtrate evaporated to give an oil. To this was added an aqueous solution of NaOH (30 ml; 30% w/v); the product separated as an upper layer and was extracted into $Et_2O$. The organic extracts were dried over $MgSO_4$ and evaporated. The residue was crystallised from thanol to give the product as colourless plates, yield 14.3 g, 93%, mp 62-63°C. Mass spectrum, elm/z 219 ($M^+$, 11%). Analysis, found: C,33.20, H,3.26, Br 36.90, N, 12.7%; $C_6H_7BrN_2O_2$ requires: C,32.90, H,3.33, Br 36.50, N, 12.80%.

5-Formyl-2,4-dimethoxypryrimidine

A solution of 1.6 M n-Buli in hexane (48 ml, 73.6 mmol) was added over 5 min. to a stirred suspension of 5-bromo-2,4-dimethoxypyrimidine (16 g; 72.9 mmol) in dry $Et_2O$ (240 ml) at -70°C under an atmosphere of dry $N_2$. Dry ethyl formate (28 g: 377 mmol) was added and the orange solution stirred at
-70°C for 1 h then allowed to warm slowly to ambient temperature. Water (400 ml) was added and the aqueous layer separated and extracted with $Et_2O$ (3 x 200 ml). The ether layer was combined with the extracts and dried over $MgSO_4$, filtered and evaporated. The residue was purified by column chromatography by preloading in $SiO_2$ and eluting with EtOAc-hexane (3:7, v/v). Product fractions were combined and evaporated to give fine white needles, yield 6.89 g, (56%). Mass spectrum m/z 169 $(M+H)^+$ Analysis, found: C, 50:1;H,4.5;N,16.9%;$C_7H_8N_2O_3$ requires C,50.00; H,4.79; N, 16.66%

E-5-(2-carboxyvinyl)-2,4-dimethoxypyrimidine

Malonic acid (13.03 g; 126.2 mmol) and redistilled piperidine ( 2ml) were added to a solution of 5-formyl-2,4,dimethoxypyrimidine (10.52 g; 6.2.6 mmol) in dry pyridine (60 ml). The mixture was heated on a steam bath for 10 h then the solvent was removed by distillation under reduced pressure. The residual oil was re-evaporated from water (3 x 25 ml) and the solid thus obtained recrystallised firstly from water and then from dry methanol to give the product as white needles, yield 6.45 g; a second crop was obtained from the filtrate (1.08 g). Total yield 7.53g (57%). Mass spectrum: (El) m/z 210 ($M^+$). Analysis, found: C,52.1;H,4.8;N, 13.1%: $C_9H_{10}N_2O_4$ requires: C, 52.43; H, 4.79; N, 13.33%.

E-5-(2-Bromovinyl)-2,4-dimethoxypyrimidine

To a solution of E-5-(2-carboxyvinyl)-2,4-dimethoxypyrimidine (0.300 g; 1.43 mmol) in dry DMF (5 ml) was added $K_2CO_3$ (0.45 g: 5.25 mmol). After stirring at ambient temperature for 15 min. a solution of N.-bromosuccinimide (0.258 g; 1.45 mmol) in dry DMF (4 ml) was added dropwise over 10 min. The suspension was immediately filtered, the solid washed with DMF and the filtrate evaporated in high vacuum. The solid residue was purified by column chromatography by preloading on $SiO_2$ and eluting with EtOAc-hexane (7:3, v/v). Product fractions were pooled and evaporated to give fine white crystals, yield 0.561 g (45%). FAB mass spectrum: m/z245 and 247 $(M+H)^+$. Analysis, found: C,39.9; H, 3.6; N, 11.5% $C_8H_9BrN_2O_2$ requires C, 40.20; H, 3.70; N. 11.43%.

E-5-(2-Bromovinyl)uracil

To a solution of E-5-(2-bromovinyl)-2,4-dimethoxypyrimidine (2.45 g; 10 mmol) in AcOH (10 ml) was added NaI (3.3 g; 2.2 eq.; 22 mmol) and the solution heated under reflux for 3h. The hot mixture was filtered and diluted with water (15 ml). After cooling, the precipitated product was filtered off, washed with acetone (50 ml) and ether (20 ml) and dried to give a pale yellow powder (1.40 g, 65%). Mp >320°C; 60 MHz $^1$H-NMR, DMSO-6d, δ: 7.60 (s, 1H, H-6); 7.30 (d, 1H, J=13 Hz, vinyl H); 6.80 (d, 1H, J=13 Hz, vinyl H).

The invention is illustrated by the following examples.

**EXAMPLE 1**

i) E-5-(2-Bromovinyl)-4′-thio-2′,3′,5′-tri-O-acetyl uridine

E-5-(2-bromovinyl)uracil (0.49 g, 2.27 mmol) and ammonium sulphate (0.005 g) were stirred in refluxing hexamethyldisilazane (8 ml) until dissolution (3 hrs). The solvent was evaporated, the residual oil dissolved in dry acetonitrile (10 ml) and added under rigorous exclusion of moisture to a solution of 4-thio-D-ribose 1,2,3,5-tetracetate (synthesised from L-Lyxose using a literature procedure E.J. Reist, D.E. Gueffroy & L. Goodman, J.Am. Chem. Soc., 1964,86,5658) (0.5 g, 1.5 mmol) in dry acetonitrile (10 ml). The mixture was stirred under nitrogen at 0°C and tin (IV) chloride (0.2 ml, 1.73 mmol) was added. The reaction mixture was then allowed to warm up to room temperature and stirred for 1 hour. The solution was diluted with dichloromethane (20 ml) and the mixture quenched by the addition of a saturated solution of sodium bicarbonate. The organic layer was separated, washed with water, dried ($Na_2SO_4$) and evaporated to dryness. Purification by column chromatography ($SiO_2$) in 1:1 to 2:1 ethylacetate/hexane afforded the title compound contaminated with 8% of thio-α-anomer.

E I mass spectrum: observed m/z 490 for $C_{17}H_{19}BrN_2O_8S$ 1H 200 MHz nmr $CDCl_3$ 8.23 (1H, bs, NH), 7.92 (s, H-6 of α-anomer), 7.7 (1H,s, H-6), 7.45 (1H, d, vinylic H, J = 14 Hz), 0.74 (1H, d, vinylic H, J = 14 Hz), 6.47 (d, H-1′ of α-anomer, $J_{1',2'}$ = 5Hz), 6.38 (1H,d, H-1′, $J_{1'2'}$ = 7.5 Hz), 5.49 (1H, m, H-2′), 5.4 (1H, m, H-3′), 4.56-4.28 (2H, m, H-5′), 3.68 (1H, m, H-4′), 2.2-2.0 ppm (9H, 3 x s, acetyl H).

ii) E-5-(2-Bromovinyl)-4′-thiouridine

E-5-(2-bromovinyl)-4′-thio-2′,3′-5′-tri-O-acetyluridine from the previous reaction (0.4 g, 0.8 mmol) was added to a solution of sodium methoxide (0.8 mmol) in dry methanol (5 ml) and the mixture was stirred at room temperature for 4 hours. The solution was neutralised with Dowex 50 ($H^+$) resin, the resin filtered, washed with methanol and the combined filtrate and washings evaporated to dryness to give the crude product. Recrystallisation from ethanol gave a pure sample of the title compound containing 4.5% of the α-anomer.

M.pt - decomposes at 192°C.

1H 200 Mhz nmr $d_6$DMSO: 11.55 (1H, bs, NH), 8.23 (1H, s, H-6) 7.99 (s, H-6 of α-anomer), 7.3 (1H, d, vinylic H, J = 14 Hz), 6.96 (1H, d, vinylic H, J = 14 Hz), 6.09 (d, H-1′ of α-anomer $J_{1',2'}$ = 5 Hz), 5.89 (1H, d, H-1′ $J_{1'2'}$ = 7.5 Hz), 5.55-5.15 (3H, m, OH-2′, OH-3′, OH-5′), 4.2 (1H, m, H-2′), 4.05 (1H, m, H-3′), 3.65 (2H, m, H-5′) 3.18 ppm (1H, m, H-4′).

E1 Mass spectrum: observed m/z 364 for $C_{11}H_{13}BrN_2O_5S$

| Microanalysis: Calculated for $C_{11}H_{13}BrN_2O_5S.0.66\ H_2O$ | | | |
|---|---|---|---|
| | C,35.02; | H,3.799; | N,7.43% |
| Found | C,35.00; | H,3.90; | N.7.45% |

**EXAMPLE 2**

i) 5-Ethynyl-4′-thio-2′,3′,5′-tri-O-acetyluridine

Using 5-ethynyluracil, this compound was prepared in an analogous manner to example 1(i). Chromatographic separation of the final product allowed isolation of thio α- and β-anomers in a crude form.

α-anomer: 1H 200 MHz nmr $d_6$DMSO: 11.65 (1H, bs, NM), 8.2 (1H, s, H-6), 6.23 (1H, d, H-1′), 5.05 (1H, m, H-2′), 4.45 (1H, m, H-3′), 4.4-4.15 (2H, m, H-5′), 4.08 (1H, s, ethynyl H), 4.0 (1H, m, H-4′), 2.07-1.96 ppm (9H, 3 x S, acetyl H).

β-anomer: 1H 200 MHz nmr $d_6$DMSO: 11.75 (1H, bs, NH), 8.38 (1H, s, H-6), 6.11 (1H, d, H-1′), 5.7 (1H, m, H-2′), 5.44 (1H, m, H-3′), 4.5-4.2 (2M, m, H-5′), 4.19 (1H, s, ethynyl H), 3.68 (1H, m, H-4′), 2.2-2.0 (9H, 3 x S, acetyl H).

ii) 5-Ethynyl-4′-thiouridine

Deprotection of the individual anomers of the compound of (i) above in an analogous manner to Example 1(ii) afforded the α- and β-anomers of the title compound.

α-anomer: M.pt. decomposes at 217°C

El mass spectrum: observed m/z 284 for $C_{11}H_{12}N_2O_5S$

1H 200 MHz nmr $d_6$DMSO: 11.55 (1H, bs, NH), 8.33 (1H, s, H-6), 6.1 (1H, d, H-1′), 5.65 (1H, d, OH-2′) 5.3 (1H, d, OH-3′), 4.93 (1H, t, OH-5′), 4.13 (1H, m, H-2′), 4.07 (1H, s, ethynyl H), 3.94, (1H, m, H-3′), 3.8-3.5 (2H, m, H-5′) 3.9 ppm (1H, m, H-4′).

β-anomer: M.pt. 230-5°C (decomp.)
EI Mass Spectrum: observed m/z 284 for $C_{11}H_{12}N_2O_5S$
1H 200MHz nmr $d_6$DMSO: 11.6 (1H, bs, NH), 8.4 (1H, s, H-6), 5.86 (1H, d, H-1′), 5.5 (1H, d, OH-2′) 5.7 (2H, m, OH-3′ and OH-5′), 4.02 (1H, m, H-2′), 4.13 (1H, s, ethynyl H), 4.0 (1H, m, H-3′), 3.62 (2H, m, H-5′), 3.2 ppm (1H, m, H-4′).

**EXAMPLE 3**

i) 5-Iodo-4′-thio-2′,3′,5′-tri-O-acetyluridine

Using 5-iodouracil, this compound was prepared in an analogous manner to Example 1(i).

1H 200 MHz nmr $CDCl_3$: 8.58 (1H, bs, NH), 8.23 (1H, s, H-6), 6.33 (1H, d, H-1′), 5.55 (1H, m, H-2′), 5.4 (1H, m, H-3′) 4.55-4.15 (2H, m, H-5′), 3.7 (1H, m, H-4′), 2.3-2.05 ppm (9 H, 3 x s, acetyl H).

ii) 5-Iodo-4′-thiouridine

Deprotection of the compound of (i) above in an analogous manner to Example 1(ii) afforded the title compound.

M.pt. Decomposes at 227°C 1H 200 MHz nmr $d_6$DMSO: 11.65 (1H, bs, NH), 8.48 (H1, s, H-6), 5.85 (1H, d, H-1′), 5.48 (1H, d, OH-2′), 5.25 (1H, t, OH-5′), 5.2 (1H, d, OH-3′), 4.2 (1H, m, OH-2′), 3.99 (1H, m, H-3′), 3.62 (2H, m, H-5′), 3.2 ppm (1H, m, H-4′).

E1 Mass Spectrum: observed m/z 386 for $C_9H_{11}N_2IO_5S$

**EXAMPLE 4**

5-(2-Chloroethyl)-2′-deoxy-4′thiouridine

5-(2-chloroethyl)uracil (prepared by the method J.D. Fissekis & F. Sweet, J-Org. Chem., 1973, 28, 264(0.122 g, 0.7 mmol) was added to hexamethyldisilazane (3 ml) and chloromethylsilane (0.1 ml) and the mixture was heated at reflux for 2 hours. The mixture was evaporated to dryness and to the residue was added a solution of 1-acetoxy-3,5-di-O-p-toluoyl-2-deoxy-4-thio-D-erythropentofuranoside (See Example D) (0.2 g, 0.46 mmol) in dry dichloromethane (10 ml), the mixture cooled to 0°C with stirring and trimethylsilyltrifluoromethanesulphonate (0.1 ml) was added. After stirring at 0°C for 2 hours, dichloromethane (30 ml) was added and the reaction was quenched with a saturated solution of sodium bicarbonate (20 ml). The aqueous phase was extracted with dichloromethane (2 x 25 ml) and the combined organic phases dried ($Na_2SO_4$) and evaporated to dryness to give the crude product in the p-toluoyl protected form. This intermediate (0.23 g) was added to a solution of sodium methoxide (0.9 mmol) in dry methanol (20 ml) and the mixture stirred at room temperature overnight. The solution was neutralised with Dowex 50 ($H^+$) resin, the resin filtered and washed with methanol. The combined filtrate and washings was evaporated to dryness, the residue partitioned between ether and water, the organic layer re-extracted with water and the combined aqueous phases evaporated to dryness. The residue was chromatographed on silica gel eluting with 7% methanol/dichloromethane, the product freeze dried from water to give the title compound as a mixture of anomers in the ratio β/α ca. 1:1.25.

$^1$H200 MHz nmr in $d_6$-DMSO: 11.4 (1H, m, NH), 8.2 (0.55H, s, H-6 of α-anomer), 7.93 (0.44 H, s, H-6 of β-anomer), 6.26 (0.44H, s, H-1' of β-anomer), 6.15 (0.55H, dd, H-1' of α-anomer), 4.38 (0.44H, m, H-3' of β-anomer), 4.27 (0.55 H, m, H-3' of α-anomer), 3.7 (2 H, m, $\underline{CH_2}CH_2Cl$), 3.67-3.45 (2H, m, H-5'), 3.3 (1H, m obscured by solvent, H-4'), 2.69 (2H, m, $CH_2\underline{CH_2}Cl$), 2.25-2.0 ppm (2H, m, H-2').

EI Mass Spectrum: observed m/z 306 for $C_{11}H_{15}ClN_2O_4S$

| Microanalysis: Calculated for $C_{11}H_{15}ClN_2O_4S.0.5H_2O$ | | | |
|---|---|---|---|
| | C,41.83; | H, 5.07; | N,8.87% |
| Found: | C,41.78; | H, 4.94; | N.8.83 % |

**EXAMPLE 5**

**2'-Deoxy-5-nitro-4'-thiourdine**

A solution of 5-nitrouracil-2,4-bis-trimethylsilyl ether [from 5-nitrouracil (118 mg, 0.75 mmol) and hexamethyldisilazide (3 ml)-chlorotrimethylsilane (3 drops), reflux 1 h.] in dry $CH_2Cl_2$ (6 ml) was added to a solution of 1-acetoxy-3,5-di-p-toluoyl-2-deoxy-4-thio-D-erythro-pentofuranoside (200 mg, 0.5 mmol) in dry $CH_2Cl_2$ (10 ml). The stirred mixture

was cooled in an ice-bath and freshly distilled trimethylsilyl trifluoromethanesulphonate (96 µl) added. After 30 min. at 0°C the mixture was poured into saturated $NaHCO_3$ (50 ml), the layers separated and the aqueous phase extracted with $CH_2Cl_2$. The combined $CH_2Cl_2$ phase was dried over $MgSO_4$, then evaporated and the residue triturated with ether to leave the crude protected nucleoside as a white solid. Deprotection was accomplished by adding a solution of sodium (20 mg) in MeOH (1 ml) to a suspension of the product (140 mg) in MeOH (10 ml). The solid dissolved within 5 min. stirring at ambient temperature. After 2 hours the solution was neutralised with Dowex 50X8 H+, filtered and evaporated. The residue was triturated with ether and the residue purified by HPLC on Zorbax C8 reversed phase eluted with MeCN-$H_2O$ (1:9, v/v). The pure β-anomer was isolated by freeze drying appropriate fractions. 200 MHz 1H-NMR, DMSO-d6, δ: 12.0 (br s, 1H, NH); 9.55 (s, 1H, H6); 6.13 (t, 1H, 1′-H); 5.25 (m, 2H, 2 x OH); 4.3 (m, 1H, 3′-H); 3.65 (m, 2H, 5′-H); 3.1-3.4 (m, 4′-H obscured by DOH); 2.3 (t, 2H, 2′-H). FAB mass spectrum showed $(M+H)^+$ at 290 and $(M+Na)^+$ at 311 for $C_9H_{11}N_3O_6S$.

## EXAMPLE 6

E-5-(2-Bromovinyl)-1-(4-thio-β-D-arabinofuranosyl)uracil

E-5-(2-Bromovinyl)-4′-thiouridine (0.196 g, 0.54 mmol) was dissolved in dry pyridine (2 ml) and 1,3-dichloro-1,1,3,3-tetraisopropyldisiloxane (0.24 ml, 0.74 mmol) was added and the mixture was stirred at room temperature overnight. The solvent was removed by evaporation under reduced pressure and the residual oil was chromatographed on a silica gel column eluting with 5% $MeOH/CH_2Cl_2$ to yield the 3′,5′-disiloxanyl derivative. This was dissolved in dry pyridine (5 ml) at 0°C and methanesulphonyl chloride (0.04 ml, 0.48 mmol) was added and the mixture was stirred at room temperature overnight. The reaction mixture was cooled in an ice/water bath and quenched with water. The solvent was removed by evaporation under reduced pressure. The residue was dissolved in dichloromethane (10 ml) and washed with water (10 ml). The aqueous layer was extracted with dichloromethane (10 ml), and the combined organic phases were dried ($Na_2SO_4$) and evaporated to dryness. The residue was co-evaporated with several portions of dichloromethane then toluene to remove traces of pyridine to give the 3′,5′-disiloxanyl-2′-mesylate derivative. The resulting foamy solid was dissolved in 50% aqueous ethanol (20 ml) and potassium carbonate (58 mg, 0.4 mmol) was added and the mixture was heated at 80°C for ½ hour. The reaction mixture was neutralised with Dowex 50($H^+$) resin, the resin was filtered, washed with ethanolol and the combined filtrate and washings were evaporated to dryness. The residue was partitioned between dichloromethane and water, the aqueous layer was extracted with dichloromethane (2 x 20 ml), the combined organic extracts were dried ($Na_2SO_4$) and evaporated to dryness. Purification by column chromatography on silica gel eluting with 5% $MeOH/CH_2Cl_2$ gave the 2′-arabinoside-3′,5′-disiloxanyl derivative. This was dissolved in dry tetrahydrofuran (10 ml) and tetrabutylammonium fluoride, trihydrate (80 mg, 0.25 mmol) was added. After stirring at room temperature for ½ hour, the solvent was removed by evaporation and the residue was purified by column chromatography (silica gel), eluting with 10%$MeOH/CH_2Cl_2$. The appropriate fractions were combined, evaporated to dryness to give a pure sample of the title product.
Melting point starts to decompose at 165°C.
200 MHz $^1H$ NMR, $d_6$-DMSO:- 11.55 ppm (1H, bs, N-H); 8.39 (1H, s, H-6); 7.25 (1H, d, vinylic H, J = 14 Hz); 6.9 (1H, d, vinylic H, J = 14 Hz); 6.08 (1H, d, H-1′ $J_{1',2'}$ = 5Hz); 5.75 (1H, d, OH-2′); 5.45 (1H, d, OH-3′); 5.35 (1H, t, OH-5′); 3.9 - 4.1 (2H, m, H-2′ & H-3′); 3.75 (2H, m, H-5′); 3.15 (1H, m, H-4′).
EI Mass Spectrum: m/z: M, -Br 285; M, -base 149; base, +H, -Br 137; M -base, -$H_2O$ 131.

BIOLOGICAL DATA

a) Anti-HSV Activity

Herpes Simplex Virus types 1 (HSV 1) and 2 (HSV2) were assayed in monolayers of Vero cells in multiwell trays. The virus strains used were SC16 and 186 for HSV-1 and HSV-2 respectively. Activity of compounds was determined in the plaque reduction assay, in which a cell monolayer was infected with a suspension of the appropirate HSV, and then overlaid with nutrient agarose in the form of a gel to ensure that there was no spread of virus throughout the culture. A range of concentrations of compound of known molarity was incorporated in the nutrient agarose overlay. Plaque numbers at each concentration were expressed as percentages of the control and a dose-response curve was drawn. From this curve the 50% inhibitory concentration ($IC_{50}$) was estimated to be 0.66 µm for the compound of formula (I) in which X represents a 2-bromovinyl group.

b) Anti-CMV Activity

Human cytomogalovirus (HCMV) was assayed in monolayers of either MRC5 cells (human embryonic lung) in multiwell trays. The standard CMV strain AD 169 was used. Activity of compounds is determined in the plaque reduction

assay, in which a cell monolayer is infected with a suspension of HCMV, and then overlaid with nutrient agarose in the form of a gel to ensure that there is no spread of virus throughout the culture. A range of concentrations of compound of known molarity was incorporated in the nutrient agarose overlay. Plaque numbers at each concentration of drug are expressed as percentage of the control and a dose-response curve is drawn.

c)Anti-VZV Activity

Clinical isolates of varicella zoster virus (VZV) were assayed in monolayers of MRC-5 cells. MRC-5 cells are derived from human embyonic lung tissue. A plaque reduction assay was used in which a suspension of the virus stock was used to infect monolayers of the cells in multiwell trays. a range of concentrations of the compound under test of known molarity was added to the wells. Plaque numbers at each concnetration were expressed as percentages of the control and a dose response curve was constructed. From these curves the 50% inhibitory concentration of each drug was determined.

The antiviral activity of 5-(2-chloroethyl)-2′-deoxy-4′-thiouridine was determined to be as follows:

$$IC_{50}\ (\mu M)\ \text{vs HSV-1} = 0.156$$

$$IC_{50}\ (\mu M)\ \text{vs HSV-2} = >2$$

$$IC_{50}\ (uM)\ \text{vs VSV} = 0.29$$

FORMULATION EXAMPLES

The following examples illustrate pharmaceutical formulations according to the invention in which the active ingredient is a compound of formula (I).

**Formulation Example A Tablet**

| | |
|---|---|
| Active ingredient | 100 mg |
| Lactose | 200 mg |
| Starch | 50 mg |
| Polyvinylpyrrolidone | 5 mg |
| Magnesium stearate | 4 mg |
| | 359 mg |

Tablets are prepared from the foregoing ingredients by wet granulation followed by compression.

**Formulation Example B Opthalmic Solution**

| | |
|---|---|
| Active ingredient | 0.5 g |
| Sodium chloride, analytical grade | 0.9 g |
| Thiomersal | 0.001 g |
| Purified water to | 100 ml |
| pH adjusted to | 7.5 |

**Formulation Example C: Tablet Formulations**

The following formulations a and b are prepared by wet granulation of the ingredients with a solution of povidone, followed by addition of magnesium stearate and compression.

**Tablet Formulation a**

| | mg/tablet | mg/tablet |
|---|---|---|
| (a) Active ingredient | 250 | 250 |
| (b) Lactose B.P. | 210 | 26 |
| (c) Povidone B.P. | 15 | 9 |
| (d) Sodium Starch Glycolate | 20 | 12 |
| (e) Magnesium Stearate | 5 | 3 |
| | 500 | 300 |

**Tablet Formulation b**

| | mg/tablet | mg/tablet |
|---|---|---|
| (a) Active ingredient | 250 | 250 |
| (b) Lactose | 150 | - |
| (c) Avicel PH 101 | 60 | 26 |
| (d) Povidone B.P. | 15 | 9 |
| (e) Sodium Starch Glycollate | 20 | 12 |
| (f) Magnesium Stearate | 5 | 3 |
| | 500 | 300 |

**Tablet Formulation c**

| | mg/tablet |
|---|---|
| Active ingredient | 100 |
| Lactose | 200 |
| Starch | 50 |
| Povidone | 5 |
| Magnesium stearate | 4 |
| | 359 |

The following formulations, D and E, are prepared by direct compression of the admixed ingredients. The lactose used in formulation E is of the direct compression type.

**Tablet Formulation d**

| | mg/capsule |
|---|---|
| Active Ingredient | 250 |
| Pregelatinised Starch NF15 | 150 |
| | 400 |

**Tablet Formulation e**

| | mg/capsule |
|---|---|
| Active Ingredient | 250 |
| Lactose | 150 |
| Avicel | 100 |
| | 500 |

**Tablet Formulation f (Controlled Release Formulation)**

The formulation is prepared by wet granulation of the ingredients (below) with a solution of povidone followed by the addition of magnesium stearate and compression.

| | mg/tablet |
|---|---|
| (a) Active Ingredient | 500 |
| (b) Hydroxpropylmethylcellulose (Methocel K4M Premium) | 112 |
| (c) Lactose B.P. | 53 |
| (d) Povidone B.P.C. | 28 |
| (e) Magnesium Stearate | 7 |
| | 700 |

Drug release takes place over a period of about 6-8 hours and was complete after 12 hours.

**Formulation Example D: Capsule Formulations**

**Capsule Formulation a**

A capsule formulation is prepared by admixing the ingredients of Formulation D in Example C above and filling into a two-part hard gelatin capsule. Formulation B (infra) is prepared in a similar manner.

**Capsule Formulation b**

| | mg/capsule |
|---|---|
| (a) Active ingredient | 250 |
| (b) Lactose B.P. | 143 |
| (c) Sodium Starch Glycollate | 25 |
| (d) Magnesium Stearate | 2 |
| | 420 |

**Capsule Formulation c**

| | mg/capsule |
|---|---|
| (a) Active ingredient | 250 |
| (b) Macrogol 4000 BP | 350 |
| | 600 |

Capsules are prepared by melting the Macrogol 4000 BP, dispersing the active ingredient in the melt and filling the melt into a two-part hard gelatin capsule.

**Capsule Formulation d**

| | mg/capsule |
|---|---|
| Active ingredient | 250 |
| Lecithin | 100 |
| Arachis Oil | 100 |
| | 450 |

Capsules are prepared by dispersing the active ingredient in the lecithin and arachis oil and filling the dispersion into soft, elastic gelatin capsules.

**Capsule Formulation e (Controlled Release Capsule)**

The following controlled release capsule formulation is prepared by extruding ingredients a, b, and c using an extruder, followed by spheronisation of the extrudate and drying. The dried pellets are then coated with release-controlling membrane (d) and filled into a two-piece, hard gelatin capsule.

| | mg/capsule |
|---|---|
| (a) Active Ingredient | 250 |
| (b) Microcrystalline Cellulose | 125 |
| (c) Lactose BP | 125 |
| (d) Ethyl Cellulose | 13 |
| | 513 |

**Formulation Example E: Injectable Formulation**

| | |
|---|---|
| **Active ingredient** | 0.200 g |
| Sterile, pyrogen free phosphate buffer (pH 7.0) to | 10 ml |

The active ingredient is dissolved in most of the phosphate buffer (35-40°C), then made up to volume and filtered through a sterile micropore filter into a sterile 10ml amber glass vial (type 1) and sealed with sterile closures and overseals.

**Formulation Example F: Intramuscular injection**

| | |
|---|---|
| Active Ingredient | 0.20 g |
| Benzyl Alcohol | 0.10 g |
| Glucofurol 75 | 1.45 g |
| Water for Injection q.s. to | 3.00 ml |

The active ingredient is dissolved in the glycofurol. The benzyl alcohol is then added and dissolved, and water added to 3 ml. The mixture is then filtered through a sterile micropore filter and sealed in sterile 3 ml glass vials (type 1).

**Formulation Example G: Syrup Suspension**

| | |
|---|---|
| Active ingredient | 0.2500 g |
| Sorbitol Solution | 1.5000 g |
| Glycerol | 2.0000 g |
| Dispersible Cellulose | 0.0750 g |
| Sodium Benzoate | 0.0050 g |
| Flavour, Peach 17.42.3169 | 0.0125 ml |
| Purified Water q.s. to | 5.0000 ml |

The sodium benzoate is dissolved in a portion of the purified water and the sorbitol solution added. The active ingredient is added and dispersed. In the glycerol is dispersed the thickener (dispersible cellulose). The two dispersions are mixed and made up to the required volume with the purified water. Further thickening is achieved as required by extra shearing of the suspension.

**Formulation Example H: Suppository**

| | mg/suppository |
|---|---|
| Active Ingredient (63µm)* | 250 |
| Hard Fat, BP (Witepsol H15 - Dynamit NoBel) | 1770 |
| | 2020 |

* The active ingredient is used as a powder wherein at least 90% of the particles are of 63µm diameter or less.

One-fifth of the Witepsol H15 is melted in a steam-jacketed pan at 45°C maximum. The active ingredient is sifted through a 200µm sieve and added to the molten base with mixing, using a silverson fitted with a cutting head, until a smooth dispersion is achieved. Maintaining the mixture at 45°C, the remaining Witepsol H15 is added to the suspension and stirred to ensure a homogenous mix. The entire suspension is passed through a 250µm stainless steel screen and, with continuous stirring, is allowed to cool to 40°C. At a temperature of 38°C to 40°C 2.02g of the mixture is filled into suitable plastic moulds. The suppositories are allowed to cool to room temperature.

**Formulation Example I: Pessaries**

| | mg/pessary |
|---|---|
| Active ingredient 63µm | 250 |
| Anydrate Dextrose | 380 |
| Potato Starch | 363 |
| Magnesium Stearate | 7 |
| | 1000 |

The above ingredients are mixed directly and pessaries prepared by direct compression of the resulting mixture.

## Claims

### Claims for the following Contracting States : AT, BE, CH, DE, DK, FR, GB, IT, LI, LU, NL, SE

**1.** A pyrimidine 4'-thionucleoside of the formula (I)

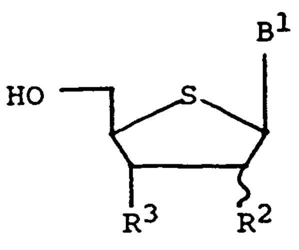

(I)

where $B^1$ is a pyrimidine base;
and either (a) $R^2$ is hydrogen and $R^3$ is hydrogen, hydroxy or fluoro,
or (b) $R^2$ is hydroxy and $R^3$ is hydrogen, hydroxy or fluoro,
or (c) $R^2$ is fluoro and $R^3$ is hydrogen or hydroxy,
or (d) $R^2$ and $R^3$ together form a carbon-carbon bond; and physiologically functional derivatives thereof,

with the proviso that when $R^2$ is hydrogen and $R^3$ is hydroxy, $B^1$ is not a pyrimidine base of formula (X)

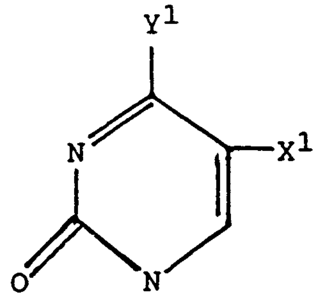

(X)

where $Y^1$ is hydroxy or amino and $X^1$ is chloro, bromo, iodo, trifluoromethyl, $C_{2-6}$ alkyl, $C_{2-6}$ alkenyl, $C_{2-6}$ haloalkenyl or $C_{2-6}$ alkynyl, for use in a method of treatment or prophylaxis of virus infections of the human or animal body.

2. A pyrimidine 4'-thionucleoside of formula (I)

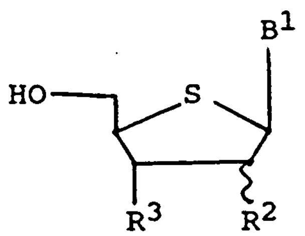

(I)

where $B^1$ is a pyrimidine base;
and either (a) $R^2$ is hydrogen and $R^3$ is hydrogen, hydroxy or fluoro,
or (b) $R^2$ is hydroxy and $R^3$ is hydrogen, hydroxy or fluoro,
or (c) $R^2$ is fluoro and $R^3$ is hydrogen or hydroxy,
or (d) $R^2$ and $R^3$ together form a carbon-carbon bond; and physiologically functional derivatives thereof, with the provisos:

(i) that when $R^2$ is hydrogen and $R^3$ is hydroxy, $B^1$ is not a pyrimidine base of formula (X)

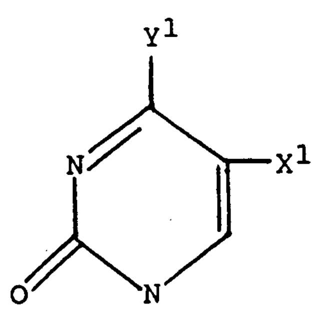

(X)

where $Y^1$ is hydroxy or amino and $X^1$ is fluoro, chloro, bromo, iodo, methyl, trifluoromethyl, $C_{2-6}$ alkyl, $C_{2-6}$ alkenyl, $C_{2-6}$ haloalkenyl or $C_{2-6}$ alkynyl;
(ii) that the compound of the formula (I) is not 4'-thiocytidine, 4′-thiouracil, 1-(4-thio-β-D-arabinofuranosyl)cytosine, 1-(4-thio-β-D-arabinofuranosyl)thymine, or 1-(4-thio-β-D-arabinofuranosyl)uracil; and
(iii) that the compound of formula (I) is not a 5-substituted-4′-thiouridine wherein the 5-substituent is halogen or methyl.

3. A compound according to claim 1 or 2 wherein $B^1$ is of the formula (II)

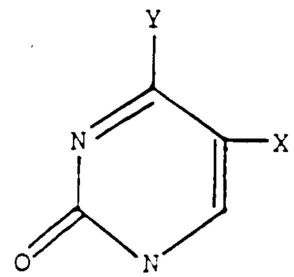

(II)

wherein Y is hydroxy or amino, monoalkylamino or dialkylamino; and X is halogen, alkoxy, alkyl, haloalkyl, alkenyl, haloalkenyl, alkynyl, amino, monoalkylamino, dialkylamino, cyano or nitro.

4. A compound according to claim 3 wherein X is $C_{2-4}$ alkyl, haloalkyl or haloalkenyl, or $C_{3-4}$ alkenyl or alkynyl.

5. A compound according to any one of the preceding claims wherein the 4-thiopentofuranoside moiety of the compound of formula (I) is selected from the residues of:
Thio-D-ribofuranose,
4-Thio-D-arabinofuranose,
2-Deoxy-4-thio-D-ribofuranose,
2,3-Dideoxy-4-thio-D-pentenofuranose,
2,3-Dideoxy-4-thio-D-ribofuranose,
2-Fluoro-4-thio-D-arabinofuranose,
2-Fluoro-4-thio-D-ribofuranose, and
2,3-Dideoxy-3-fluoro-4-thio-D-ribofuranose.

6. A compound according to claim 1 or 2 which is selected from:
5-(2-chloroethyl)-2′-deoxy-4′-thiouridine;
5-nitro-2′-deoxy-4′-thiouridine;
5-amino-2′-deoxy-4′-thiouridine;
5-methylamino-2′-deoxy-4′-thiouridine;
5-ethynyl-4′-thiourdine;
E-5-(2-bromovinyl)-4′-thiouridine;
5-ethynyl-1-(4-thio-β-D-arabinofuranosyl)uracil;
5-ethyl-1-(4-thio-β-D-arabinofuranosyl)uracil;
5-(prop-1-ynyl)-1-(4-thio-β-D-arabinofuranosyl)uracil;
E-5-(2-bromovinyl)-1-(4-thio-β-D-arabinofuranosyl)uracil;
1-(2-fluoro-4-thio-β-D-arabinofuranosyl)-5-methyl uracil;
1-(2-fluoro-4-thio-β-D-arabinofuranosyl)-5-iodocytosine;
1-(2,3-didehydro-2,3-dideoxy-4-thio-D-ribofuranosyl)-5-methyluracil; and
2′-deoxy-2′-fluoro-4′-thiocytidine.

7. A physiologically acceptable derivative of a compound according to any one of the preceding claims which is an alkali metal, alkaline earth metal, ammonium, $NR_4$ (wherein R is $C_{1-4}$ alkyl), hydrochloride or acetate salt.

8. A compound according to any one of the preceding claims in association with a pharmaceutically acceptable carrier or diluent.

**9.** A process for the production of a compound of formula (I)

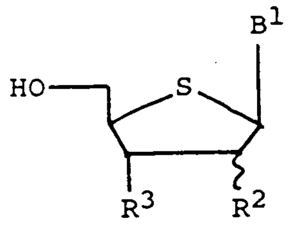

(I)

where $B^1$ is a pyrimidine base;
and either (a) $R^2$ is hydrogen and $R^3$ is hydrogen, hydroxy or fluoro,
or (b) $R^2$ is hydroxy and $R^3$ is hydrogen, hydroxy or fluoro,
or (c) $R^2$ is fluoro and $R^3$ is hydrogen or hydroxy,
or (d) $R^2$ and $R^3$ together form a carbon-carbon bond; and physiologically functional derivatives thereof,
with the provisos:

(i) that when $R^2$ is hydrogen and $R^3$ is hydroxy, $B^1$ is not a pyrimidine base of formula (X)

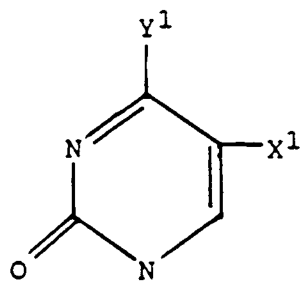

(X)

where $Y^1$ is hydroxy or amino and $X^1$ is fluoro, chloro, bromo, iodo, methyl, trifluoromethyl, $C_{2-6}$ alkyl, $C_{2-6}$ alkenyl, $C_{2-6}$ haloalkenyl or $C_{2-6}$ alkynyl;
(ii) that the compound of the formula (I) is not 4′-thiocytidine, 4′-thiouracil, 1-(4-thio-β-D-arabinofuranosyl)cytosine, 1-(4-thio-β-D-arabinofuranosyl)thymine, or 1-(4-thio-β-D-arabinofuranosyl)uracil; and
(iii) that the compound of formula (I) is not a 5-substituted-4′-thiouridine wherein the 5-substituent is halogen or methyl;

which process comprises:

A) reacting a compound of formula (III-A)

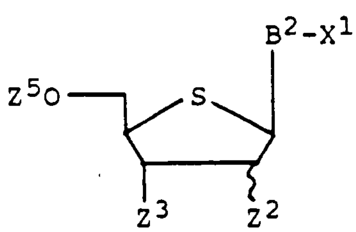

(III-A)

wherein $X^1$ is a precursor for a substituent of a pyrimidine base as defined in relation to $B^1$ in formula (I);
$B^2$ is a pyrimidine substituted by the group $X^1$;
$Z^2$ and $Z^3$ are the same or different and are groups $R^2$ and $R^3$ or protected hydroxyls; and
$Z^5$ is hydrogen or a hydroxyl protecting group
with a reagent or reagents serving to convert the group $X^1$ to the desired group X;

B) reacting a compound of formula (IV-A)

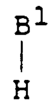

(IV-A)

or a protected form thereof with a 4-thio sugar derivative serving to introduce the 4-thio sugar moiety, or a protected form thereof, at the 1-position of the base $B^1$;

C) reacting a compound of formula (V-A)

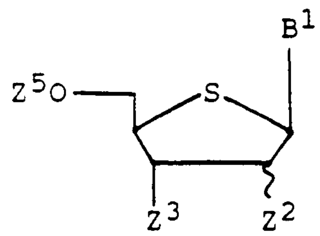

(V-A)

wherein

$B^1$ is a pyrimidine base as hereinbefore defined;

$Z^5$ is hydrogen or a hydroxy protecting group, and

$Z^2$ and $Z^3$ are as defined above wherein at least one of $Z^2$ and $Z^3$ represents a precursor group for the group(s) $R^2$ and/or $R^3$ in formula (I)

under conditions and/or with a reagent serving to convert the groups $Z^2$ and/or $Z^3$ into the respective groups $R^2$ and/or $R^3$.

**10.** A process according to claim 9 wherein $B^1$ is of the formula (II)

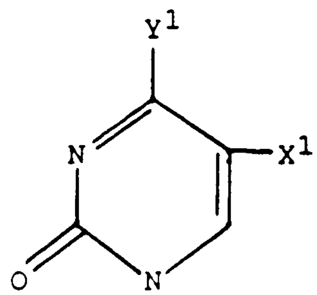

(X)

wherein Y is hydroxy or amino, monoalkylamino or dialkylamino; and X is halogen, alkoxy, alkyl, halo, alkenyl, haloalkenyl, alkynyl, amino, monoalkylamino, dialkylamino, cyano or nitro, which comprises

D) reacting a compound of formula (III)

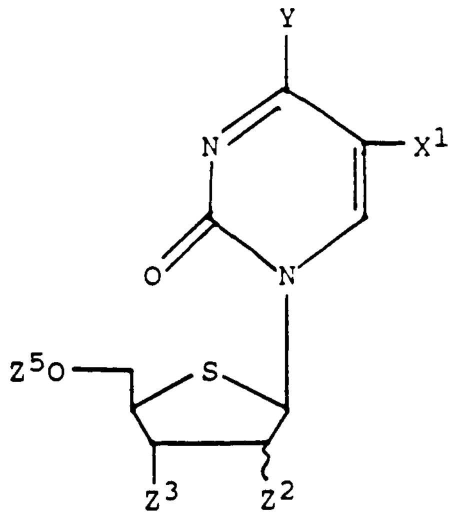

(III)

wherein $X^1$ is a precursor for the group X as defined in relation to formula (I);

Y is as defined in relation to formula (II);

$Z^2$ and $Z^3$ are the same or different and are groups $R^2$ and $R^3$ or protected hydroxyl groups and $Z^5$ is hydrogen or a hydroxyl protecting group

with a reagent or reagents serving to convert the group $X^1$ to the desired group X;

E) reacting a compound of formula (IV)

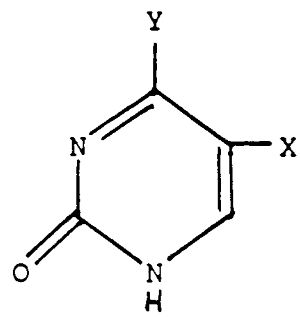

(IV)

wherein X and Y are as defined in relation to formula (I) or a protected form thereof with a 4-thio sugar derivative serving to introduce the 4-thio sugar moiety of formula (I), or a protected form thereof, at the 1-position of the compound of formula (IV);

F) reacting a compound of formula (V)

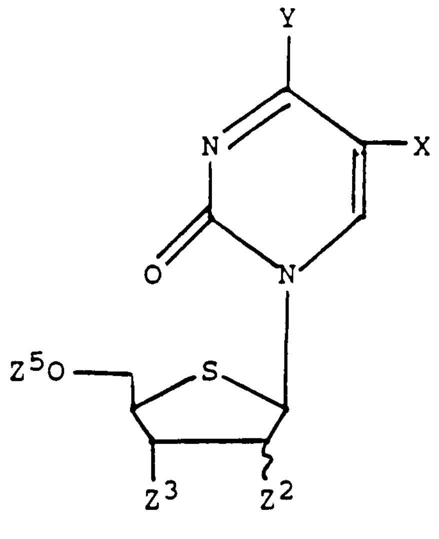

(V)

wherein X and Y are as defined in relation to formula (I), $Z^5$ is a hydroxy protecting group or hydrogen; $Z^2$ and $Z^3$ are as defined above wherein at least one of $Z^2$ and $Z^3$ represents a precursor group for the group(s) $R^2$ and/or $R^3$ in formula (I)

under conditions and/or with a reagent serving to convert the groups $Z^2$ and/or $Z^3$ into the respective groups $R^2$ and/or $R^3$.

**11.** A process according to claim 9 or 10 which further includes, following one or more of processes A to F, any one or more of the following further steps in any order:

a) removing each of the protecting groups,
b) converting a compound of formula (I) or a protected form thereof into a further compound of formula (I) or a protected form thereof,
c) converting the compound of formula (I) or a protected form thereof into a physiologically acceptable derivative of the compound of formula (I) or a protected form thereof,
d) converting a physiologically acceptable derivative of the compound of formula (I) or a protected form thereof into the compound of formula (I) or a protected form, thereof,
e) converting a physiologically acceptable derivative of the compound of formula (I) or a protected form thereof into another physiologically acceptable derivative of the compound of formula (I) or a protected form thereof,
f) where necessary, separating the α and β anomers of the compound of formula I or a protected derivative thereof or of a physiologically acceptable derivative of a compound of formula (I) or a derivative thereof, and
g) when 4′-sulphone or sulphoxide sugar moiety derivatives are required, partially or completely oxidising the sulphur of the 4-thio sugar moiety of the corresponding compound of formula (I) or a protected form thereof or a compound of formula (II) or a protected form thereof, with a peracid.

**12.** A process according to any one of claims 9B, 10E or 11 which comprises:

a) Reacting the compound of formula (IV) or (IV-A) as defined in claims 9 and 10 respectively, or a protected form thereof, with a 4-thio sugar compound of formula (VI)

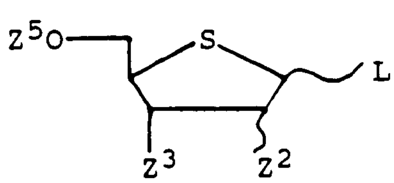

(VI)

wherein $Z^2$, $Z^3$ and $Z^5$ are as defined in claim 9 and L is a leaving group,

in the presence of a Lewis Acid catalyst in a solvent at reduced, ambient or elevated temperature;

b) Reacting the compound of formula (IV-A) or (IV) as defined above, or a protected form thereof, with a compound of formula (VII)

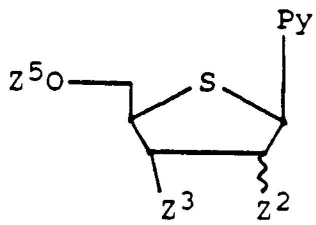

(VII)

wherein $Z^2$, $Z^3$ and $Z^5$ are as defined above and Py represents a pyrimidine base,

in the presence of a silylating agent and in the presence of a Lewis Acid catalyst.

**13.** A process according to any one of claims 10 to 12 wherein X is $C_{2-4}$ alkyl, haloalkyl or haloalkenyl, or $C_{3-4}$ alkenyl or alkynyl.

**14.** A process according to any one of claims 9 to 13 wherein the 4-thiopentofuranoside moiety of the compound of formula (I) is selected from the residues of:

Thio-D-ribofuranose,
4-Thio-D-arabinofuranose,
2-Deoxy-4-thio-D-ribofuranose,
2,3-Dideoxy-4-thio-D-pentenofuranose,
2,3-Dideoxy-4-thio-D-ribofuranose,
2-Fluoro-4-thio-D-arabinofuranose,
2-Fluoro-4-thio-D-ribofuranose, and
2,3-Dideoxy-3-fluoro-4-thio-D-ribofuranose.

**15.** A process according to any one of claims 9 to 14 wherein the compound of formula (I) obtained is selected from:

5-(2-chloroethyl)-2′-deoxy-4′-thiouridine;
5-nitro-2′-deoxy-4′-thiouridine;
5-amino-2′-deoxy-4′-thiouridine;
5-methylamino-2′-deoxy-4′-thiouridine;
5-ethynyl-4′-thiourdine;
E-5-(2-bromovinyl)-4′-thiouridine;
5-ethynyl-1-(4-thio-β-D-arabinofuranosyl)uracil;
5-ethyl-1-(4-thio-β-D-arabinofuranosyl)uracil;
5-(prop-l-ynyl)-1-(4-thio-β-D-arabinofuranosyl)uracil;
E-5-(2-bromovinyl)-1-(4-thio-β-D-arabinofuranosyl)uracil;
1-(2-fluoro-4-thio-β-D-arabinofuranosyl)-5-methyl uracil;
1-(2-fluoro-4-thio-β-D-arabinofuranosyl)-5-iodocytosine;
1-(2,3-didehydro-2,3-dideoxy-4-thio-β-D-arabinofuranosyl)-5-methyluracil; and
2′-deoxy-2′-fluoro-4′-thiocytidine.

**16.** A process according to any one of claims 9 to 15 wherein a physiologically acceptable derivative of the compound of formula (I) is obtained, the derivative being an alkali metal, alkaline earth metal, ammonium, $NR_4$ (wherein R is $C_{1-4}$ alkyl), hydrochloride or acetate salt.

**17.** A process according to any one of claims 9 to 16 which further includes formulating the compound of formula (I) obtained with a pharmaceutically acceptable carrier or diluent.

**18.** Use of a compound according to any one of claims 1 to 8 for the manufacture of a medicament for use in the treatment or prophylaxis of a virus infection.

**Claims for the following Contracting States : ES, GR**

**1.** A process for the production of a compound of formula (I)

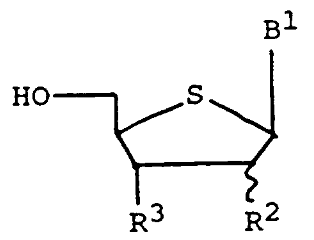

(I)

where $B^1$ is a pyrimidine base;
and either (a) $R^2$ is hydrogen and $R^3$ is hydrogen, hydroxy or fluoro,
or (b) $R^2$ is hydroxy and $R^3$ is hydrogen, hydroxy or fluoro,
or (c) $R^2$ is fluoro and $R^3$ is hydrogen or hydroxy,
or (d) $R^2$ and $R^3$ together form a carbon-carbon bond; and physiologically functional derivatives thereof,
with the provisos:

(i) that when $R^2$ is hydrogen and $R^3$ is hydroxy, $B^1$ is not a pyrimidine base of formula (X)

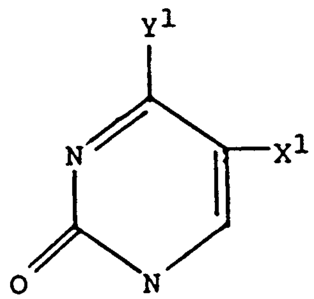

(X)

where $Y^1$ is hydroxy or amino and $X^1$ is fluoro, chloro, bromo, iodo, methyl, trifluoromethyl, $C_{2-6}$ alkyl, $C_{2-6}$ alkenyl, $C_{2-6}$ haloalkenyl or $C_{2-6}$ alkynyl;
(ii) that the compound of the formula (I) is not 4'-thiocytidine, 4'-thiouracil, 1-(4-thio-β-D-arabinofuranosyl)cytosine, 1-(4-thio-β-D-arabinofuranosyl)thymine, or 1-(4-thio-β-D-arabinofuranosyl)uracil; and
(iii) that the compound of formula (I) is not a 5-substituted-4'-thiouridine wherein the 5-substituent is halogen or methyl;

which process comprises:

A) reacting a compound of formula (III-A)

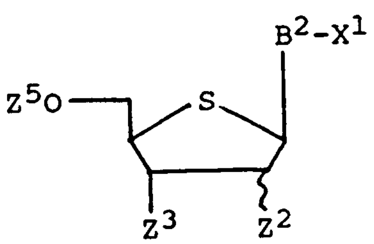

(III-A)

wherein $X^1$ is a precursor for a substituent of a pyrimidine base as defined in relation to $B^1$ in formula (I);

$B^2$ is a pyrimidine substituted by the group $X^1$;
$Z^2$ and $Z^3$ are the same or different and are groups $R^2$ and $R^3$ or protected hydroxyls; and
$Z^5$ is hydrogen or a hydroxyl protecting group
with a reagent or reagents serving to convert the group $X^1$ to the desired group X;
B) reacting a compound of formula (IV-A)

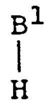

(IV-A)

or a protected form thereof with a 4-thio sugar derivative serving to introduce the 4-thio sugar moiety, or a protected form thereof, at the 1-position of the base $B^1$;
C) reacting a compound of formula (V-A)

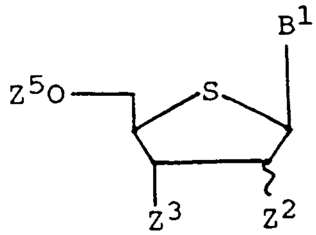

(V-A)

wherein
$B^1$ is a pyrimidine base as hereinbefore defined;
$Z^5$ is hydrogen or a hydroxy protecting group, and
$Z^2$ and $Z^3$ are as defined above wherein at least one of $Z^2$ and $Z^3$ represents a precursor group for the group(s) $R^2$ and/or $R^3$ in formula (I)
under conditions and/or with a reagent serving to convert the groups $Z^2$ and/or $Z^3$ into the respective groups $R^2$ and/or $R^3$.

**2.** A process according to claim 1 wherein $B^1$ is of the formula (II)

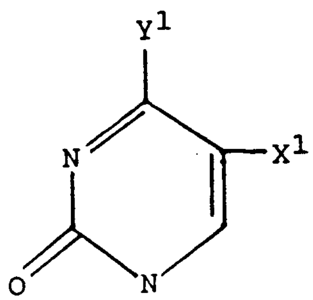

(X)

wherein Y is hydroxy or amino, monoalkylamino or dialkylamino; and X is halogen, alkoxy, alkyl, halo, alkenyl, haloalkenyl, alkynyl, amino, monoalkylamino, dialkylamino, cyano or nitro, which comprises

D) reacting a compound of formula (III)

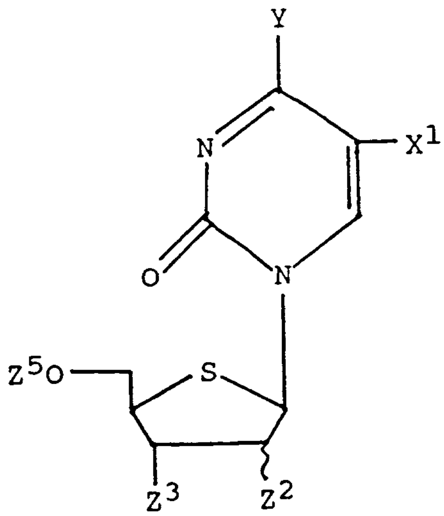

(III)

wherein $X^1$ is a precursor for the group X as defined in relation to formula (I);

Y is as defined in relation to formula (II);

$Z^2$ and $Z^3$ are the same or different and are groups $R^2$ and $R^3$ or protected hydroxyl groups and $Z^5$ is hydrogen or a hydroxyl protecting group

with a reagent or reagents serving to convert the group $X^1$ to the desired group X;

E) reacting a compound of formula (IV)

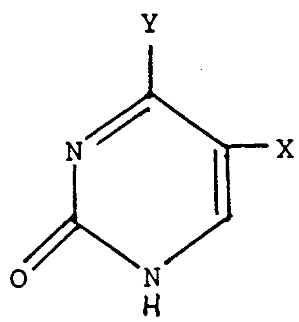

(IV)

wherein X and Y are as defined in relation to formula (I) or a protected form thereof with a 4-thio sugar derivative serving to introduce the 4-thio sugar moiety of formula (I), or a protected form thereof, at the 1-position of the compound of formula (IV);

F) reacting a compound of formula (V)

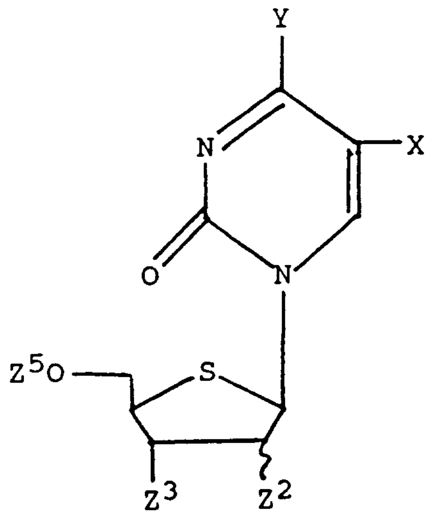

(V)

wherein X and Y are as defined in relation to formula (I), $Z^5$ is a hydroxy protecting group or hydrogen; $Z^2$ and $Z^3$ are as defined above wherein at least one of $Z^2$ and $Z^3$ represents a precursor group for the group(s) $R^2$ and/or $R^3$ in formula (I)

under conditions and/or with a reagent serving to convert the groups $Z^2$ and/or $Z^3$ into the respective groups $R^2$ and/or $R^3$.

**3.** A process according to claim 1 or 2 which further includes, following one or more of processes A to F, any one or more of the following further steps in any order:

a) removing each of the protecting groups,
b) converting a compound of formula (I) or a protected form thereof into a further compound of formula (I) or a protected form thereof,
c) converting the compound of formula (I) or a protected form thereof into a physiologically acceptable derivative of the compound of formula (I) or a protected form thereof,
d) converting a physiologically acceptable derivative of the compound of formula (I) or a protected form thereof into the compound of formula (I) or a protected form, thereof,
e) converting a physiologically acceptable derivative of the compound of formula (I) or a protected form thereof into another physiologically acceptable derivative of the compound of formula (I) or a protected form thereof,
f) where necessary, separating the $\alpha$ and $\beta$ anomers of the compound of formula I or a protected derivative thereof or of a physiologically acceptable derivative of a compound of formula (I) or a derivative thereof, and
g) when 4'-sulphone or sulphoxide sugar moiety derivatives are required, partially or completely oxidising the sulphur of the 4-thio sugar moiety of the corresponding compound of formula (I) or a protected form thereof or a compound of formula (II) or a protected form thereof, with a peracid.

**4.** A process according to any one of claims 1B, 2E or 3 which comprises:

a) Reacting the compound of formula (IV) or (IV-A) as defined in claims 1 and 2 respectively, or a protected form thereof, with a 4-thio sugar compound of formula (VI)

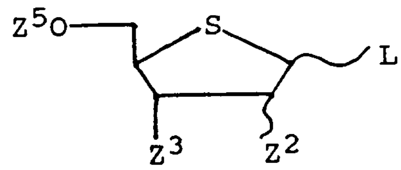

(VI)

wherein $Z^2$, $Z^3$ and $Z^5$ are as defined in claim 9 and L is a leaving group,
in the presence of a Lewis Acid catalyst in a solvent at reduced, ambient or elevated temperature;
b) Reacting the compound of formula (IV-A) or (IV) as defined above, or a protected form thereof, with a compound of formula (VII)

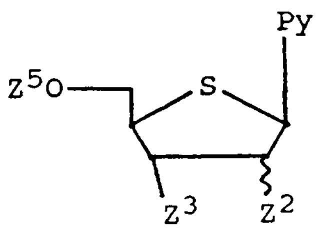

(VII)

wherein $Z^2$, $Z^3$ and $Z^5$ are as defined above and Py represents a pyrimidine base,

in the presence of a silylating agent and in the presence of a Lewis Acid catalyst.

**5.** A process according to any one of claims 2 to 4 wherein X is $C_{2-4}$ alkyl, haloalkyl or haloalkenyl, or $C_{3-4}$ alkenyl or alkynyl.

**6.** A process according to any one of claims 1 to 5 wherein the 4-thiopentofuranoside moiety of the compound of formula (I) is selected from the residues of:
Thio-D-ribofuranose,
4-Thio-D-arabinofuranose,
2-Deoxy-4-thio-D-ribofuranose,
2,3-Dideoxy-4-thio-D-pentenofuranose,
2,3-Dideoxy-4-thio-D-ribofuranose,
2-Fluoro-4-thio-D-arabinofuranose,
2-Fluoro-4-thio-D-ribofuranose, and
2,3-Dideoxy-3-fluoro-4-thio-D-ribofuranose.

**7.** A process according to any one of claims 1 to 6, wherein the compound of formula (I) obtained is selected from:
5-(2-chloroethyl)-2'-deoxy-4'-thiouridine;
5-nitro-2'-deoxy-4'-thiouridine;
5-amino-2'-deoxy-4'-thiouridine;
5-methylamino-2'-deoxy-4'-thiouridine;
5-ethynyl-4'-thiourdine;
E-5-(2-bromovinyl)-4'-thiouridine;
5-ethynyl-1-(4-thio-β-D-arabinofuranosyl)uracil;
5-ethyl-1-(4-thio-β-D-arabinofuranosyl)uracil;
5-(prop-l-ynyl)-1-(4-thio-β-D-arabinofuranosyl)uracil;
E-5-(2-bromovinyl)-1-(4-thio-β-D-arabinofuranosyl)uracil;
1-(2-fluoro-4-thio-β-D-arabinofuranosyl)-5-methyl uracil;
1-(2-fluoro-4-thio-β-D-arabinofuranosyl)-5-iodocytosine;
1-(2,3-didehydro-2,3-dideoxy-4-thio-β-D-arabinofuranosyl)-5-methyluracil; and
2'-deoxy-2'-fluoro-4'-thiocytidine.

8. A process according to any one of claims 1 to 7 wherein a physiologically acceptable derivative of the compound of formula (I) is obtained, the derivative being an alkali metal, alkaline earth metal, ammonium, $NR_4$ (wherein R is $C_{1-4}$ alkyl), hydrochloride or acetate salt.

9. A process according to any one of claims 1 to 8 which further includes formulating the compound of formula (I) obtained with a pharmaceutically acceptable carrier or diluent.

10. Use of a compound according to any one of claims 1 to 8 for the manufacture of a medicament for use in the treatment or prophylaxis of a virus infection.

**Patentansprüche**

**Patentansprüche für folgende Vertragsstaaten : AT, BE, CH, DE, DK, FR, GB, IT, LI, LU, NL, SE**

1. Pyrimidin-4'-thionukleosid der Formel (I)

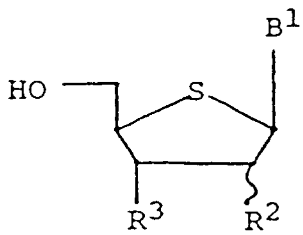

(I)

in der $B^1$ eine Pyrimidinbase darstellt und

(a) $R^2$ Wasserstoff und $R^3$ Wasserstoff, Hydroxy oder Fluor darstellt oder
(b) $R^2$ eine Hydroxygruppe und $R^3$ Wasserstoff, Hydroxy oder Fluor oder
(c) $R^2$ Fluor und $R^3$ Wasserstoff oder Hydroxy bedeuten oder
(d) $R^2$ und $R^3$ zusammen eine Kohlenstoff-Kohlenstoffbindung bilden

sowie dessen physiologisch wirksame Derivate,
mit der Maßgabe, daß dann, wenn $R^2$ Wasserstoff und $R^3$ Hydroxy bedeuten, $B^1$ keine Pyridinbase der Formel (X)

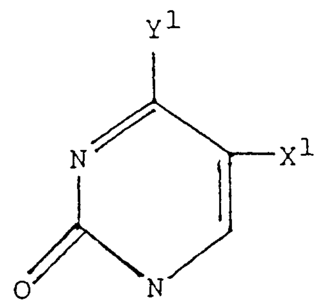

(X)

ist, in der
$Y^1$ eine Hydroxy- oder Aminogruppe darstellt und $X^1$ Chlor, Brom, Jod, Trifluormethyl, $C_{2-6}$-Alkyl, $C_{2-6}$-Alkenyl, $C_{2-6}$-Halogenalkenyl oder $C_{2-6}$-Alkinyl bedeutet, zur Verwendung in einem Verfahren zur Behandlung oder zur Prophylaxe von Virusinfektionen des menschlichen oder tierischen Körpers.

**2.** Pyrimidin-4'-thionukleosid der Formel (I)

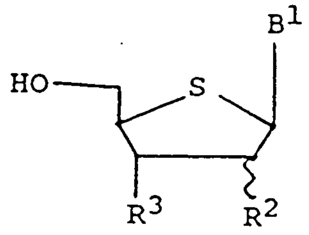

(I)

in der $B^1$ eine Pyrimidinbase darstellt und

(a) $R^2$ Wasserstoff und $R^3$ Wasserstoff, Hydroxy oder Fluor darstellt oder
(b) $R^2$ Hydroxy und $R^3$ Wasserstoff, Hydroxy oder Fluor oder
(c) $R^2$ Fluor und $R^3$ Wasserstoff oder Hydroxy bedeuten oder
(d) $R^2$ und $R^3$ zusammen eine Kohlenstoff-Kohlenstoffbindung bilden

sowie dessen physiologisch wirksame Derivate,
mit der Maßgabe, daß

(i) dann, wenn $R^2$ Wasserstoff und $R^3$ Hydroxy bedeutet, $B^1$ keine Pyridinbase der Formel (X)

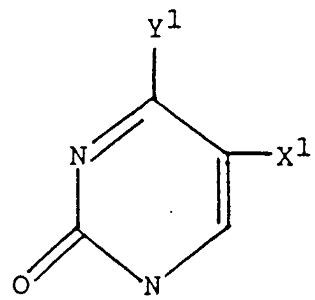

(X)

ist, in der
$Y^1$ eine Hydroxy- oder Aminogruppe darstellt und $X^1$ Chlor, Brom, Jod, Trifluormethyl, $C_{2-6}$-Alkyl, $C_{2-6}$-Alkenyl, $C_{2-6}$-Halogenalkenyl oder $C_{2-6}$-Alkinyl bedeutet,
(ii) daß es sich bei der Verbindung der Formel (I) nicht um 4'-Thiocytidin, 4'-Thiouracil, 1-(4-Thio-β-D-arabinofuranosyl)-cytosin, 1-(4-Thio-β-D-arabinofuranosyl)-thymidin oder 1-(4-Thio-β-D-arabinofuranosyl)-uracil handelt und
(iii) daß die Verbindung der Formel (I) kein 5-substituiertes 4'-Thiouridin ist, bei dem der Substituent in Position 5 Halogen oder Methyl ist.

**3.** Verbindung gemäß Anspruch 1 oder 2, in der $B^1$ der Formel (II) entspricht

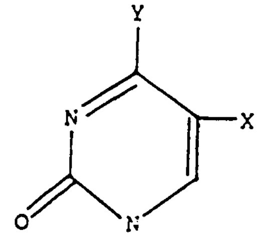

(II)

in der Y eine Hydroxy-, Amino-, Monoalkylamino- oder Dialkylaminogruppe darstellt und X Halogen, Alkoxy, Alkyl,

Halogenalkyl, Alkenyl, Halogenalkenyl, Alkinyl, eine Amino-, Monoalkylamino-, Dialkylamino-, Cyano- oder Nitrogruppe ist.

4. Verbindung gemäß Anspruch 3, in der X $C_{2-4}$-Alkyl, Halogenalkyl oder Halogenalkenyl oder $C_{3-4}$-Alkenyl oder Alkinyl ist.

5. Verbindung gemäß einem der vorstehenden Ansprüche, wobei die 4-Thiopentofuranosideinheit der Verbindung der Formel (I) ausgewählt ist aus den Resten:
Thio-D-ribofuranose,
4-Thio-D-arabinofuranose,
2-Desoxy-4-thio-D-ribofuranose,
2,3-Didesoxy-4-thio-D-pentenofuranose,
2,3-Didesoxy-4-thio-D-ribofuranose,
2-Fluoro-4-thio-D-arabinofuranose,
2-Fluoro-4-thio-D-ribofuranose und
2,3-Didesoxy-3-fluoro-4-thio-D-ribofuranose.

6. Verbindung gemäß Anspruch 1 oder 2, ausgewählt aus:
5-(2-Chlorethyl)-2'-desoxy-4'-thiouridin,
5-Nitro-2'-desoxy-4'-thiouridin,
5-Amino-2'-desoxy-4'-thiouridin,
5-Methylamino-2'-desoxy-4'-thiouridin,
5-Ethinyl-4'-thiouridin,
E-5-(2-Bromvinyl)-4'-thiouridin,
5-Ethinyl-1-(4-thio-β-D-arabinofuranosyl)-uracil,
5-Ethyl-1-(4-thio-β-D-arabinofuranosyl)-uracil,
5-(1-Propinyl)-1-(4-thio-β-D-arabinofuranosyl)-uracil,
E-5-(2-Bromvinyl)-1-(4-thio-β-D-arabinofuranosyl)-uracil,
1-(2-Fluor-4-thio-β-D-arabinofuranosyl)-5-methyluracil,
1-(2-Fluor-4-thio-β-D-arabinofuranosyl)-5-jodcytosin,
1-(2,3-Didehydro-2,3-didesoxy-4-thio-D-ribofuranosyl)-5-methyluracil und
2'-Desoxy-2'-fluor-4'-thiocytidin.

7. Physiologisch akzeptables Derivat einer Verbindung gemäß einem der vorstehenden Ansprüche, bei dem es sich um ein Alkalimetall-, Erdalkalimetall-, Ammonium- oder $NR_4$-Salz (wobei R ein $C_{1-4}$-Alkyl darstellt), ein Hydrochlorid oder ein Acetat handelt.

8. Verbindung gemäß einem der vorstehenden Ansprüche in Verbindung mit einem pharmazeutisch geeigneten Träger oder Verdünnungsmittel.

9. Verfahren zur Herstellung einer Verbindung der Formel (I)

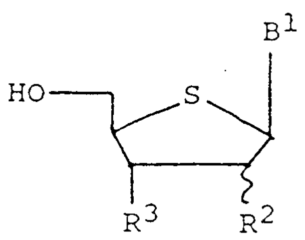

(I)

in der $B^1$ eine Pyrimidinbase darstellt und

(a) $R^2$ Wasserstoff und $R^3$ Wasserstoff, Hydroxy oder Fluor darstellt oder
(b) $R^2$ Hydroxy und $R^3$ Wasserstoff, Hydroxy oder Fluor oder
(c) $R^2$ Fluor und $R^3$ Wasserstoff oder Hydroxy bedeuten oder
(d) $R^2$ und $R^3$ zusammen eine Kohlenstoff-Kohlenstoffbindung bilden

sowie deren physiologisch wirksame Derivate,
mit der Maßgabe, daß

(i) dann, wenn $R^2$ Wasserstoff und $R^3$ Hydroxy bedeuten, $B^1$ keine Pyridinbase der Formel (X)

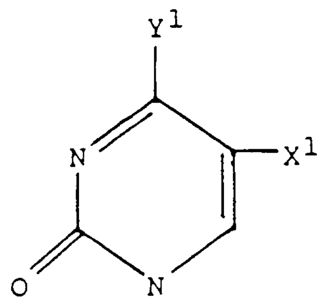

(X)

ist, in der
$Y^1$ eine Hydroxy- oder Aminogruppe darstellt und $X^1$ Fluor, Chlor, Brom, Jod, Methyl, Trifluormethyl, $C_{2-6}$-Alkyl, $C_{2-6}$-Alkenyl, $C_{2-6}$-Halogenalkenyl oder $C_{2-6}$-Alkinyl bedeutet,
(ii) daß es sich bei der Verbindung der Formel (I) nicht um 4'-Thiocytidin, 4'-Thiouracil, 1-(4-Thio-β-D-arabinofuranosyl)-cytosin, 1-(4-Thio-β-D-arabinofuranosyl)-thymidin oder 1-(4-Thio-β-D-arabinofuranosyl)-uracil handelt und
(iii) daß die Verbindung der Formel (I) kein 5-substituiertes 4'-Thiouridin ist, bei dem der Substituent in Position 5 Halogen oder Methyl ist,

bei dem man:

A) eine Verbindung der Formel (III-A)

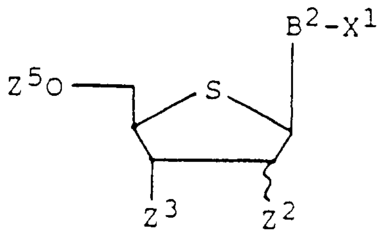

(III-A)

in der
$X^1$ einen Vorläufer eines Substituenten einer Pyridinbase darstellt, wie sie unter Bezugnahme auf $B^1$ in der Formel (I) definiert ist,
$B^2$ ein durch die Gruppe $X^1$ substituiertes Pyrimidin ist,
$Z^2$ und $Z^3$, die gleich oder verschieden sein können, Gruppen $R^2$ und $R^3$ oder geschützte Hydroxylgruppen darstellen und
$Z^5$ Wasserstoff oder eine Hydroxylschutzgruppe bedeutet,
mit einem Reagenz bzw. Reagenzien umsetzt, die dazu dienen, den Rest $X^1$ in die gewünschte Gruppe X umzuwandeln;

B) eine Verbindung der Formel (IV-A)

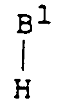

(IV-A)

oder eine geschützte Form derselben mit einem 4-Thiozuckerderivat umsetzt, um die 4-Thiozuckereinheit oder eine geschützte Form derselben in Position 1 der Base $B^1$ einzuführen;

C) eine Verbindung der Formel (V-A)

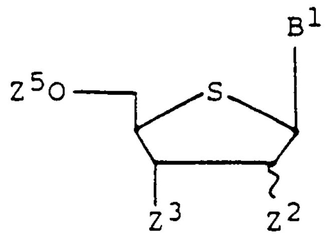

(V-A)

in der
$B^1$ eine wie oben definierte Pyrimidinbase darstellr,
$Z^5$ Wasserstoff oder eine Hydroxylschutzgruppe bedeutet, und
$Z^2$ und $Z^3$ wie oben definiert sind, wobei mindestens einer der Reste $Z^2$ und $Z^3$ eine Vorläufergruppe für den bzw. die Reste $R^2$ und/oder $R^3$ in Formel (I) darstellt,
unter Bedingungen und/oder mit einem Reaktionspartner zur Umsetzung bringt, die dazu dienen bzw. das dazu dient, die Reste $Z^2$ und/oder $Z^3$ in den jeweiligen Rest $R^2$ und/oder $R^3$ zu überführen.

**10.** Verfahren gemäß Anspruch 9, bei dem $B^1$ der Formel (II) entspricht

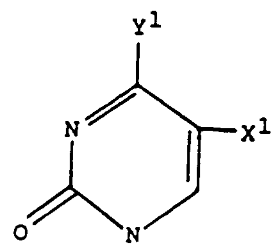

(X)

in der Y eine Hydroxy-, Amino-, Monoalkylamino- oder Dialkylaminogruppe darstellt und X Halogen, Alkoxy, Alkyl, Halogenalkyl, Alkenyl, Halogenalkenyl, Alkinyl, eine Amino-, Monoalkylamino-, Dialkylamino-, Cyano- oder Nitrogruppe darstellt,
bei dem man

D) eine Verbindung der Formel (III)

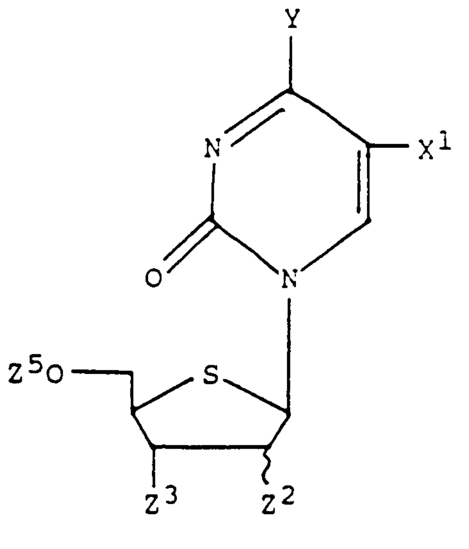

(III)

in der
$X^1$ ein Vorläufer der wie in bezug auf die Formel (I) definierten Gruppe X ist, Y wie in Beziehung auf Formel (II) definiert ist, $Z^2$ und $Z^3$ gleich oder verschieden sind und die Gruppen $R^2$ und $R^3$ oder geschützte Hydroxylgruppen darstellen und $Z^5$ Wasserstoff oder eine Hydroxylschutzgruppe bedeutet, mit einem oder mehreren Reaktionspartner umsetzt, um den Rest $X^1$ in die gewünschte Gruppe X umzuwandeln,

E) eine Verbindung der Formel (IV)

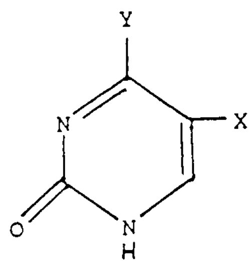

(IV)

in der X und Y die oben in bezug auf Formel (I) genannte Bedeutung haben, oder eine geschützte Form derselben
mit einem 4-Thiozuckerderivat umsetzt, um die 4-Thiozuckereinheit der Formel (I) bzw. eine geschützte Form derselben in Position 1 der Verbindung der Formel (I) einzuführen;

F) eine Verbindung der Formel (V)

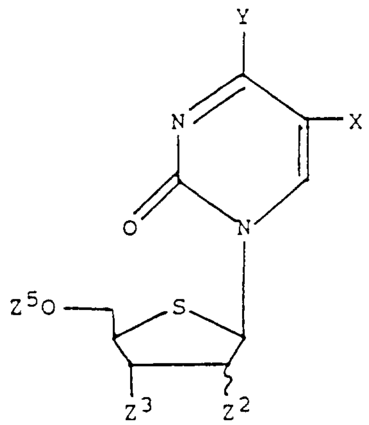

(V)

in der X und Y die oben in bezug auf Formel (I) angegebene Bedeutung haben, $Z^5$ eine Hydroxyschutzgruppe oder Wasserstoff ist, $Z^2$ und $Z^3$ wie oben definiert sind, wobei mindestens einer der Reste $Z^2$ und $Z^3$ eine Vorläufergruppe für den bzw. die Reste $R^2$ und/oder $R^3$ in Formel (I) darstellt,
unter Bedingungen und/oder mit einem Reaktionspartner zur Umsetzung bringt, die dazu dienen bzw. das dazu dient, die Reste $Z^2$ und/oder $Z^3$ in den jeweiligen Rest $R^2$ und/oder $R^3$ zu überführen.

**11.** Verfahren gemäß Anspruch 9 oder 10, das nach einem oder mehreren der Schritte A bis F einen oder mehrere der folgenden Schritte in beliebiger Reihenfolge einschließt:

a) Abspalten der Schutzgruppen,
b) Umwandlung einer Verbindung der Formel (I) oder einer geschützten Form derselben in eine andere Verbindung der Formel (I) oder eine geschützte Form derselben,
c) Umwandeln der Verbindung der Formel (I) oder einer geschützten Form derselben in ein physiologisch annehmbares Derivat der Verbindung der Formel (I) oder dessen geschützter Form,
d) Umwandeln des physiologisch annehmbaren Derivats der Verbindung der Formel (I) oder des Derivats der geschützten Form derselben zur Verbindung der Formel (I) oder zu deren geschützter Form,
e) Überführen eines physiologisch annehmbaren Derivats der Verbindung der Formel (I) oder eines Derivats einer geschützten Form derselben in ein anderes physiologisch annehmbares Derivat der Verbindung der Formel (I) oder ein Derivat einer geschützten Form derselben,
f) falls erforderlich, Trennung der α- und β-Anomeren der Verbindung der Formel (I) oder von deren geschütztem Derivat oder von deren physiologisch geeignetem Derivat bzw. dessen geschützter Form und
g) bei Bedarf an den 4'-Sulfon- oder Sulfoxidderivaten die teilweise oder vollständige Oxidation des Schwefels der 4-Thiozuckereinheit der entsprechenden Verbindung der Formel (I), der geschützten Form derselben oder einer Verbindung der Formel (II) bzw. einer geschützten Form derselben mit einer Persäure.

**12.** Verfahren gemäß einem der Ansprüche 9B, 10E oder 11, bei dem man:

a) die Verbindung der Formel (IV) oder (IV-A), wie sie in den Ansprüchen 9 bzw. 10 definiert sind, oder eine geschützte Form derselben mit einer 4-Thiozuckerverbindung der Formel (VI),

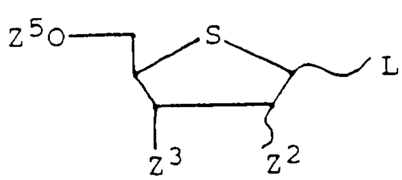

(VI)

in der $Z^2$, $Z^3$ und $Z^5$ wie in Anspruch 9 definiert sind und L eine Fluchtgruppe darstellt,
in Anwesenheit eines Lewissäure-Katalysators in einem Lösungsmittel bei erniedrigter, erhöhter oder Raumtemperatur umsetzt,

b) die wie oben definierte Verbindung der Formel (IV-A) oder (IV) oder eine geschützte Form derselben mit einer Verbindung der Formel (VII)

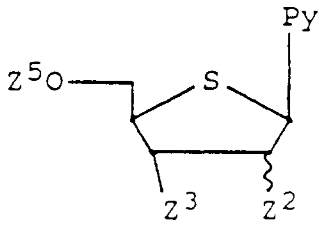

(VII)

in der $R^2$, $R^3$ und $R^5$ wie oben definiert sind und Py eine Pyrimidinbase darstellt,
in Anwesenheit eines Silylierungsmittels sowie eines Lewissäure-Katalysators zur Umsetzung bringt.

**13.** Verfahren gemäß einem der Ansprüche 10 bis 12, bei dem X $C_{2-4}$-Alkyl, Halogenalkyl oder Halogenalkenyl bzw. $C_{3-4}$-Alkenyl oder Alkinyl ist.

**14.** Verfahren gemäß einem der Ansprüche 9 - 13, bei dem die 4-Thiopentofuranosideinheit der Verbindung der Formel (I) ausgewählt ist aus den Resten:
Thio-D-ribofuranose,
4-Thio-D-arabinofuranose,
2-Desoxy-4-thio-D-ribofuranose,
2,3-Didesoxy-4-thio-D-pentenofuranose,
2,3-Didesoxy-4-thio-D-ribofuranose,
2-Fluor-4-thio-D-arabinofuranose,
2-Fluor-4-thio-D-ribofuranose und
2,3-Didesoxy-3-fluoro-4-thio-D-ribofuranose.

**15.** Verfahren gemäß einem der Ansprüche 9 bis 14, bei dem die erhaltene Verbindung der Formel (I) ausgewählt ist aus:
5-(2-Chlorethyl)-2'-desoxy-4'-thiouridin,
5-Nitro-2'-desoxy-4'-thiouridin,
5-Amino-2'-desoxy-4'-thiouridin,
5-Methylamino-2'-desoxy-4'-thiouridin,
5-Ethinyl-4'-thiouridin,
E-5-(2-Bromvinyl)-4'-thiouridin,
5-Ethinyl-1-(4-thio-β-D-arabinofuranosyl)-uracil,
5-Ethyl-1-(4-thio-β-D-arabinofuranosyl)-uracil,
5-(1-Propinyl)-1-(4-thio-β-D-arabinofuranosyl)-uracil,
E-5-(2-Bromvinyl)-1-(4-thio-β-D-arabinofuranosyl)-uracil,
1-(2-Fluor-4-thio-β-D-arabinofuranosyl)-5-methyluracil,
1-(2-Fluor-4-thio-β-D-arabinofuranosyl)-5-jodcytosin,
1-(2,3-Didehydro-2,3-didesoxy-4-thio-D-ribofuranosyl)-5-methyluracil und
2'-Desoxy-2'-fluor-4'-thiocytidin
ausgewählt ist.

**16.** Verfahren gemäß einem der Ansprüche 9 bis 15, bei dem ein physiologisch annehmbares Derivat der Verbindung der Formel (I) erhalten wird, bei dem es sich um ein Alkalimetall-, Erdalkalimetall-, Ammonium- oder $NR_4$-Salz (wobei R ein $C_{1-4}$-Alkyl darstellt), ein Hydrochlorid oder ein Acetat handelt.

**17.** Verfahren gemäß einem der Ansprüche 9 bis 16, bei dem man außerdem die erhaltene Verbindung der Formel (I) mit einem pharmazeutisch geeigneten Träger oder Verdünnungsmittel zubereitet.

**18.** Verwendung einer Verbindung gemäß einem der Ansprüche 1 bis 8 zur Herstellung eines Arzneimittels zur Anwendung bei der Behandlung oder Prophylaxe von Virusinfektionen.

**Patentansprüche für folgende Vertragsstaaten : ES, GR**

1. Verfahren zur Herstellung einer Verbindung der Formel (I)

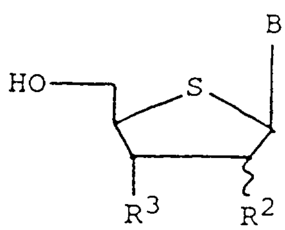

(I)

in der $B^1$ eine Pyrimidinbase darstellt und

(a) $R^2$ Wasserstoff und $R^3$ Wasserstoff, Hydroxy oder Fluor darstellen oder
(b) $R^2$ Hydroxy und $R^3$ Wasserstoff, Hydroxy oder Fluor oder
(c) $R^2$ Fluor und $R^3$ Wasserstoff oder Hydroxy bedeuten oder
(d) $R^2$ und $R^3$ zusammen eine Kohlenstoff-Kohlenstoffbindung bilden

sowie deren physiologisch wirksame Derivate,
mit der Maßgabe, daß

(i) dann, wenn $R^2$ Wasserstoff und $R^3$ Hydroxy bedeuten, $B^1$ keine Pyridinbase der Formel (X)

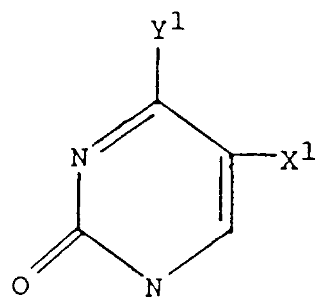

(X)

ist, in der
$Y^1$ eine Hydroxy- oder Aminogruppe darstellt und $X^1$ Fluor, Chlor, Brom, Jod, Methyl, Trifluormethyl, $C_{2-6}$-Alkyl, $C_{2-6}$-Alkenyl, $C_{2-6}$-Halogenalkenyl oder $C_{2-6}$-Alkinyl bedeutet,
(ii) daß es sich bei der Verbindung der Formel (I) nicht um 4'-Thiocytidin, 4'-Thiouracil, 1-(4-Thio-β-D-arabinofuranosyl)-cytosin, 1-(4-Thio-β-D-arabinofuranosyl)-thymidin oder 1-(4-Thio-β-D-arabinofuranosyl)-uracil handelt und
(iii) daß die Verbindung der Formel (I) kein 5-substituiertes 4'-Thiouridin ist, bei dem der Substituent in Position 5 Halogen oder Methyl ist,

bei dem man:

A) eine Verbindung der Formel (III-A)

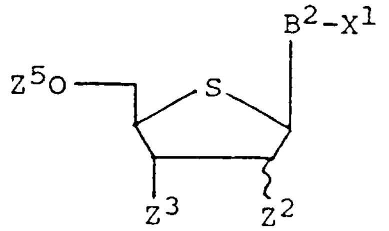

(III-A)

in der

$X^1$ einen Vorläufer eines Substituenten einer Pyridinbase darstellt, wie sie unter Bezugnahme auf $B^1$ in der Formel (I) definiert ist,

$B^2$ ein durch die Gruppe $X^1$ substituiertes Pyrimidin ist,

$Z^2$ und $Z^3$, die gleich oder verschieden sein können, Gruppen $R^2$ und $R^3$ oder geschützte Hydroxylgruppen darstellen und

$Z^5$ Wasserstoff oder eine Hydroxylschutzgruppe bedeutet,

mit einem Reagenz bzw. Reagenzien umsetzt, die dazu dienen, den Rest $X^1$ in die gewünschte Gruppe X umzuwandeln;

B) eine Verbindung der Formel (IV-A)

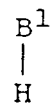

(IV-A)

oder eine geschützte Form derselben mit einem 4-Thiozuckerderivat umsetzt, um die 4-Thiozuckereinheit oder eine geschützte Form derselben in Position 1 der Base $B^1$ einzuführen;

C) eine Verbindung der Formel (V-A)

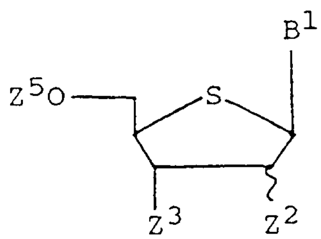

(V-A)

in der

$B^1$ eine wie oben definierte Pyrimidinbase darstellt,

$Z^5$ Wasserstoff oder eine Hydroxylschutzgruppe bedeutet, und

$Z^2$ und $Z^3$ wie oben definiert sind, wobei mindestens einer der Reste $Z^2$ und $Z^3$ eine Vorläufergruppe für den bzw. die Reste $R^2$ und/oder $R^3$ in Formel (I) darstellt,

unter Bedingungen und/oder mit einem Reaktionspartner zur Umsetzung bringt, die dazu dienen bzw. das dazu dient, die Reste $Z^2$ und/oder $Z^3$ in den jeweiligen Rest $R^2$ und/oder $R^3$ zu überführen.

**2.** Verfahren gemäß Anspruch 1, bei dem $B^1$ der Formel (II) entspricht

(II)

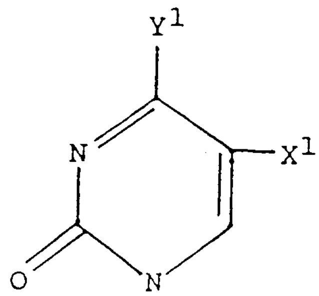

in der Y eine Hydroxy-, Amino-, Monoalkylamino- oder Dialkylaminogruppe darstellt und X Halogen, Alkoxy, Alkyl, Halogenalkyl, Alkenyl, Halogenalkenyl, Alkinyl oder eine Amino-, Monoalkylamino-, Dialkylamino-, Cyano- oder Nitrogruppe darstellt,
bei dem man

D) eine Verbindung der Formel (III)

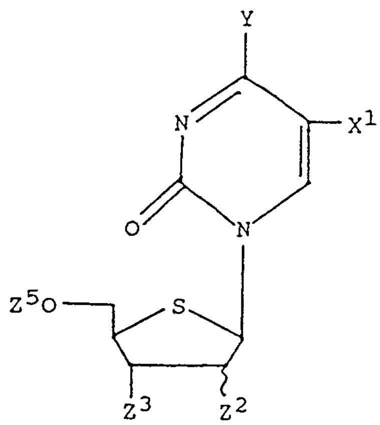

(III)

in der
$X^1$ ein Vorläufer der wie in bezug auf Formel (I) definierten Gruppe X ist, Y wie in bezug auf Formel (II) definiert ist, $Z^2$ und $Z^3$ gleich oder verschieden sind und Gruppen $R^2$ und $R^3$ oder geschützte Hydroxylgruppen darstellen und $Z^5$ Wasserstoff oder eine Hydroxylschutzgruppe bedeutet, mit einem oder mehreren Reaktionspartnern umsetzt, um den Rest $X^1$ in die gewünschte Gruppe X umzuwandeln,
E) eine Verbindung der Formel (IV)

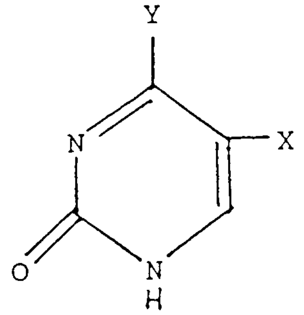

(IV)

in der X und Y die oben in bezug auf Formel (I) genannte Bedeutung haben, oder eine geschützte Form derselben
mit einem 4-Thiozuckerderivat umsetzt, um die 4-Thiozuckereinheit der Formel (I) bzw. eine geschützte Form derselben in Position 1 der Verbindung der Formel (I) einzuführen;

F) eine Verbindung der Formel (V)

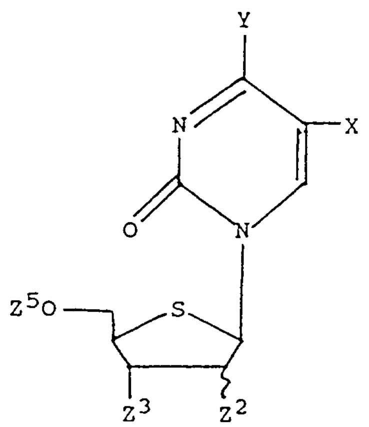

(V)

in der X und Y die oben in Bezug auf Formel (I) angegebene Bedeutung haben, $Z^5$ eine Hydroxyschutzgruppe oder Wasserstoff ist, $Z^2$ und $Z^3$ wie oben definiert sind, wobei mindestens einer der Reste $Z^2$ und $Z^3$ eine Vorläufergruppe für den bzw. die Reste $R^2$ und/oder $R^3$ in Formel (I) darstellt,
unter Bedingungen und/oder mit einem Reaktionspartner zur Umsetzung bringt, die dazu dienen bzw. das dazu dient, die Reste $Z^2$ und/oder $Z^3$ in den jeweiligen Rest $R^2$ und/oder $R^3$ zu überführen.

**3.** Verfahren gemäß Anspruch 1 oder 2, das nach einem oder mehreren der Schritte A bis F einen oder mehrere der folgenden Schritte in beliebiger Reihenfolge einschließt:

a) Abspalten der Schutzgruppen,
b) Umwandlung einer Verbindung der Formel (I) oder einer geschützten Form derselben in eine andere Verbindung der Formel (I) oder eine geschützte Form derselben,
c) Umwandeln der Verbindung der Formel (I) oder einer geschützten Form derselben in ein physiologisch annehmbares Derivat der Verbindung der Formel (I) oder dessen geschützter Form,
d) Umwandeln des physiologisch annehmbaren Derivats der Verbindung der Formel (I) oder des Derivats der geschützten Form derselben zur Verbindung der Formel (I) oder zu deren geschützter Form,
e) Überführen eines physiologisch annehmbaren Derivats der Verbindung der Formel (I) oder eines Derivats einer geschützten Form derselben in ein anderes physiologisch annehmbares Derivat der Verbindung der Formel (I) oder ein Derivat einer geschützten Form derselben,
f) falls erforderlich, Trennung der α- und β-Anomeren der Verbindung der Formel (I) oder von deren geschütztem Derivat oder von deren physiologisch geeignetem Derivat bzw. dessen geschützter Form und
g) bei Bedarf an den 4'-Sulfon- oder Sulfoxidderivaten die teilweise oder vollständige Oxidation des Schwefels der 4-Thiozuckereinheit der entsprechenden Verbindung der Formel (I), der geschützten Form derselben oder einer Verbindung der Formel (II) bzw. einer geschützten Form derselben mit einer Persäure.

**4.** Verfahren gemäß einem der Ansprüche 1B, 2E oder 3, bei dem man:

a) die Verbindung der Formel (IV) oder (IV-A), wie sie in den Ansprüchen 1 bzw. 2 definiert sind, oder eine geschützte Form derselben mit einer 4-Thiozuckerverbindung der Formel (VI),

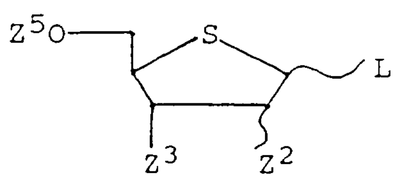

(VI)

in der $Z^2$, $Z^3$ und $Z^5$ wie in Anspruch 9 definiert sind und L eine Fluchtgruppe darstellt,
in Anwesenheit eines Lewissäure-Katalysators in einem Lösungsmittel bei erniedrigter, erhöhter oder Raumtemperatur umsetzt,

b) die wie obenstehend definierte Verbindung der Formel (IV-A) oder (IV) oder eine geschützte Form derselben mit einer Verbindung der Formel (VII)

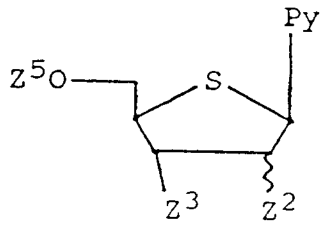

(VII)

in der $R^2$, $R^3$ und $R^5$ wie oben definiert sind und Py eine Pyrimidinbase darstellt,
in Anwesenheit eines Silylierungsmittels sowie eines Lewissäure-Katalysators zur Umsetzung bringt.

**5.** Verfahren gemäß einem der Ansprüche 2 bis 4, bei dem X $C_{2-4}$-Alkyl, Halogenalkyl oder Halogenalkenyl bzw. $C_{3-4}$-Alkenyl oder Alkinyl ist.

**6.** Verfahren gemäß einem der Ansprüche 1 bis 5, bei dem die 4-Thiopentofuranosideinheit der Verbindung der Formel (I) ausgewählt ist aus den Resten:
Thio-D-ribofuranose,
4-Thio-D-arabinofuranose,
2-Desoxy-4-thio-D-ribofuranose,
2,3-Didesoxy-4-thio-D-pentenofuranose,
2,3-Didesoxy-4-thio-D-ribofuranose,
2-Fluor-4-thio-D-arabinofuranose,
2-Fluor-4-thio-D-ribofuranose und
2,3-Didesoxy-3-fluor-4-thio-D-ribofuranose.

**7.** Verfahren gemäß einem der Ansprüche 1 bis 6, bei dem die erhaltene Verbindung der Formel (I) ausgewählt ist aus:
5-(2-Chlorethyl)-2'-desoxy-4'-thiouridin,
5-Nitro-2'-desoxy-4'-thiouridin,
5-Amino-2'-desoxy-4'-thiouridin,
5-Methylamino-2'-desoxy-4'-thiouridin,
5-Ethinyl-4'-thiouridin,
E-5-(2-Bromvinyl)-4'-thiouridin,
5-Ethinyl-1-(4-thio-β-D-arabinofuranosyl)-uracil,
5-Ethyl-1-(4-thio-β-D-arabinofuranosyl)-uracil,
5-(1-Propinyl)-1-(4-thio-β-D-arabinofuranosyl)-uracil,
E-5-(2-Bromvinyl)-1-(4-thio-β-D-arabinofuranosyl)-uracil,
1-(2-Fluor-4-thio-β-D-arabinofuranosyl)-5-methyluracil,
1-(2-Fluor-4-thio-β-D-arabinofuranosyl)-5-jodcytosin,
1-(2,3-Didehydro-2,3-didesoxy-4-thio-D-ribofuranosyl)-5-methyluracil und
2'-Desoxy-2'-fluor-4'-thiocytidin.

**8.** Verfahren gemäß einem der Ansprüche 1 bis 7, bei dem ein physiologisch annehmbares Derivat der Verbindung der Formel (I) erhalten wird, bei dem es sich um ein Alkalimetall-, Erdalkalimetall-, Ammonium- oder $NR_4$-Salz (wobei R ein $C_{1-4}$-Alkyl darstellt), ein Hydrochlorid oder ein Acetat handelt.

**9.** Verfahren gemäß einem der Ansprüche 1 bis 8, bei dem man außerdem die erhaltene Verbindung der Formel (I) mit einem pharmazeutisch geeigneten Träger oder Verdünnungsmittel zubereitet.

**10.** Verwendung einer Verbindung gemäß einem der vorstehenden Ansprüche zur Herstellung eines Arzneimittels zur Anwendung bei der Behandlung oder Prophylaxe von Virusinfektionen.

## Revendications

**Revendications pour les Etats contractants suivants : AT, BE, CH, DE, DK, FR, GB, IT, LI, LU, NL, SE**

**1.** Pyrimidine 4'-thionucléoside de formule (I)

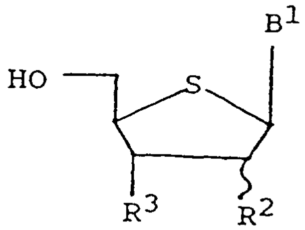

(I)

dans laquelle $B^1$ est une base pyrimidine ;
et soit (a) $R^2$ est l'hydrogène et $R^3$ est l'hydrogène, hydroxyle ou fluoro,
soit (b) $R^2$ est hydroxyle et $R^3$ est l'hydrogène, hydroxyle ou fluoro,
soit (c) $R^2$ est fluoro et $R^3$ l'hydrogène ou hydroxyle,
soit (d) $R^2$ et $R^3$ ensemble forment une liaison carbone-carbone ; et les dérivés à fonctionnalité physiologique de celui-ci,
à la condition que lorsque $R^2$ est l'hydrogène et $R^3$ est hydroxyle, $B^1$ ne soit pas une base pyrimidine de formule (X)

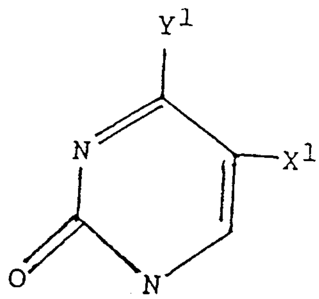

(X)

où $Y^1$ est hydroxyle ou amino et $X^1$ est chloro, bromo, iodo, trifluorométhyle, alkyle en $C_2$-$C_6$, alcényle en $C_2$-$C_6$, halogénoalkyle en $C_2$-$C_6$ ou alcynyle en $C_2$-$C_6$, pour l'utilisation dans une méthode de traitement ou de prévention d'infections virales de l'organisme humain ou animal.

**2.** Pyrimidine 4'-thionucléoside de formule (I)

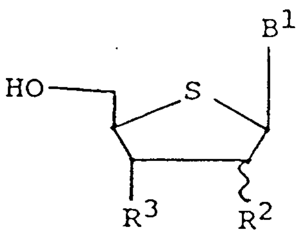

(I)

dans laquelle $B^1$ est une base pyrimidine ;
et soit (a) $R^2$ est l'hydrogène et $R^3$ est l'hydrogène, hydroxyle ou fluoro,
soit (b) $R^2$ est hydroxyle et $R^3$ est l'hydrogène, hydroxyle ou fluoro,
soit (c) $R^2$ est fluoro et $R^3$ l'hydrogène ou hydroxyle,
soit (d) $R^2$ et $R^3$ ensemble forment une liaison carbone-carbone ; et les dérivés à fonctionnalité physiologique de celui-ci,

à la condition que lorsque $R^2$ est l'hydrogène et $R^3$ est hydroxyle, $B^1$ ne soit pas une base pyrimidine de formule (X)

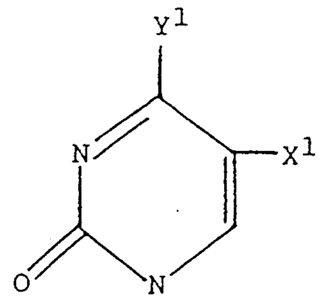

(X)

où $Y^1$ est hydroxyle ou amino et $X^1$ est chloro, bromo, iodo, trifluorométhyle, alkyle en $C_2$-$C_6$, alcényle en $C_2$-$C_6$, halogénoalkyle en $C_2$-$C_6$ ou alcynyle en $C_2$-$C_6$ ;

(ii) que le composé de formule (I) ne soit pas 4'-thiocytidine, 4'-thiouracile, 1-(4-thio-β-D-arabinofuranosyl)cytosine, 1-(4-thio-β-D-arabinofuranosyl)thymine, ou 1-(4-thio-β-D-arabinofuranosyl)uracile ; et
(iii) que le composé de formule (I) ne soit pas une 4'-thiouridine substituée en position 5 où le substituant en position 5 est un halogène ou méthyle.

**3.** Composé selon la revendication 1 ou 2 dans lequel $B^1$ répond à la formule (II)

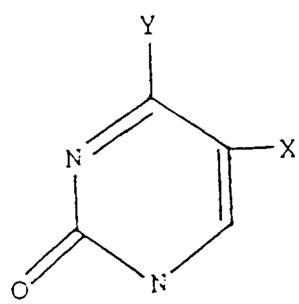

(II)

dans laquelle Y est hydroxyle ou amino, monoalkylamino ou dialkylamino ; et X est halogène, alcoxyle, alkyle, halogéno-alkyle, alcényle, halogéno-alcényle, alcynyle, amino, monoalkylamino, dialkylamino, cyano ou nitro.

**4.** Composé selon la revendication 3 où X est halogénoalcényle, halogénoalkyle ou alkyle en $C_2$-$C_4$, ou alcynyle ou alcényle en $C_3$-$C_4$.

**5.** Composé selon l'une quelconque des revendications précédentes où le fragment 4-thiopentofuranoside du composé de formule (I) est choisi parmi les résidus :
Thio-D-ribofuranose,
4-Thio-D-arabinofuranose,
2-Désoxy-4-thio-D-ribofuranose,
2,3-Didésoxy-4-thio-D-penténofuranose,
2,3-Didésoxy-4-thio-D-ribofuranose,
2-Fluoro-thio-D-arabinofuranose,
2-Fluoro-4-thio-D-ribofuranose, et
2,3-Didésoxy-3-fluoro-4-thio-D-ribofuranose.

**6.** Composé selon la revendication 1 ou 2 qui est choisi parmi :
5-(2-chloroéthyl)-2'-désoxy-4'-thiouridine ;
5-nitro-2'-désoxy-4'-thiouridine ;
5-amino-2'-désoxy-4'-thiouridine ;
5-méthylamino-2'-désoxy-4'-thiouridine ;
5- éthynyle-4'-thiouridine ;

E-5-(2-bromovinyl)-4'-thiouridine ;
5-éthynyl-1-(4-thio-β-D-arabino-furanosyl)uracile ;
5-éthyl-1-(4-thio-β-D-arabinofuranosyl)uracile;
5-(prop-1-ynyl)-1-(4-thio-β-D-arabinofuranosyl)uracile ;
E-5-(2-bromovinyl)-1-(4-thio-β-D-arabinofuranosyl)uracile ;
1-(2-fluoro-4-thio-β-D-arabinofuranosyl)-5-méthyluracile ;
1-(2-fluoro-4-thio-β-D-arabinofuranosyl)-5-iodocytosine ;
1-(2,3-didéshydro-2,3-didésoxy-4-thio-D-ribofuranosyl)-5-méthyluracile; et
2'-désoxy-2'-fluoro-4'-thiocytidine.

**7.** Dérivé physiologiquement acceptable d'un composé selon l'une quelconque des revendications précédentes qui est un sel de métal alcalin, de métal alcalinoterreux, d'ammonium, de $NR_4$ (dans lequel R est un alkyle en $C_1$-$C_4$), chlorhydrate ou acétate.

**8.** Composé selon l'une quelconque des revendications précédentes en association avec un véhicule ou diluant pharmaceutiquement acceptable.

**9.** Procédé pour la production d'un composé de formule (I)

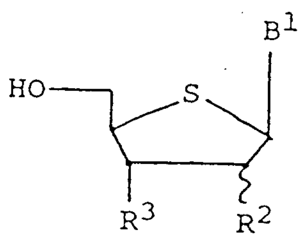

(I)

dans laquelle $B^1$ est une base pyrimidine ;
et soit (a) $R^2$ est l'hydrogène et $R^3$ est l'hydrogène, hydroxyle ou fluoro,
soit (b) $R^2$ est hydroxyle et $R^3$ est l'hydrogène, hydroxyle ou fluoro,
soit (c) $R^2$ est fluoro et $R^3$ l'hydrogène ou hydroxyle,
soit (d) $R^2$ et $R^3$ ensemble forment une liaison carbone-carbone ; et les dérivés à fonctionnalité physiologique de celui-ci,
à la condition que lorsque $R^2$ est l'hydrogène et $R^3$ est hydroxyle, $B^1$ ne soit pas une base pyrimidine de formule (X)

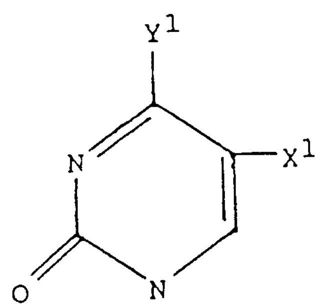

(X)

où $Y^1$ est hydroxyle ou amino et $X^1$ est chloro, bromo, iodo, trifluorométhyle, alkyle en $C_2$-$C_6$, alcényle en $C_2$-$C_6$, halogénoalkyle en $C_2$-$C_6$ ou alcynyle en $C_2$-$C_6$ ;

(ii) que le composé de formule (I) ne soit pas 4'-thiocytidine, 4'-thiouracile, 1-(4-thio-β-D-arabinofuranosyl)cytosine, 1-(4-thio-β-D-arabinofuranosyl)thymine ou 1-(4-thio-β-D-arabinofuranosyl)uracile, et
(iii) et que le composé de formule (I) ne soit pas une 4'-thiouridine substituée en position 5 où le substituant en position 5 est un halogène ou méthyle ;

lequel procédé comprend :

A) la réaction d'un composé de formule (III-A)

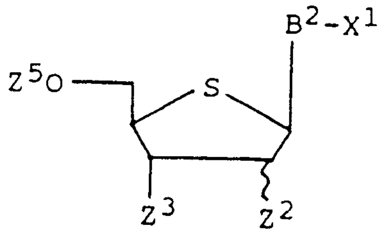

(III-A)

dans laquelle $X^1$ est un précurseur d'un substituant d'une base pyrimidine définie en relation à $B^1$ dans la formule (I) ;

$B^2$ est une pyrimidine substituée par le groupe $X^1$ ;

$Z^2$ et $Z^3$ sont identiques ou différents et correspondent aux groupes $R^2$ et $R^3$ ou sont des hydroxyles protégés ; et

$Z^5$ est l'hydrogène ou un groupe protecteur d'hydroxyle avec un réactif ou des réactifs qui servent à convertir le groupe $X^1$ en le groupe X désiré ;

B) la réaction d'un composé de formule (IV-A)

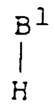

(IV-A)

ou une forme protégée de celui-ci avec un dérivé 4-thioglucidique qui sert à introduire le fragment 4-thioglucidique ou une forme protégée de celui-ci dans la position 1 de la base $B^1$ ;

C) la réaction d'un composé de formule (V-A)

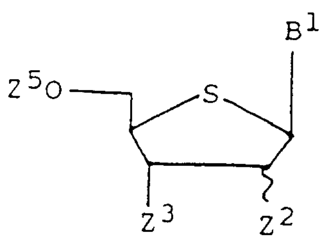

(V-A)

où

$B^1$ est une base pyrimidine définie ci-dessus ;

$Z^5$ est l'hydrogène ou un groupe protecteur d'hydroxyle, et

$Z^2$ et $Z^3$ sont définis ci-dessus où au moins un des $Z^2$ et $Z^3$ représente un groupe précurseur pour le(s) groupe(s) $R^2$ et/ou $R^3$ dans la formule (I)

dans ces conditions et/ou avec un réactif servant à convertir les groupes $Z^2$ et/ou $Z^3$ en les groupes respectifs $R^2$ et/ou $R^3$.

**10.** Procédé selon la revendication 9 où $B^1$ répond à la formule (II)

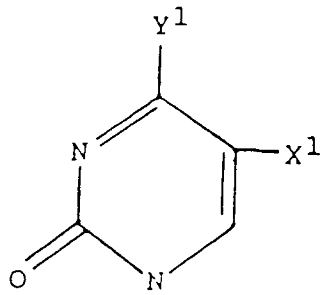

(X)

dans laquelle Y est hydroxyle ou amino, monoalkylamino ou dialkylamino ; et X est halogène, alcoxyle, alkyle, halo, alcényle, halogénoalcényle, alcynyle, amino, monoalkylamino, dialkylamino, cyano ou nitro ;

D) la réaction d'un composé de formule (III)

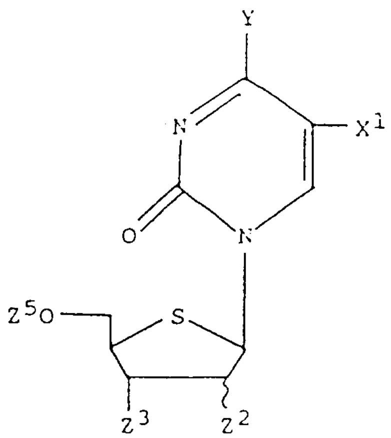

(III)

où $X^1$ est un précurseur du groupe X tel que défini en relation à la formule (I) ;
Y est défini en relation à la formule (II) ;
$Z^2$ et $Z^3$ sont identiques ou différents et correspondent aux groupes $R^2$ et $R^3$ ou aux groupes hydroxyles protégés et $Z^5$ est l'hydrogène ou un groupe protecteur d'hydroxyle
avec un réactif ou des réactifs servant à convertir le groupe $X^1$ en le groupe X désiré ;
E) la réaction d'un composé de formule (IV)

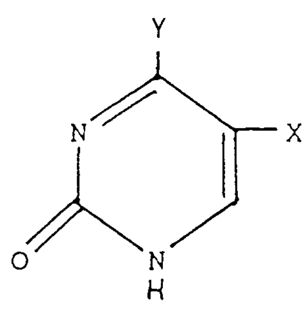

(IV)

dans lequel X et Y sont définis en relation à la formule (I) ou une forme protégé de celui-ci avec le dérivé 4-thioglucidique servant à introduire le fragment 4-thioglicidique de formule (I) ou une forme protégé de celui-ci dans la position 1 du composé de formule (IV) ;

F) la réaction d'un composé de formule (V)

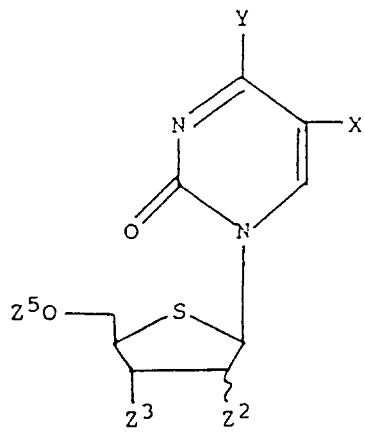

(V)

où X et Y sont définis en relation à la formule (I), $Z^5$ est un groupe protecteur d'hydroxyle ou l'hydrogène ; $Z^2$ et $Z^3$ sont définis ci-dessus où au moins l'un des $Z^2$ et $Z^3$ représente un groupe précurseur du (des) groupe(s) $R^2$ et/ou $R^3$ dans la formule (I) dans ces conditions et/ou avec un réactif servant à convertir les groupes $Z^2$ et/ou $Z^3$ en les groupes respectifs $R^2$ et/ou $R^3$.

**11.** Procédé selon la revendication 9 ou 10 qui comprend en outre selon un ou plusieurs procédés A à F, n'importe quelle ou plusieurs des étapes ultérieures suivantes dans n'importe quel ordre :

a) élimination de chacun des groupes protecteurs,
b) conversion d'un composé de formule (I) ou d'une forme protégée de celui-ci en un autre composé de formule (I) ou une forme protégée de celui-ci,
c) conversion du composé de formule (I) ou d'une forme protégée de celui-ci en un dérivé physiologiquement acceptable du composé de formule (I) ou une forme protégée de celui-ci,
d) conversion d'un dérivé physiologiquement acceptable du composé de formule (I) ou d'une forme protégée de celui-ci en le composé de formule (I) ou une forme protégée de celui-ci,
e) conversion d'un dérivé physiologiquement acceptable du composé de formule (I) ou d'une forme protégée de celui-ci en un autre dérivé du composé de formule (I) physiologiquement acceptable ou d'une forme protégée de celui-ci,
f) si nécessaire séparation des anomères $\alpha$ et $\beta$ du composé de formule (I) ou d'un dérivé protégé de celui-ci ou d'un dérivé physiologiquement acceptable d'un composé de formule (I) ou d'un dérivé de celui-ci, et
g) lorsqu'on a besoin de dérivés du fragment 4'-sulfone ou sulfoxyde glucidique, oxydation partielle ou complète du soufre du fragment 4-thioglucidique du composé de formule (I) correspondant ou d'une forme protégée de celui-ci ou d'un composé de formule (II) ou d'une forme protégée de celui-ci, par un peracide.

**12.** Procédé selon l'une quelconque des revendications 9B, 10E ou 11 qui comprend :

a) la réaction du composé de formule (IV) ou (IVa) définie dans les revendications 9 et 10 respectivement, ou d'une forme protégée de celui-ci, avec un composé de 4-thioglucidique de formule (VI)

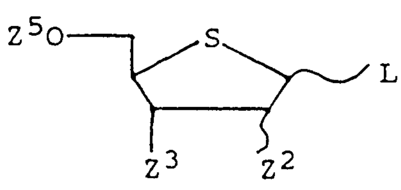

(VI)

dans lequel $Z^2$, $Z^3$ et $Z^5$ sont définis dans la revendication 9 et L est un groupe éliminable,
en présence d'un catalyseur de type acide de Lewis dans un solvant, à une température réduite, ambiante ou élevée ;

b) la réaction du composé de formule (IV-A) ou (IV) défini ci-dessus ou d'une forme protégée de celui-ci avec un composé de formule (VII)

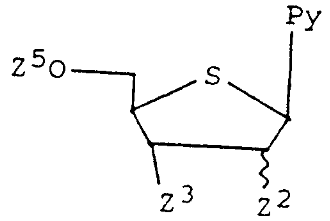

(VII)

dans laquelle $Z^2$, $Z^3$ et $Z^5$ sont définis comme ci-dessus et Py représente une base pyrimidine, en présence d'un agent silylant et en présence d'un catalyseur de type acide de Lewis.

**13.** Procédé selon l'une quelconque des revendications 10 à 12 où X est halogénoalcényle, halogénoalkyle ou alkyle en $C_2$-$C_4$, ou alcynyle ou alcényle en $C_3$-$C_4$.

**14.** Procédé selon l'une quelconque des revendications 9 à 13, où le fragment 4-thiopentanofuranoside du composé de formule (I) est choisi parmi les résidus :

Thio-D-ribofuranose,
4-Thio-D-arabinofuranose,
2-Désoxy-4-thio-D-ribofuranose,
2,3-Didésoxy-4-thio-D-pentanofuranose,
2,3-Didésoxy-4-thio-D-ribofuranose,
2-Fluoro-4-thio-D-arabinofuranose,
2-Fluoro-4-thio-D-ribofuranose, et
2,3-Didésoxy-3-fluoro-4-thio-D-ribofuranose.

**15.** Procédé selon l'une quelconque des revendications 9 à 14, où le composé le formule (I) obtenu est choisi parmi :

5-(2-chloroéthyl)-2'-désoxy-4'-thiouridine,
5-nitro-2'-désoxy-4'-thiouridine,
5-amino-2'-désoxy-4'-thiouridine,
5-méthylamino-2'-désoxy-4'-thiouridine,
5-éthynyl-4'-thiouridine,
E-5-(2-bromovinyl)-4'-thiouridine,
5-éthynyl-1-(4-thio-β-D-arabinofuranosyl)uracile,
5-éthyl-1-(4-thio-β-D-arabinofuranosyl)uracile,
5-(prop-1-ynyl)-1-(4-thio-β-D-arabinofuranosyl)uracile,
E-5-(2-bromovinyl)-1-(4-thio-β-D-arabinofuranosyl)uracile,
1-(2-fluoro-4-thio-β-D-arabinofuranosyl)-5-méthyl)uracile,
1-(2-fluoro-4-thio-β-D-arabinofuranosyl)-5-iodocytosine,
1-(2,3-didéshydro-2,3-didésoxy-4-thio-β-D-arabinofuranosyl)-5-méthyluracile, et
2'-désoxy-2'-fluoro-4'-thiocytidine.

**16.** Procédé selon l'une quelconque des revendications 9 à 15, où on obtient un dérivé physiologiquement acceptable du composé de formule (I), le dérivé étant un sel de métal alcalin, de métal alcalinoterreux, d'ammonium, de $NR_4$ (où R est alkyle en $C_1$-$C_4$), chlorhydrate ou acétate.

**17.** Procédé selon l'une quelconque des revendications 9 à 16, qui comprend de plus la formulation du composé de formule (I) obtenu avec un véhicule ou diluant pharmaceutiquement acceptable.

**18.** Utilisation d'un composé selon l'une quelconque des revendications 1 à 8 pour la production de médicament destiné au traitement ou à la prévention d'une infection virale.

**Revendications pour les Etats contractants suivants : ES, GR**

1. Procédé pour la préparation d'un composé de formule (I)

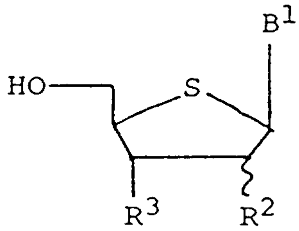

(I)

dans laquelle $B^1$ est une base pyrimidine ;
et soit (a) $R^2$ est l'hydrogène et $R^3$ est l'hydrogène, hydroxyle ou fluoro,
soit (b) $R^2$ est hydroxyle et $R^3$ est l'hydrogène, hydroxyle ou fluoro,
soit (c) $R^2$ est fluoro et $R^3$ l'hydrogène ou hydroxyle,
soit (d) $R^2$ et $R^3$ ensemble forment une liaison carbone-carbone ; et les dérivés à fonctionnalité physiologique de celui-ci,
à la condition que

(i) lorsque $R^2$ est l'hydrogène et $R^3$ est hydroxyle, $B^1$ ne soit pas une base pyrimidine de formule (X)

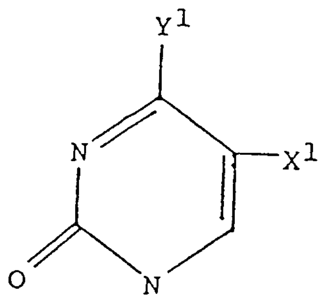

(X)

où $Y^1$ est hydroxyle ou amino et $X^1$ est chloro, bromo, iodo, méthyle, trifluorométhyle, alkyle en $C_2$-$C_6$, alcényle en $C_2$-$C_6$, halogénoalkyle en $C_2$-$C_6$ ou alcynyle en $C_2$-$C_6$ ;
(ii) que le composé de formule (I) ne soit pas 4'-thiocytidine, 4'-thiouracile, 1-(4-thio-β-D-arabinofuranosyl)cytosine, 1-(4-thio-β-D-arabinofuranosyl)thymine ou 1-(4-thio-β-D-arabinofuranosyl)uracile, et
(iii) et que le composé de formule (I) ne soit pas une 4'-thiouridine substituée en position 5 où le substituant en position 5 est un halogène ou méthyle ;

lequel procédé comprend :

A) la réaction d'un composé de formule (III-A)

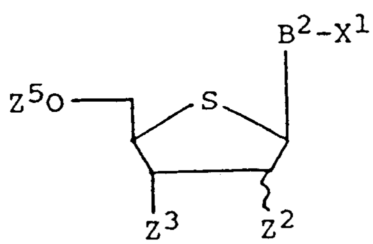

(III-A)

dans laquelle $X^1$ est un précurseur d'un substituant d'une base pyrimidine définie en relation à $B^1$ dans la formule (I) ;

$B^2$ est une pyrimidine substituée par le groupe $X^1$ ;

$Z^2$ et $Z^3$ sont identiques ou différents et correspondent aux groupes $R^2$ et $R^3$ ou sont des hydroxyles protégés ; et

$Z^5$ est l'hydrogène ou un groupe protecteur d'hydroxyle avec un réactif ou des réactifs qui servent à convertir le groupe $X^1$ en le groupe X désiré;

B) la réaction d'un composé de formule (IV-A)

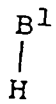

(IV-A)

ou une forme protégée de celui-ci avec un dérivé 4-thioglucidique qui sert à introduire le fragment 4-thioglucidique ou une forme protégée de celui-ci dans la position 1 de la base $B^1$ ;

C) la réaction un composé de formule (V-A)

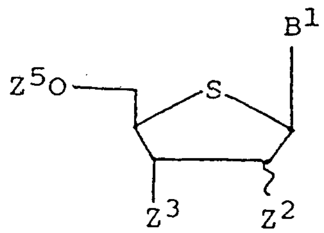

(V-A)

où

$B^1$ est une base pyrimidine définie ci-dessus ;

$Z^5$ est l'hydrogène ou un groupe protecteur d'hydroxyle, et

$Z^2$ et $Z^3$ sont définis ci-dessus, où au moins un des $Z^2$ et $Z^3$ représente un groupe précurseur du (des) groupe(s) $R^2$ et/ou $R^3$ dans la formule (I)

dans ces conditions et/ou avec un réactif servant à convertir les groupes $Z^2$ et/ou $Z^3$ en les groupes respectifs $R^2$ et/ou $R^3$.

2. Procédé selon la revendication 1 où $B^1$ est un composé de formule (II)

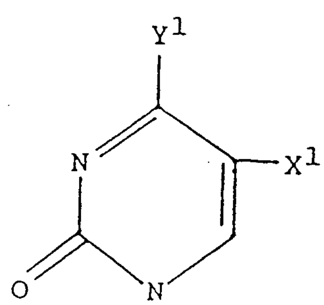

(X)

dans laquelle Y est hydroxyle ou amino, monoalkylamino ou dialkylamino ; et X est halogène, alcoxyle, alkyle, halo, alcényle, halogénoalcényle, alcynyle, amino, monoalkylamino, dialkylamino, cyano ou nitro, qui comprend

D) la réaction d'un composé de formule (III)

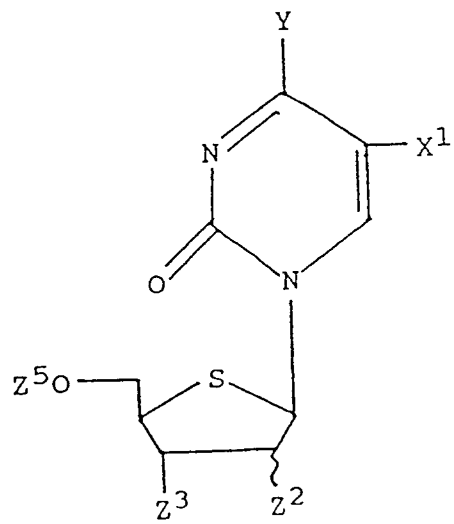

(III)

où $X^1$ est un précurseur du groupe X tel que défini en relation à la formule (I) ;

Y est défini en relation à la formule (II) ;

$Z^2$ et $Z^3$ sont identiques ou différents et correspondent aux groupes $R^2$ et $R^3$ ou aux groupes hydroxyles protégés et $Z^5$ est l'hydrogène ou un groupe protecteur d'hydroxyle

avec un réactif ou des réactifs servant à convertir le groupe $X^1$ en le groupe X désiré ;

E) la réaction d'un composé de formule (IV)

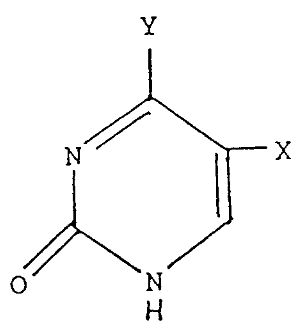

(IV)

dans lequel X et Y sont définis en relation à la formule (I) ou une forme protégé de celui-ci avec le dérivé 4-thioglucidique servant à introduire le fragment 4-thioglicidique de formule (I) ou une forme protégé de celui-ci dans la position 1 du composé de formule (IV) ;

F) la réaction d'un composé de formule (V)

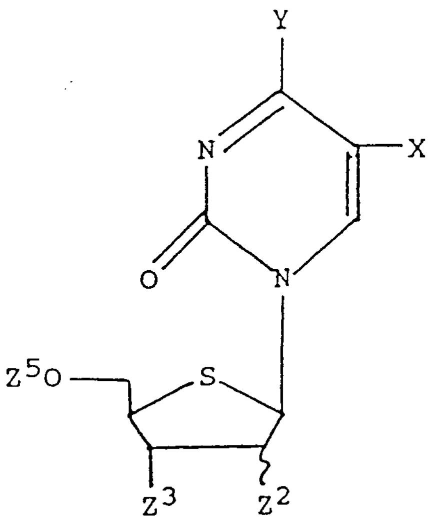

(V)

où X et Y sont définis en relation à la formule (I), $Z^5$ est un groupe protecteur d'hydroxyle ou l'hydrogène ; $Z^2$ et $Z^3$ sont définis ci-dessus où au moins l'un des $Z^2$ et $Z^3$ représente un groupe précurseur du (des) groupe(s) $R^2$ et/ou $R^3$ dans la formule (I)

dans ces conditions et/ou avec un réactif servant à convertir les groupes $Z^2$ et/ou $Z^3$ en les groupes respectifs $R^2$ et/ou $R^3$.

**3.** Procédé selon la revendication 1 ou 2 qui comprend de plus, selon un ou plusieurs des procédés A à F, n'importe quelle ou plusieurs des étapes suivantes dans n'importe quelle succession :

a) élimination de chacun des groupes protecteurs,
b) conversion d'un composé de formule (I) ou d'une forme protégée de celui-ci en un autre composé de formule (I) ou une forme protégée de celui-ci,
c) conversion du composé de formule (I) ou d'une forme protégée de celui-ci en un dérivé physiologiquement acceptable du composé de formule (I) ou une forme protégée de celui-ci,
d) conversion d'un dérivé physiologiquement acceptable du composé de formule (I) ou d'une forme protégée de celui-ci en le composé de formule (I) ou une forme protégée de celui-ci,
e) conversion d'un dérivé physiologiquement acceptable du composé de formule (I) ou d'une forme protégée de celui-ci en un autre dérivé du composé physiologiquement acceptable de formule (I) ou une forme protégée de celui-ci,
f) si nécessaire séparation des anomères $\alpha$ et $\beta$ du composé de formule (I) ou d'un dérivé protégé de celui-ci ou d'un dérivé physiologiquement acceptable d'un composé de formule (I) ou un dérivé de celui-ci, et
g) lorsqu'on a besoin de dérivés du fragment 4'-sulfone ou sulfoxyde glucidique, oxydation partielle ou complète du soufre du fragment 4-thioglucidique du composé de formule (I) correspondant ou d'une forme protégée de celui-ci ou d'un composé de formule (II) ou d'une forme protégée de celui-ci, par un peracide.

**4.** Procédé selon l'une quelconque des revendications 1, 2 ou 3 qui comprend :

a) la réaction du composé de formule (IV) ou (IVa) définie dans les revendications 1 et 2 respectivement, ou d'une forme protégée de celui-ci, avec un composé de 4-thioglucidique de formule (VI)

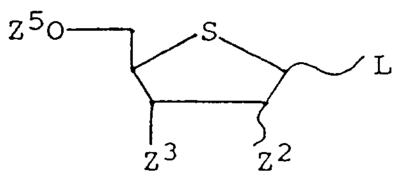

(VI)

dans lequel $Z^2$, $Z^3$ et $Z^5$ sont définis dans la revendication 9 et L est un groupe éliminable, en présence d'un catalyseur de type acide de Lewis dans un solvant, à une température réduite, ambiante ou élevée ;

b) la réaction du composé de formule (IV-A) ou (IV) défini ci-dessus ou d'une forme protégée de celui-ci avec un composé de formule (VII)

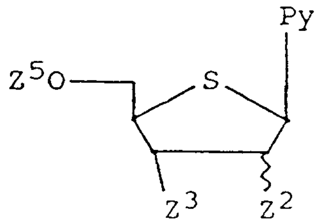

(VII)

dans laquelle $Z^2$, $Z^3$ et $Z^5$ sont définis comme ci-dessus et Py représente une base pyrimidine, en présence d'un agent silylant et en présence d'un catalyseur de type acide de Lewis.

**5.** Procédé selon l'une quelconque des revendications 2 à 4 où X est halogénoalcényle, halogénoalkyle ou alkyle en $C_2$-$C_4$, ou alcynyle ou alcényle en $C_3$-$C_4$.

**6.** Procédé selon l'une quelconque des revendications 1 à 5 où le fragment 4-thiopentanofuranoside du composé de formule (I) est choisi parmi les résidus :

Thio-D-ribofuranose,
4-Thio-D-arabinofuranose,
2-Désoxy-4-thio-D-ribofuranose,
2,3-Didésoxy-4-thio-D-penténofuranose,
2,3-Didésoxy-4-thio-D-ribofuranose,
2-Fluoro-4-thio-D-arabinofuranose,
2-Fluoro-4-thio-D-ribofuranose, et
2,3-Didésoxy-3-fluoro-4-thio-D-ribofuranose.

**7.** Procédé selon l'une quelconque des revendications 1 à 6, où le composé de formule (I) obtenu est choisi parmi :

5-(2-chloroéthyl)-2'-désoxy-4'-thiouridine ;
5-nitro-2'-désoxy-4'-thiouridine ;
5-amino-2'-désoxy-4'-thiouridine ;
5-méthylamino-2'-désoxy-4'-thiouridine ;
5-éthynyl-4'-thiouridine ;
E-5-(2-bromovinyl)-4'-thiouridine ;
5-éthynyl-1-(4-thio-β-D-arabinofuranosyl)uracile ;
5-éthyl-1-(4-thio-β-D-arabinofuranosyl)uracile ;
5-(prop-1-ynyl)-1-(4-thio-β-D-arabinofuranosyl)uracile ;
E-5-(2-bromovinyl)-1-(4-thio-β-D-arabinofuranosyl)uracile ;
1-(2-fluoro-4-thio-β-D-arabinofuranosyl)-5-méthyl)uracile ;
1-(2-fluoro-4-thio-β-D-arabinofuranosyl)-5-iodocytosine ;

1-(2,3-didéshydro-2,3-didésoxy-4-thio-β-D-arabinofuranosyl)-5-méthyluracile ; et
2'-désoxy-2'-fluoro-4'-thiocytidine.

**8.** Procédé selon l'une quelconque des reendications 1 à 7, où on obtient un dérivé physiologiquement acceptable du composé de formule (I), lequel dérivé étant un sel de métal alcalin, de métal alcalinoterreux, d'ammonium, de $NR_4$ (où R est alkyle en $C_1$-$C_4$), chlorhydrate ou acétate.

**9.** Procédé selon l'une quelconque des revendications 1 à 8, qui comprend de plus la formulation du composé de formule (I) obtenu, avec des véhicules et diluants pharmaceutiquement acceptables.

**10.** Utilisation du composé de l'une quelconque des revendications 1 à 8 pour la production d'un médicament pour l'utilisation dans le traitement ou dans la prévention d'une infection virale.